(19) Europäisches Patentamt
European Patent Office
Office européen des brevets

(11) **EP 3 774 995 B1**

(12) **EUROPÄISCHE PATENTSCHRIFT**

(45) Veröffentlichungstag und Bekanntmachung des Hinweises auf die Patenterteilung:
**21.06.2023 Patentblatt 2023/25**

(21) Anmeldenummer: **19713462.0**

(22) Anmeldetag: **26.03.2019**

(51) Internationale Patentklassifikation (IPC):
***C08G 77/388*** *(2006.01)* ***C11D 3/37*** *(2006.01)*
***A61K 8/898*** *(2006.01)* ***D06M 15/643*** *(2006.01)*
***C11D 3/00*** *(2006.01)*

(52) Gemeinsame Patentklassifikation (CPC):
(C-Sets verfügbar)
**C11D 3/3742; A61K 8/898; A61Q 19/00; A61Q 19/10; C08G 77/12; C08G 77/16; C08G 77/26; C08G 77/38; C08G 77/388; C08K 5/1515; C08K 5/16; C08K 5/17; C09D 183/08; C11D 3/0015; D06M 15/652;** (Forts.)

(86) Internationale Anmeldenummer:
**PCT/EP2019/057491**

(87) Internationale Veröffentlichungsnummer:
**WO 2019/192876 (10.10.2019 Gazette 2019/41)**

(54) **SILOXANE ZUR BEHANDLUNG VON TEXTILIEN UND ZUR VERWENDUNG IN REINIGUNGS- UND PFLEGEFORMULIERUNGEN**

SILOXANES FOR THE TREATMENT OF TEXTILES AND FOR THEIR USE IN CLEANING AND CARE FORMULATIONS

SILOXANE DESTINÉ AU TRAITEMENT DE TEXTILES ET À L'UTILISATION DANS DES FORMULATIONS DE NETTOYAGE ET DE SOIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.04.2018 EP 18165408**

(43) Veröffentlichungstag der Anmeldung:
**17.02.2021 Patentblatt 2021/07**


(73) Patentinhaber: **Evonik Operations GmbH
45128 Essen (DE)**

(72) Erfinder:
- **HENNING, Frauke
45259 Essen (DE)**
- **PEGGAU, Jörg
45357 Essen (DE)**
- **LOHSE, Andrea
46236 Bottrop (DE)**
- **ZÜNDORFF, Astrid
45472 Mülheim an der Ruhr (DE)**
- **RADLOFF, Sarah
44805 Bochum (DE)**
- **TRAMBITAS, Alexandra
63755 Alzenau (DE)**

(74) Vertreter: **Evonik Patent Association
c/o Evonik Industries AG
IP Management
Bau 1042A/PB 15
Paul-Baumann-Straße 1
45772 Marl (DE)**


(56) Entgegenhaltungen:
**WO-A1-2011/088937 DE-A1-102009 029 450
DE-A1-102010 001 531 DE-A1-102011 078 382
US-A- 5 248 783**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
D06M 2200/50

C-Sets
**C08K 5/1515, C08L 83/04;**
**C08K 5/16, C08L 83/06;**
**C08K 5/17, C08L 83/06**

## Beschreibung

Die Erfindung betrifft spezielle Siloxane, Zusammensetzungen, die diese speziellen Siloxane enthalten, Verfahren zu ihrer Herstellung, sowie die Verwendung dieser Zusammensetzungen zur Behandlung von Flächengebilden, in Reinigungs- und Pflegeformulierungen für den Haushalt und für industrielle Anwendungen und in kosmetischen, pharmazeutischen und dermatologischen Zusammensetzungen, insbesondere in kosmetischen Reinigungs- und Pflegeformulierungen, Haarbehandlungsmitteln und Haarnachbehandlungsmitteln, sowie zur Reinigung und Pflege von harten Oberflächen, vorzugsweise zur Reinigung und Pflege von Fahrzeugen, insbesondere als Additiv in Trocknungshilfen für Autowaschstraßen.

Insbesondere betrifft die Erfindung griffgebende Wirkstoffe zur Behandlung von Textilien oder Geweben, welche sowohl eine höhere Wirksamkeit als auch minimierte Anteile unerwünschter niedermolekularer Nebenproduktbestandteile aufweisen.

Siloxane oder Silikone mit quartären (quaternären) Ammoniumgruppen (im Folgenden auch als Silikonquats bezeichnet) und ihre Verwendung zum Ausrüsten von Textilien sowie in Reinigungs- und Pflegeformulierungen sind aus dem Stand der Technik bekannt.

Besonders vorteilhaft sind dabei Silikonquats, die durch Umsetzung von epoxyfunktionellen Siloxanen mit tertiären Aminen erhalten werden. Dabei findet zunächst ein nukleophiler Angriff des tertiären Stickstoffs des tertiären Amins an den Epoxidring des epoxyfunktionellen Silans statt, der schließlich zur Ringöffnung des Epoxidrings führt. Es bildet so sich ein Zwitterion, das in der Folge durch eine Brønsted-Säure protoniert wird, wodurch das Silikonquat erhalten wird. Da die Epoxidgruppen möglichst vollständig umgesetzt werden sollen, werden in der Regel stöchiometrische Mengen des tertiären Amins eingesetzt. Dies kann wiederum zu Restmengen von tertiären Aminen führen. Diese Restmengen sind unerwünscht. Restgehalte von niedermolekularen Reaktanden, wie beispielsweise den eingesetzten Aminen oder niedermolekularen organischen Nebenprodukten, können hautreizend, sensibilisierend und/oder aquatoxisch sein. Beim Einbringen der Silikonquats in wässrige Textilbehandlungsflotten können sich die niedermolekularen organischen Nebenprodukte als auch die nicht umgesetzten tertiären Amine lösen und so auf das Textil oder ins Abwasser geraten.

Die Verwendung von Aminen, die weniger bedenklich sind, insbesondere weniger oder gar nicht hautreizend, sensibilisierend und/oder aquatoxisch sind, führt in der Regel zu Silikonquats mit schlechteren anwendungstechnischen Eigenschaften. Wässrige Emulsionen zur Textilausrüstung auf der Basis der entsprechenden Silikonquats zeigen häufig eine verminderte Phasenstabilität/Lagerstabiliät und/oder eine schlechtere Griffbeurteilung des mit Ihnen ausgerüsteten Textils.

DE 102010000993 A1 offenbart Polysiloxane, die mindestens eine quatäre (quartäre, quaternäre) Ammoniumgruppe aufweisen. Diese Polysiloxane sind in Körperreinigungs- und Pflegemitteln, wie Shampoos, Haarbehandlungsmittel und Haarnachbehandlungsmittel, einsetzbar. Diese Polysiloxane sollen sowohl Eigenschaften wie die Kämmbarkeit, die Weichheit, das Volumen, die Formbarkeit, die Handhabbarkeit und die Entwirrbarkeit von ungeschädigten und geschädigten Haaren verbessern, als auch dem Haar einen schönen Glanz verleihen. In den Beispielen werden Polysiloxane offenbart, die durch Umsetzung von Epoxysilanen mit Amidaminen auf Basis von Fettsäuren, und zwar 3-N,N-Dimethyl-aminopropyl-laurylsäureamid, erhalten werden. Die ausschließliche Verwendung von Amidaminen als tertiäre Amine in der Synthese dieser Silikonquats führt zu einem Restgehalt an nicht umgesetzten Amidaminen. Dies ist unerwünscht. Polysiloxane mit mindestens einer Amidammoniumgruppe als auch mindestens einer Dialkanolammoniumgruppe werden nicht offenbart.

DE 102009029450 A1 offenbart Polysiloxane mit quartären Ammoniumgruppen. Diese Polysiloxane finden als Weichmacher für Flächengebilde wie beispielsweise Gewebe, Tissue, Non-wovens und/oder Fasern aus natürlichen und/oder synthetischen Rohstoffen und/oder Leder Verwendung. In den Beispielen werden Polysiloxane offenbart, die durch Umsetzung von Epoxysilanen mit Amidaminen auf Basis von Kokosfettsäure erhalten werden. Die ausschließliche Verwendung von Amidaminen als tertiäre Amine in der Synthese dieser Silikonquats führt auch hier zu einem unerwünschten Restgehalt an nicht umgesetzten Amidaminen. Polysiloxane mit mindestens einer Amidammoniumgruppe als auch mindestens einer Dialkanolammoniumgruppe werden nicht offenbart.

DE 102011078382 A1 offenbart Mikroemulsionen, welche als Öl-Phase ein mindestens eine quaternäre Ammoniumgruppe enthaltendes Polysiloxan aufweisen. In den Beispielen werden Polysiloxane offenbart, die durch Umsetzung von Epoxysilanen mit Amidaminen auf Basis von Fettsäuren erhalten werden. Die ausschließliche Verwendung von Amidaminen als tertiäre Amine in der Synthese dieser Silikonquats führt auch hier zu einem unerwünschten Restgehalt an nicht umgesetzten Amidaminen. Polysiloxane mit mindestens einer Amidammoniumgruppe als auch mindestens einer Dialkanolammoniumgruppe werden nicht offenbart.

DE 102010001531 A1 offenbart Siloxane mit primären Aminofunktionen sowie organomodifizierte Siloxane mit quaternären Ammoniumfunktionen. Polysiloxane, die eine Amidammoniumgruppe oder eine Dialkanolammoniumgruppe aufweisen, werden nicht offenbart.

US 5248783 offenbart die Verwendung von Amidaminen zur Neutralisation von carbonsäurefunktionellen Silikonen. Polysiloxane, die eine Amidammoniumgruppe oder eine Dialkanolammoniumgruppe aufweisen, werden nicht offenbart

Es besteht weiterhin der Bedarf, Siloxane bereitzustellen, die Vorteile gegenüber dem Stand der Technik aufweisen.

Insbesondere besteht der Bedarf an Siloxanen, die als griffgebende Wirkstoffe zur Behandlung von Textilien oder Geweben geeignet sind, und sich durch eine hohe Wirksamkeit auszeichnen, bei denen aber zudem der Anteil unerwünschter organischer Nebenprodukte oder Restmengen von Reaktanden, die hautreizend, sensibilisierend und/oder aquatoxisch sind, minimiert ist. Es besteht weiterhin der Bedarf an Siloxanen, die zu verbesserten anwendungstechnischen Eigenschaften führen, wie beispielsweise einer verbesserten Phasenstabilität/Lagerstabilität und/oder einer besseren Griffbeurteilung des behandelten Textils. Häufig setzen sich Silikonquats unter alkalischen Bedingungen ab einem pH-Wert von größer oder gleich 9, wie er während oder nach dem Waschen der Gewebe oder Textilien vorliegen kann, auf dem Gewebe oder Textil ab. Dies kann zu einer Fleckenbildung führen, insbesondere wenn die Textilien oder Gewebe einem Färbeprozess unterzogen werden. Es besteht daher weiterhin der Bedarf an Siloxanen, die eine höhere pH-Stabilität aufweisen, insbesondere bis zu einem pH-Wert von 11. Es besteht insbesondere Bedarf an haut- und umweltfreundlichen griffgebenden Wirkstoffe oder Zusammensetzungen für die Ausrüstung von textilen Flächengebilden wie beispielsweise Baumwolle/Polyester oder Baumwolle/Polyamid/Elastan oder auch Vliesstoffen (sogenannte *non-wovens)* die aus Cellulose beziehungsweise Cellulose-Mischgeweben hergestellt werden.

Aufgabe der vorliegenden Erfindung war es daher, zumindest einen Nachteil des Standes der Technik zu überwinden.

Insbesondere bestand die Aufgabe darin, Siloxane/Silikone mit quartären/quaternären Ammoniumgruppen (sogenannte Silikonquats) bereitzustellen, welche deutlich reduzierte Anteile an unerwünschten organischen Nebenprodukten und/oder geringe Restgehalte von unerwünschten organischen Reaktanden aufweisen. Insbesondere bestand die Aufgabe der vorliegenden Erfindung darin, diese unerwünschten organischen Verbindungen zu minimieren bei gleichzeitiger Herstellung eines griffgebenden Wirkstoffes mit höherer Wirksamkeit auf dem Textil.

Überraschenderweise wurde gefunden, dass spezielle Siloxane und spezielle Zusammensetzungen, wie in den Ansprüchen beschrieben, mindestens einen Nachteil des Standes der Technik überwinden. Insbesondere wurde gefunden, dass diese speziellen Siloxane und speziellen Zusammensetzungen zu einer besseren Griffbeurteilung, einem besseren Phasenverhalten und/oder einem geringeren Amidamingehalt führen.

Diese speziellen Siloxane tragen mindestens zwei unterschiedliche quaternäre Ammoniumgruppen, wobei mindestens eine quaternäre Ammoniumgruppe ausgewählt ist aus der Gruppe bestehend aus quartären Amidammoniumgruppen und quartären Esterammoniumgruppen, vorzugsweise quartären Amidammoniumgruppen, und mindestens eine quaternäre Ammoniumgruppe ausgewählt ist aus der Gruppe bestehend aus quartären Dialkanolammoniumgruppen.

Die speziellen Zusammensetzungen wiederum enthalten diese speziellen Siloxane.

Gelöst wird die Aufgabe der vorliegenden Erfindung daher durch die Gegenstände der unabhängigen Ansprüche. Vorteilhafte Ausgestaltungen der Erfindung sind in den nachgeordneten Ansprüchen, den Beispielen und der Beschreibung angegeben.

Die erfindungsgemäßen Siloxane, also die erfindungsgemäßen griffgebenden Wirkstoffe, die erfindungsgemäße Zusammensetzung, das erfindungsgemäße Verfahren sowie die erfindungsgemäße Verwendung der Zusammensetzungen und/oder der Verfahrensprodukte werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Jede Ausführungsform, die durch Kombination von Bereichen/Teilbereichen und/oder Gruppen/Teilgruppen erhalten werden kann, wie beispielsweise durch Kombinationen von erfindungsgemäßen, wesentlichen, optionalen, bevorzugten, vorzugsweisen bzw. vorzugsweise ausgewählten, weiter bevorzugten, noch weiter bevorzugten, besonders bevorzugten oder insbesondere bevorzugten Bereichen/Teilbereichen und/oder Gruppen/Teilgruppen, gehört vollständig zum Offenbarungsgehalt der vorliegenden Erfindung und gilt als explizit, unmittelbar und eindeutig offenbart. Die Ausdrücke "vorzugsweise" und "bevorzugt" werden synonym verwendet.

Werden im Rahmen der vorliegenden Beschreibung Dokumente zitiert, so soll deren Inhalt vollständig zum Offenbarungsgehalt der vorliegenden Erfindung gehören.

Werden nachfolgend Gehaltsangaben (ppm oder %) gemacht, so handelt es sich, wenn nicht anders angegeben, um Angaben in Gewichts-% oder Gewichts-ppm (wppm). Bei Zusammensetzungen beziehen sich die Gehaltsangaben, wenn nicht anders angegeben, auf die Gesamtzusammensetzung. Werden nachfolgend Mittelwerte angegeben, so handelt es sich, wenn nicht anders angegeben, um Zahlenmittel. Werden Molmassen verwendet, so handelt es sich, wenn nicht ausdrücklich anders vermerkt, um gewichtsmittlere Molmassen Mw. Werden nachfolgend Messwerte oder Stoffeigenschaften, wie z. B. Oberflächenspannungen oder ähnliches, angegeben, so handelt es sich, wenn nicht anders angegeben, um Messwerte oder Stoffeigenschaften gemessen bei 25 °C sowie vorzugsweise bei einem Druck von

101325 Pa (Normaldruck). Werden im Umfang dieser Erfindung Werte für Viskositäten angegeben, handelt es sich, falls nicht anders vermerkt, um dynamische Viskositäten, die mit den dem Fachmann geläufigen Methoden ermittelt werden können.

Werden nachfolgend Zahlenbereiche in der Form "von X bis Y" angegeben, wobei X und Y die Grenzen des Zahlenbereichs darstellen, so ist dies gleichbedeutend mit der Angabe "von mindestens X bis einschließlich Y", sofern nicht explizit anders angegeben. Bereichsangaben schließen die Bereichsgrenzen X und Y also mit ein, sofern nicht explizit anders angegeben.

Wo immer Moleküle beziehungsweise Molekülfragmente ein oder mehrere Stereozentren aufweisen oder aufgrund von Symmetrien in Isomere unterschieden werden können oder aufgrund anderer Effekte, z.B. eingeschränkter Rotation, in Isomere unterschieden werden können, sind alle möglichen Isomere von der vorliegenden Erfindung mit eingeschlossen.

Das Wortfragment "Poly" umfasst im Zusammenhang mit dieser Erfindung nicht nur ausschließlich Verbindungen mit zumindest 2, insbesondere 3 Wiederholungseinheiten eines oder mehrerer Monomere im Molekül, sondern vorzugsweise auch solche Zusammensetzungen von Verbindungen, die eine Molekulargewichtsverteilung aufweisen und dabei ein mittleres Molekulargewicht von mindestens 200 g/mol besitzen. Bei dieser Definition ist dem Umstand Rechnung getragen, dass es auf dem betrachteten Gebiet der Technik üblich ist, solche Verbindungen bereits als Polymere zu bezeichnen, auch wenn sie nicht einer Polymerdefinition analog OECD- oder REACH-Richtlinien zu genügen scheinen.

Die verschiedenen Fragmente in den nachfolgenden Formeln (I), (IV), (V) und (VI) können statistisch verteilt sein. Statistische Verteilungen können blockweise aufgebaut sein mit einer beliebigen Anzahl an Blöcken und einer beliebigen Sequenz oder sie können einer randomisierten Verteilung unterliegen, sie können auch alternierend aufgebaut sein oder auch über die Kette, sofern ein solche vorliegt, einen Gradienten bilden, insbesondere können sie auch alle Mischformen bilden, bei denen gegebenenfalls Gruppen unterschiedlicher Verteilungen aufeinander folgen können. Die in den Formeln verwendeten Indizes a1, a2, a3, a4, b1, b2, b3, c1, c4, d, m, v, w, x, y, a5 und b5 sind natürliche Zahlen. Die Alkylenoxy-Einheiten in Formel (IV) können unterschiedlich an die benachbarten Gruppen oder Atome gebunden sein, das heißt in Formel (IV) steht

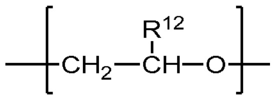

jeweils unabhängig voneinander für einen Alkylenoxyrest der Form $[CH_2CH(R^{12})O]$ und/oder der Form $[CH(R^{12})CH_2O]$, vorzugsweise aber für einen Alkylenoxyrest der Form $[CH_2CH(R^{12})O]$, Spezielle Ausführungen können im Folgenden so definiert sein, dass Merkmale wie Indizes oder Strukturbestandteile oder Bereiche oder statistische Verteilungen durch die Ausführung Beschränkungen erfahren. Alle anderen Merkmale, die nicht von der Beschränkung betroffen sind, bleiben unverändert.

Ein erster Gegenstand der vorliegenden Erfindung ist ein Siloxan (A) gemäß der Formel (I),

$$M^1_{a1}M^2_{a2}M^3_{a3}M^4_{a4}D^1_{b1}D^2_{b2}D^3_{b3}T^1_{c1}T^4_{c4}Q_d \qquad \text{Formel (I),}$$

mit

$M^1$ = $[R^1{}_3SiO_{1/2}]$;
$M^2$ = $[R^2R^1{}_2SiO_{1/2}]$;
$M^3$ = $[R^3R^1{}_2SiO_{1/2}]$;
$M^4$ = $[R^4R^1{}_2SiO_{1/2}]$;
$D^1$ = $[R^1{}_2SiO_{2/2}]$;
$D^2$ = $[R^1R^2SiO_{2/2}]$;
$D^3$ = $[R^1R^3SiO_{2/2}]$;
$T^1$ = $[R^1SiO_{3/2}]$;
$T^4$ = $[R^4SiO_{3/2}]$;
Q = $[SiO_{4/2}]$;

a1 = 0 bis 32, bevorzugt 0 bis 19, insbesondere 0 bis 12;
a2 = 0 bis 32, bevorzugt 1 bis 10, insbesondere 1 bis 3;
a3 = 0 bis 32, bevorzugt 1 bis 10, insbesondere 1 bis 2;
a4 = 0 bis 6, bevorzugt 0 bis 1, insbesondere 0;

b1 = 1 bis 1000, bevorzugt 5 bis 500, insbesondere 10 bis 400;
b2 = 0 bis 10, bevorzugt 0 bis 5, insbesondere 0;
b3 = 0 bis 10, bevorzugt 0 bis 5, insbesondere 0;
c1 = 0 bis 10, bevorzugt 0 bis 5, insbesondere 0 bis 4;
c4 = 0 bis 5, bevorzugt 0 bis 2, insbesondere 0;
d = 0 bis 10, bevorzugt 0 bis 5, insbesondere 0 bis 4;

$R^1$ = jeweils unabhängig voneinander gleiche oder verschiedene Kohlenwasserstoffreste, vorzugsweise mit 1 bis 30 Kohlenstoffatomen, weiter bevorzugt Alkylreste mit 1 bis 30 Kohlenstoffatomen oder aromatische Kohlenwasserstoffreste mit 6 bis 30 Kohlenstoffatomen, noch weiter bevorzugt Alkylreste mit 1 bis 14 Kohlenstoffatomen oder monocyclische aromatische Kohlenwasserstoffreste, wobei die Alkylreste vorzugsweise linear oder verzweigt, gesättigt oder ungesättigt sind, noch weiter bevorzugt Methyl, Ethyl, Propyl oder Phenyl, insbesondere Methyl;

$R^2$ = $R^{21}$-$R^{22}$;

$R^{21}$ = jeweils unabhängig voneinander gleiche oder verschiedene zweiwertige Kohlenwasserstoffreste mit mindestens einer Hydroxylgruppe und gegebenenfalls weiteren Sauerstoffatomen und vorzugsweise 2 bis 30 Kohlenstoffatomen, weiter bevorzugt zusätzlich enthaltend 1 bis 2 weitere Sauerstoffatome, noch weiter bevorzugt enthaltend funktionelle Gruppen ausgewählt aus Ether-, Carbonyl- und Estergruppen, noch weiter bevorzugt jeweils unabhängig voneinander gleiche oder verschiedene zweiwertige Reste ausgewählt aus der Gruppe bestehend aus

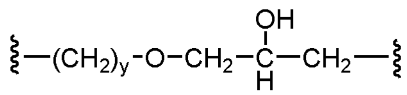
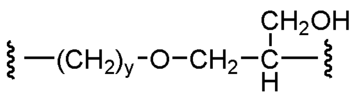

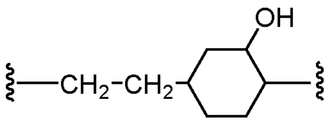
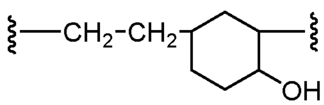

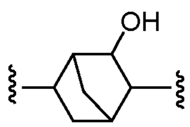
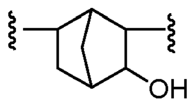

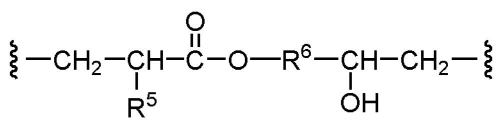
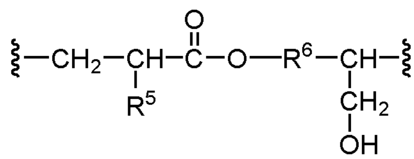
.

insbesondere jeweils unabhängig voneinander gleiche oder verschiedene zweiwertige Reste ausgewählt aus der Gruppe bestehend aus

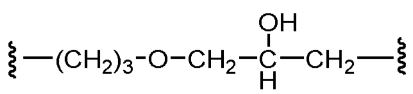
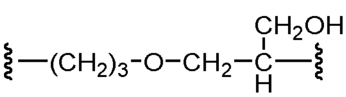
;

$R^{22}$ = jeweils unabhängig voneinander gleiche oder verschiedene Reste der Formel (II),

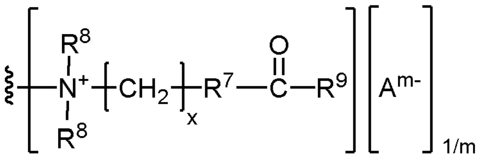 Formel (II);

$R^3$ = $R^{31}$-$R^{32}$;

$R^{31}$ = $R^{21}$;

$R^{32}$ = jeweils unabhängig voneinander gleiche oder verschiedene Reste der Formel (III),

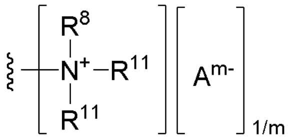 Formel (III)

$R^4$ = jeweils unabhängig voneinander gleiche oder verschiedene Alkoxygruppen oder Acyloxygruppen, vorzugsweise mit 1 bis 6 Kohlenstoffatomen, weiter bevorzugt Acetoxygruppen und/oder Methoxy-, Ethoxy-, n-Propoxy-, iso-Propoxy-, n-Butoxy-, tert-Butoxy-Gruppen und/oder Alkoxygruppen, abgeleitet von Glykolresten, beispielsweise Propylenglykol, Dipropylenglykol, Tripropylenglykol, Hexylenglykol, Pentylenglykol, Butyldiglykol, insbesondere iso-Propoxy-Gruppen;

$R^5$ = jeweils unabhängig voneinander gleiche oder verschiedene Reste ausgewählt aus der Gruppe bestehend aus Wasserstoff und Kohlenwasserstoffresten, vorzugsweise mit 1 bis 6 Kohlenstoffatomen, weiter bevorzugt ausgewählt aus der Gruppe bestehend aus Alkylresten mit 1 bis 6 Kohlenstoffatomen, wobei die Alkylreste vorzugsweise linear oder verzweigt, gesättigt oder ungesättigt sind, insbesondere Methyl;

$R^6$ = jeweils unabhängig voneinander gleiche oder verschiedene zweiwertige Kohlenwasserstoffreste, die optional Ethergruppen enthalten, vorzugsweise mit 1 bis 6 Kohlenstoffatomen, bevorzugt Methylen;

$R^7$ = jeweils unabhängig voneinander gleiche oder verschiedene zweiwertige Reste ausgewählt aus der Gruppe bestehend aus -O- und -$NR^{10}$-, vorzugsweise -$NR^{10}$-;

$R^8$ =jeweils unabhängig voneinander gleiche oder verschiedene Reste ausgewählt aus der Gruppe bestehend aus Kohlenwasserstoffresten vorzugsweise mit 1 bis 30 Kohlenstoffatomen, weiter bevorzugt ausgewählt aus der Gruppe bestehend aus linearen oder verzweigten, gesättigten oder ungesättigten Alkylresten mit 1 bis 12 Kohlenstoffatomen, noch weiter bevorzugt jeweils unabhängig voneinander gleiche oder verschiedene Reste ausgewählt aus der Gruppe bestehend aus Methyl, Ethyl, n-Propyl, iso-Propyl, n-Butyl, iso-Butyl, tert.-Butyl, noch weiter bevorzugt jeweils unabhängig voneinander gleiche oder verschiedene Reste ausgewählt aus der Gruppe bestehend aus Methyl, Ethyl, n-Propyl, iso-Propyl, insbesondere Methyl;

$R^9$ =jeweils unabhängig voneinander gleiche oder verschiedene Reste ausgewählt aus der Gruppe bestehend aus Wasserstoff und Kohlenwasserstoffresten, vorzugsweise mit 1 bis 30 Kohlenstoffatomen, weiter bevorzugt ausgewählt aus der Gruppe bestehend aus Alkylresten mit 1 bis 30 Kohlenstoffatomen, noch weiter bevorzugt Alkylresten mit 12 bis 24 Kohlenstoffatomen, insbesondere mit 16 bis 22 Kohlenstoffatomen, wobei die Kohlenwasserstoffreste beziehungsweise Alkylreste vorzugsweise linear oder verzweigt, substituiert oder unsubstituiert gesättigt oder ungesättigt, besonders bevorzugt linear, unsubstituiert und gesättigt sind;

$R^{10}$ = jeweils unabhängig voneinander gleiche oder verschiedene Reste aus der Gruppe bestehend aus Wasserstoff, -C(=O)$R^9$ und Kohlenwasserstoffresten, vorzugsweise mit 1 bis 6 Kohlenstoffatomen, weiter bevorzugt Alkylreste mit 1 bis 6 Kohlenstoffatomen, wobei die Kohlenwasserstoffreste beziehungsweise Alkylreste vorzugsweise linear oder verzweigt, substituiert oder unsubstituiert, gesättigt oder ungesättigt, besonders bevorzugt linear, unsubstituiert und gesättigt sind, insbesondere bevorzugt ist $R^{10}$ Wasserstoff;

$R^{11}$ = jeweils unabhängig voneinander gleiche oder verschiedene Reste ausgewählt aus der Gruppe bestehend aus Kohlenwasserstoffresten, die mindestens eine Hydroxygruppe und vorzugsweise 1 bis 6 Kohlenstoffatome aufweisen, bevorzugt Alkylreste, die mindestens eine Hydroxygruppe und vorzugsweise 1 bis 6 Kohlenstoffatome aufweisen, wobei die Alkylreste vorzugsweise linear oder verzweigt, gesättigt oder ungesättigt sind, und Resten der Formel (IV)

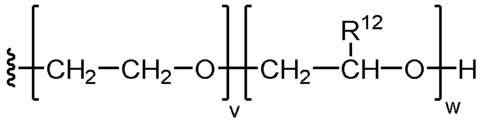

Formel (IV),

bevorzugt 2-Hydroxyethyl und/oder 2-Hydroxypropyl;

$R^{12}$ = jeweils unabhängig voneinander gleiche oder verschiedene Reste ausgewählt aus der Gruppe bestehend aus Kohlenwasserstoffresten, vorzugsweise mit 1 bis 6 Kohlenstoffatomen, weiter bevorzugt Alkylreste mit 1 bis 6 Kohlenstoffatomen, wobei die Alkylreste vorzugsweise linear oder verzweigt, gesättigt oder ungesättigt sind, vorzugsweise Methyl und Ethyl, insbesondere Methyl;

$A^{m-}$ = jeweils unabhängig voneinander gleiche oder verschiedene Anionen, ausgewählt aus anorganischen oder organischen Anionen der Säuren $H_mA$, sowie deren Derivate;

m = 1 bis 3, bevorzugt 1 bis 2, insbesondere 1;
v = 0 bis 30, bevorzugt 0 bis 10, insbesondere 1 bis 3;
w = 0 bis 30, bevorzugt 0 bis 10;
x = 2 bis 18, bevorzugt 3;
y = 2 bis 18, bevorzugt 3;

dadurch gekennzeichnet, dass die Maßgaben (i) und (ii) gelten:

(i) $a2 + b2 \geq 1$;

(ii) $a3 + b3 \geq 1$.

Die Maßgaben (i) und (ii) stellen dabei sicher, dass das Siloxan (A) mindestens eine Amidammoniumgruppe gemäß Formel (II) als auch mindestens eine Dialkanolammoniumgruppe gemäß Formel (III) aufweist, also jeweils mindestens einen Rest der folgenden Formeln (II) und (III):

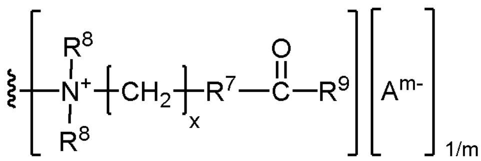

Formel (II)

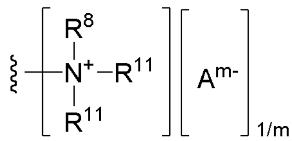

Formel (III).

Dabei sind die positiven Ladungen an den quartären Ammoniumgruppen durch eine entsprechende Anzahl von Gegenionen $A^{m-}$ ausgeglichen.

Ein bevorzugtes Siloxan (A) ist ein Siloxan (A) gemäß der Formel (I),

$$M^1_{a1}M^2_{a2}M^3_{a3}M^4_{a4}D^1_{b1}D^2_{b2}D^3_{b3}T^1_{c1}T^4_{c4}Q_d \qquad \text{Formel (I),}$$

mit

a1 = 0 bis 32, bevorzugt 0 bis 19, insbesondere 0 bis 12;
a2 = 0 bis 32, bevorzugt 1 bis 10, insbesondere 1 bis 3;

a3 = 0 bis 32, bevorzugt 1 bis 10, insbesondere 1 bis 2;
a4 = 0 bis 6, bevorzugt 0 bis 1, insbesondere 0;
b1 = 1 bis 1000, bevorzugt 5 bis 500, insbesondere 10 bis 400;
b2 = 0 bis 10, bevorzugt 0 bis 5, insbesondere 0;
b3 = 0 bis 10, bevorzugt 0 bis 5, insbesondere 0;
c1 = 0 bis 10, bevorzugt 0 bis 5, insbesondere 0 bis 4;
c4 = 0 bis 5, bevorzugt 0 bis 2, insbesondere 0;
d = 0 bis 10, bevorzugt 0 bis 5, insbesondere 0 bis 4;

$R^1$ = jeweils unabhängig voneinander gleiche oder verschiedene Kohlenwasserstoffreste mit 1 bis 30 Kohlenstoffatomen, bevorzugt ausgewählt aus der Gruppe bestehend aus Methyl, Ethyl, Propyl oder Phenyl, insbesondere Methyl;

$R^2$ = $R^{21}$-$R^{22}$;

$R^{21}$ = jeweils unabhängig voneinander gleiche oder verschiedene zweiwertige Kohlenwasserstoffreste mit mindestens einer Hydroxylgruppe und gegebenenfalls 1 bis 2 weiteren Sauerstoffatomen und 2 bis 30 Kohlenstoffatomen, bevorzugt jeweils unabhängig voneinander gleiche oder verschiedene zweiwertige Reste ausgewählt aus der Gruppe bestehend aus

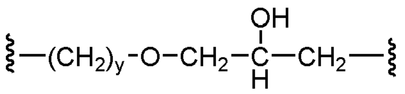 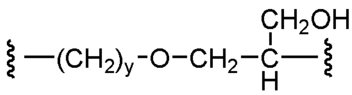

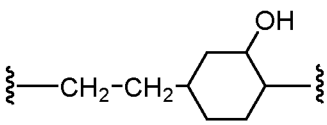 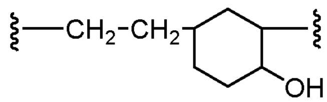

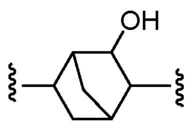 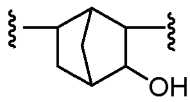

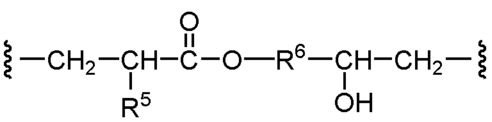 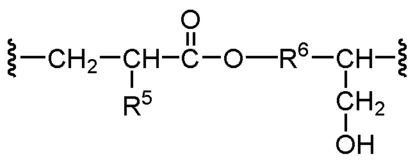 ,

insbesondere jeweils unabhängig voneinander gleiche oder verschiedene zweiwertige Reste ausgewählt aus der Gruppe bestehend aus

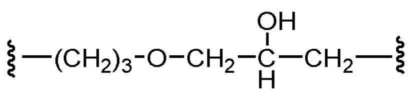 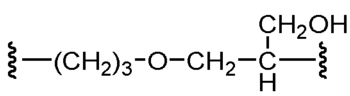 ;

$R^{22}$ = jeweils unabhängig voneinander gleiche oder verschiedene Reste der Formel (II),

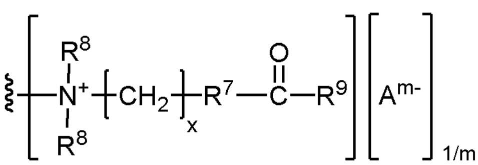 Formel (II);

$R^3$ = $R^{31}$-$R^{32}$;

$R^{31}$ = $R^{21}$;

$R^{32}$ = jeweils unabhängig voneinander gleiche oder verschiedene Reste der Formel (III),

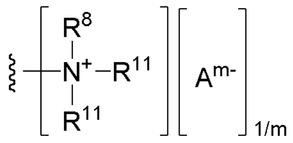

Formel (III)

$R^4$ = jeweils unabhängig voneinander gleiche oder verschiedene Alkoxygruppen oder Acyloxygruppen mit 1 bis 6 Kohlenstoffatomen, bevorzugt Acetoxygruppen und/oder Methoxy-, Ethoxy-, n-Propoxy-, iso-Propoxy-, n-Butoxy-, tert-Butoxy-Gruppen, insbesondere iso-Propoxy-Gruppen;

$R^5$ = jeweils unabhängig voneinander gleiche oder verschiedene Reste ausgewählt aus der Gruppe bestehend aus Wasserstoff und Kohlenwasserstoffresten mit 1 bis 6 Kohlenstoffatomen, bevorzugt ausgewählt aus der Gruppe bestehend aus Alkylresten mit 1 bis 6 Kohlenstoffatomen, wobei die Alkylreste linear oder verzweigt, gesättigt oder ungesättigt sind, insbesondere Methyl;

$R^6$ = jeweils unabhängig voneinander gleiche oder verschiedene zweiwertige Kohlenwasserstoffreste, die optional Ethergruppen enthalten, mit 1 bis 6 Kohlenstoffatomen, bevorzugt und insbesondere Methylen;

$R^7$ = jeweils unabhängig voneinander gleiche oder verschiedene zweiwertige Reste ausgewählt aus der Gruppe bestehend aus -O- und -$NR^{10}$-, bevorzugt und insbesondere -$NR^{10}$-;

$R^8$ =jeweils unabhängig voneinander gleiche oder verschiedene Reste ausgewählt aus der Gruppe bestehend aus Kohlenwasserstoffresten mit 1 bis 30 Kohlenstoffatomen, bevorzugt jeweils unabhängig voneinander gleiche oder verschiedene Reste ausgewählt aus der Gruppe bestehend aus Methyl, Ethyl, n-Propyl, iso-Propyl, insbesondere Methyl;

$R^9$ =jeweils unabhängig voneinander gleiche oder verschiedene Reste ausgewählt aus der Gruppe bestehend aus Kohlenwasserstoffresten mit 1 bis 30 Kohlenstoffatomen, bevorzugt jeweils unabhängig voneinander gleiche oder verschiedene Reste ausgewählt aus der Gruppe bestehend aus Alkylresten mit 12 bis 24 Kohlenstoffatomen, insbesondere mit 16 bis 22 Kohlenstoffatomen;

$R^{10}$ = jeweils unabhängig voneinander gleiche oder verschiedene Reste aus der Gruppe bestehend aus Wasserstoff, -C(=O)$R^9$ und Kohlenwasserstoffresten mit 1 bis 6 Kohlenstoffatomen, bevorzugt ausgewählt aus der Gruppe bestehend aus Wasserstoff und Alkylresten mit 1 bis 6 Kohlenstoffatomen, insbesondere Wasserstoff;

$R^{11}$ = jeweils unabhängig voneinander gleiche oder verschiedene Reste ausgewählt aus der Gruppe bestehend aus Kohlenwasserstoffresten, die mindestens eine Hydroxygruppe und 1 bis 6 Kohlenstoffatome aufweisen, und Resten der Formel (IV)

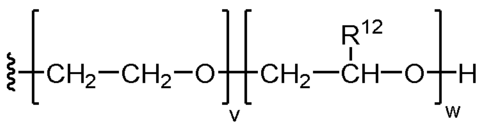

Formel (IV),

bevorzugt und insbesondere 2-Hydroxyethyl und/oder 2-Hydroxypropyl;

$R^{12}$ = jeweils unabhängig voneinander gleiche oder verschiedene Reste ausgewählt aus der Gruppe bestehend aus Kohlenwasserstoffresten mit 1 bis 6 Kohlenstoffatomen, bevorzugt Methyl und Ethyl, insbesondere Methyl;

$A^{m-}$ = jeweils unabhängig voneinander gleiche oder verschiedene Anionen, ausgewählt aus anorganischen oder organischen Anionen der Säuren $H_mA$, sowie deren Derivate;

m = 1 bis 3, bevorzugt und insbesondere 1 bis 2;

v = 0 bis 30, bevorzugt und insbesondere 0 bis 10;

w = 0 bis 30, bevorzugt und insbesondere 0 bis 10;

x = 2 bis 18, bevorzugt und insbesondere 3;

y = 2 bis 18, bevorzugt und insbesondere 3;

dadurch gekennzeichnet, dass die Maßgaben (i) und (ii) gelten:

(i)

$$a2 + b2 \geq 1;$$

(ii)

$$a3 + b3 \geq 1.$$

Ein weiter bevorzugtes Siloxan (A) ist ein Siloxan (A) gemäß der Formel (I),

$$M^1_{a1}M^2_{a2}M^3_{a3}M^4_{a4}D^1_{b1}D^2_{b2}D^3_{b3}T^1_{c1}T^4_{c4}Q_d \qquad \text{Formel (I),}$$

mit

a1 = 0 bis 32, bevorzugt 0 bis 19, insbesondere 0 bis 12;
a2 = 0 bis 32, bevorzugt 1 bis 10, insbesondere 1 bis 3;
a3 = 0 bis 32, bevorzugt 1 bis 10, insbesondere 1 bis 2;
a4 = 0 bis 6, bevorzugt 0 bis 1, insbesondere 0;
b1 = 1 bis 1000, bevorzugt 5 bis 500, insbesondere 10 bis 400;
b2 = 0 bis 10, bevorzugt 0 bis 5, insbesondere 0;
b3 = 0 bis 10, bevorzugt 0 bis 5, insbesondere 0;
c1 = 0 bis 10, bevorzugt 0 bis 5, insbesondere 0 bis 4;
c4 = 0 bis 5, bevorzugt 0 bis 2, insbesondere 0;
d = 0 bis 10, bevorzugt 0 bis 5, insbesondere 0 bis 4;
$R^1$ = jeweils unabhängig voneinander gleich oder verschieden Methyl, Ethyl, Propyl oder Phenyl, insbesondere Methyl;
$R^2$ = $R^{21}$-$R^{22}$;
$R^{21}$ = jeweils unabhängig voneinander gleiche oder verschiedene zweiwertige Reste ausgewählt aus der Gruppe bestehend aus

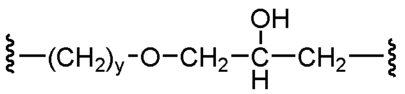 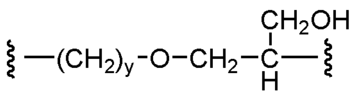

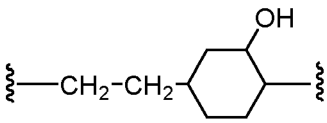 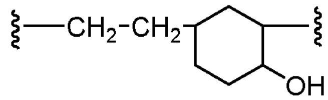

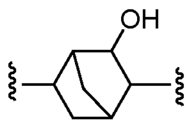 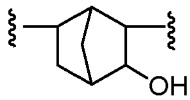

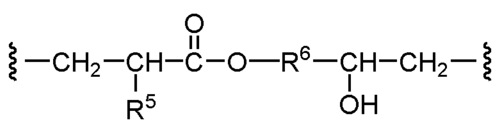 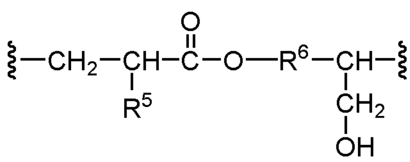 ,

insbesondere jeweils unabhängig voneinander gleiche oder verschiedene zweiwertige Reste ausgewählt aus der Gruppe bestehend aus

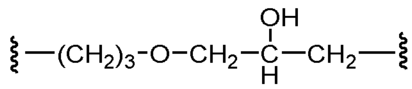 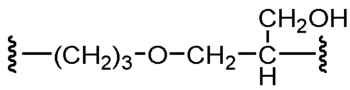 ;

$R^{22}$ = jeweils unabhängig voneinander gleiche oder verschiedene Reste der Formel (II),

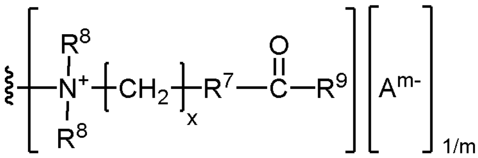

Formel (II);

$R^3$ = $R^{31}$-$R^{32}$;

$R^{31}$ = $R^{21}$;

$R^{32}$ = jeweils unabhängig voneinander gleiche oder verschiedene Reste der Formel (III),

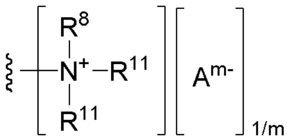

Formel (III)

$R^4$ = jeweils unabhängig voneinander gleiche oder verschiedene Reste ausgewählt aus der Gruppe bestehend aus Acetoxy, Methoxy, Ethoxy, n-Propoxy, iso-Propoxy, n-Butoxy und tert-Butoxy, insbesondere iso-Propoxy;

$R^5$ = jeweils unabhängig voneinander gleiche oder verschiedene Reste ausgewählt aus Wasserstoff und Methyl;

$R^6$ = Methylen;

$R^7$ = jeweils unabhängig voneinander gleiche oder verschiedene zweiwertige Reste ausgewählt aus der Gruppe bestehend aus -O- und -$NR^{10}$-, insbesondere -$NR^{10}$-;

$R^8$ = Methyl;

$R^9$ =jeweils unabhängig voneinander gleiche oder verschiedene Reste ausgewählt aus der Gruppe bestehend aus Alkylresten mit 12 bis 24 Kohlenstoffatomen, insbesondere mit 16 bis 22 Kohlenstoffatomen;

$R^{10}$ = Wasserstoff;

$R^{11}$ = jeweils unabhängig voneinander gleiche oder verschiedene Alkylreste, die mindestens eine Hydroxygruppe und 1 bis 6 Kohlenstoffatome aufweisen, insbesondere 2-Hydroxyethyl und/oder 2-Hydroxypropyl;

$R^{12}$ = jeweils unabhängig voneinander gleiche oder verschiedene Alkylreste mit 1 bis 6 Kohlenstoffatomen, insbesondere Methyl;

$A^{m-}$ = jeweils unabhängig voneinander gleiche oder verschiedene Anionen, ausgewählt aus anorganischen oder organischen Anionen der Säuren $H_mA$, sowie deren Derivate;

m = 1 bis 3, bevorzugt 1 bis 2, insbesondere 1;

v = 0 bis 30, bevorzugt 0 bis 10, insbesondere 1 bis 3;

w = 0 bis 30, bevorzugt und insbesondere 0 bis 10;

x = 2 bis 18, bevorzugt und insbesondere 3;

y = 2 bis 18, bevorzugt und insbesondere 3;

dadurch gekennzeichnet, dass die Maßgaben (i) und (ii) gelten:

(i)

$$a2 + b2 \geq 1;$$

(ii)

$$a3 + b3 \geq 1.$$

Ein noch weiter bevorzugtes Siloxan (A) ist ein Siloxan (A) gemäß der Formel (I),

$$M^1{}_{a1}M^2{}_{a2}M^3{}_{a3}M^4{}_{a4}D^1{}_{b1}D^2{}_{b2}D^3{}_{b3}T^1{}_{c1}T^4{}_{c4}Q_d \qquad \text{Formel (I),}$$

mit

- a1 = 0 bis 32, bevorzugt 0 bis 19, insbesondere 0 bis 12;
- a2 = 0 bis 32, bevorzugt 1 bis 10, insbesondere 1 bis 3;
- a3 = 0 bis 32, bevorzugt 1 bis 10, insbesondere 1 bis 2;
- a4 = 0 bis 6, bevorzugt 0 bis 1, insbesondere 0;
- b1 = 1 bis 1000, bevorzugt 5 bis 500, insbesondere 10 bis 400;
- b2 = 0 bis 10, bevorzugt 0 bis 5, insbesondere 0;
- b3 = 0 bis 10, bevorzugt 0 bis 5, insbesondere 0;
- c1 = 0 bis 10, bevorzugt 0 bis 5, insbesondere 0 bis 4;
- c4 = 0 bis 5, bevorzugt 0 bis 2, insbesondere 0;
- d = 0 bis 10, bevorzugt 0 bis 5, insbesondere 0 bis 4;
- $R^1$ = jeweils unabhängig voneinander gleich oder verschieden Methyl, Ethyl, Propyl oder Phenyl, insbesondere Methyl;
- $R^2$ = $R^{21}$-$R^{22}$;
- $R^{21}$ = jeweils unabhängig voneinander gleiche oder verschiedene zweiwertige Reste ausgewählt aus der Gruppe bestehend aus

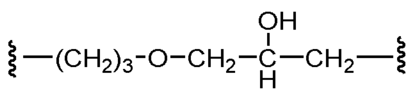

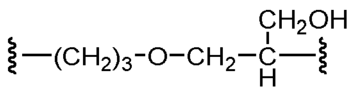

:

- $R^{22}$ = jeweils unabhängig voneinander gleiche oder verschiedene Reste der Formel (II),

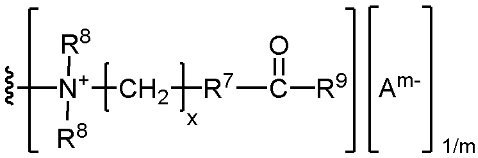

Formel (II);

- $R^3$ = $R^{31}$-$R^{32}$;
- $R^{31}$ = $R^{21}$;
- $R^{32}$ = jeweils unabhängig voneinander gleiche oder verschiedene Reste der Formel (III),

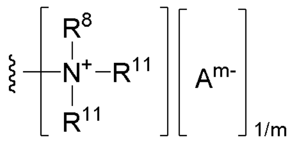

Formel (III)

- $R^4$ = jeweils unabhängig voneinander gleiche oder verschiedene Reste ausgewählt aus der Gruppe bestehend aus Acetoxy, Methoxy, Ethoxy, n-Propoxy, iso-Propoxy, n-Butoxy und tert-Butoxy, insbesondere iso-Propoxy;
- $R^7$ = -NH-;
- $R^8$ = Methyl;
- $R^9$ = jeweils unabhängig voneinander gleiche oder verschiedene Reste ausgewählt aus der Gruppe bestehend aus Alkylresten mit 16 bis 22 Kohlenstoffatomen;
- $R^{11}$ = jeweils unabhängig voneinander gleiche oder verschiedene Alkylreste, die mindestens eine Hydroxygruppe und 1 bis 6 Kohlenstoffatome aufweisen, insbesondere 2-Hydroxyethyl und/oder 2-Hydroxypropyl;
- $A^{m-}$ = jeweils unabhängig voneinander gleiche oder verschiedene Anionen, ausgewählt aus anorganischen oder organischen Anionen der Säuren $H_mA$, sowie deren Derivate;

m = 1 bis 3, bevorzugt 1 bis 2, insbesondere 1;

x = 2 bis 18, bevorzugt und insbesondere 3;

dadurch gekennzeichnet, dass die Maßgaben (i) und (ii) gelten:

(i)

$$a2 + b2 \geq 1;$$

(ii)

$$a3 + b3 \geq 1.$$

In einer bevorzugten Ausführungsform ist das Siloxan (A) weiterhin dadurch gekennzeichnet, dass zudem entweder die Maßgabe (iii) oder die Maßgabe (iv) gilt:

(iii)

$$a1 = a4 = b2 = b3 = c1 = c4 = d = 0$$

und

$$a2 = a3 = 1;$$

(iv)

$$b2 = b3 = 0$$

und

$$c1 + c4 + d \geq 1$$

und
$a2 + a3 + a4 \geq 3$, vorzugsweise $a2 \geq 2$, $a3 \geq 1$ und $a4 = 0$;

Bei einem Siloxan (A), das die Maßgabe (iii) erfüllt, handelt es sich um ein lineares Siloxan ohne seitenständige Siloxangruppen, da es keine $T^1$-, $T^4$- oder Q-Einheiten aufweist. Da das Siloxan zudem keine $M^1$-Einheit und keine $M^4$-Einheit aufweist, aber genau eine $M^2$ und genau eine $M^3$-Einheit trägt, weist dieses unverzweigte Siloxan an einem der beiden Enden der Siloxankette genau eine Amidammoniumgruppe gemäß Formel (II) und an dem anderen der beiden Kettenenenden genau eine Dialkanolammoniumgruppe gemäß Formel (III) auf. Da besagtes Siloxan (A) zudem weder $D^2$- noch $D^3$-Einheiten aufweist, trägt das Siloxan keine seitenständig gebundenen Amidammoniumgruppen gemäß Formel (II) oder Dialkanolammoniumgruppen gemäß Formel (III).

Bei einem Siloxan (A), für das die Maßgabe (iv) gilt, handelt es sich wiederum um ein verzweigtes Siloxan, da es zumindest eine Einheit ausgewählt aus der Gruppe bestehend aus $T^1$-, $T^4$- und Q-Einheiten aufweist. Dieses verzweigte Siloxan trägt an mindestens einem seiner mindestens drei Enden eine Amidammoniumgruppe gemäß Formel (II) und an mindestens einem anderen seiner mindestens drei Enden eine Dialkanolammoniumgruppe gemäß Formel (III). Da das Siloxan zudem weder $D^2$- noch $D^3$-Einheiten aufweist, können Amidammoniumgruppen gemäß Formel (II) oder Dialkanolammoniumgruppen gemäß Formel (III) nur an den mindestens drei Enden dieses verzweigten Siloxans vorliegen. Des Weiteren kann das Siloxan noch einen oder mehrere Reste $R^4$ tragen, also jeweils unabhängig voneinander gleiche oder verschiedene Alkoxygruppen oder Acyloxygruppen, Falls eine $M^4$-Einheit vorliegt, so ist mindestens ein Rest $R^4$ an den Enden des verzweigten Siloxans gebunden. Falls eine $T^4$-Einheit vorliegt, so ist mindestens ein Rest $R^4$ an einer T-Verzweigungsstelle gebunden. Bevorzugt weist das Siloxan, für das die Maßgabe (iv) erfüllt ist, aber keine $M^4$-Einheiten auf, es gilt bevorzugt also $a4 = 0$. Weiter bevorzugt weist das Siloxan, für das die Maßgabe (iv) erfüllt ist,

weiterhin genau zwei $M^2$-Einheiten und genau eine $M^3$-Einheit auf. Somit trägt dieses Siloxan an genau zwei seiner mindestens drei Enden eine Amidammoniumgruppe gemäß Formel (II) und an genau einem seiner mindestens drei Enden eine Dialkanolammoniumgruppe gemäß Formel (III). Es ist insbesondere bevorzugt, dass keine $T^4$-Einheiten vorliegen, es gilt also insbesondere bevorzugt c4 = 0. Das Siloxan, für das die Maßgabe (iv) erfüllt ist, kann weiterhin $M^1$-Einheiten aufweisen. Es ist aber bevorzugt, dass keine $M^1$-Einheiten vorliegen, es gilt also bevorzugt a1= 0.

Ein weiterer Gegenstand der Erfindung ist eine Zusammensetzung, die mindestens ein Siloxan (A) umfasst.

Die erfindungsgemäßen Zusammensetzungen können in bevorzugten Ausführungsformen entweder ein oder mehrere Siloxane (A), für die die Maßgabe (iii) gilt, oder aber ein oder mehrere Siloxane (A), für die die Maßgabe (iv) gilt, enthalten, sie können aber auch Mischungen aus ihnen enthalten.

Bevorzugt umfasst die erfindungsgemäße Zusammensetzung weiterhin mindestens ein Siloxan ausgewählt aus der Gruppe bestehend aus Siloxanen (B) und Siloxanen (C).

Siloxan (B) ist ein Siloxan, das sich zumindest darin, vorzugsweise genau darin, von einem Siloxan (A) unterscheidet, dass die folgenden Maßgaben (v) und (vi) anstelle der oben aufgeführten Maßgaben (i) bis (iv) gelten:

(v)

$$a2 = b2 = 0,$$

(vi)

$$a3 + b3 \geq 2.$$

Siloxan (B) ist also ein Siloxan, das sich zumindest darin, vorzugsweise genau darin, von einem Siloxan (A) unterscheidet, das es mindestens zwei Dialkanolammoniumgruppen gemäß Formel (III) und keine Amidammoniumgruppen gemäß Formel (II) aufweist.

Siloxan (C) ist ein Siloxan, das sich zumindest darin, vorzugsweise genau darin, von einem Siloxan (A) unterscheidet, dass die folgenden Maßgaben (vii) und (viii) anstelle der oben aufgeführten Maßgaben (i) und (iv) gelten:

(vii)

$$a3 = b3 = 0 ,$$

(viii)

$$a2 + b2 \geq 2.$$

Siloxan (C) ist also ein Siloxan, das sich zumindest darin, vorzugsweise genau darin, von einem Siloxan (A) unterscheidet, das es mindestens zwei Amidammoniumgruppen gemäß Formel (II) und keine Dialkanolammoniumgruppen gemäß Formel (III) aufweist.

Somit unterscheiden sich auch die Siloxane (B) und (C) voneinander. Entsprechend unterscheiden sich die Siloxane (A), (B) und (C) voneinander.

In einer bevorzugten Ausführungsform gilt für das Siloxan (A): $R^8$ = Methyl, x = 3, $R^7$= $-NR^{10}-$ mit $R^{10}$ = H.

In einer alternativen bevorzugten Ausführungsform gilt für das Siloxan (A): $R^8$ = Methyl, $R^{11}$ = $-CH_2CH_2OH$ und/oder $-CH_2CH(CH_3)OH$.

In einer besonders bevorzugten Ausführungsform gilt für das Siloxan (A): $R^8$ = Methyl, x = 3, $R^7$= $-NR^{10}-$ mit $R^{10}$ = H, $R^{11}$ = $-CH_2CH_2OH$ und/oder $-CH_2CH(CH_3)OH$.

Weiterhin bevorzugt gilt für das Siloxan (B): $R^8$ = Methyl, $R^{11}$ = $-CH_2CH_2OH$ oder $-CH_2CH(CH_3)OH$

Weiterhin bevorzugt gilt für das Siloxan (C): $R^8$ = Methyl, x = 3, $R^7$= $-NR^{10}-$ mit $R^{10}$ = H.

Insbesondere bevorzugt sind $R^8$, x, $R^7$ und $R^{10}$ für die Siloxane (A) und (B) sowie $R^8$ und $R^{11}$ für die Siloxane (A) und (C) gleich.

Vorzugsweise beträgt der Massenanteil des mindestens einen Siloxans (A) bezogen auf die Gesamtmasse aller Siloxane, besonders bevorzugt bezogen auf die Masse der Siloxane (A) und (B) und (C) zusammen, von 20% bis 70%, bevorzugt von 25% bis 60%, insbesondere von 30% bis 50%.

In einer bevorzugten Ausführungsform beträgt der Massenanteil des mindestens einen Siloxans (B) bezogen

auf die Gesamtmasse der Siloxane, besonders bevorzugt bezogen auf die Masse der Siloxane (A) und (B) und (C) zusammengenommen, von 0% bis 15%, bevorzugt von 1% bis 10%.

Bevorzugt beträgt der Massenanteil des mindestens einen Siloxans (C) bezogen auf die Gesamtmasse der Siloxane, besonders bevorzugt bezogen auf die Masse der Siloxane (A) und (B) und (C) zusammengenommen, von 3% bis 80%, bevorzugt von 5% bis 60%, insbesondere von 10% bis 50%.

Herstellungsbedingt ist es möglich, dass die erfindungsgemäße Zusammensetzung tertiäre Amine enthält. Es aber bevorzugt, dass der Anteil an hautreizenden, sensibilisierenden und/oder aquatoxischen tertiären Aminen gering ist.

Es ist daher weiterhin bevorzugt, dass der Massenanteil von tertiären Aminen ausgewählt aus der Gruppe der Esteramine und Amidamine in der Zusammensetzung bezogen auf die Gesamtmasse der Siloxane (A) und (B) und (C) zusammengenommen in der Summe weniger als 1%, bevorzugt weniger als 0,8%, weiter bevorzugt weniger als 0,6%, noch weiter bevorzugt weniger als 0,4%, insbesondere von 0% bis 0,3% beträgt, oder die Zusammensetzung keine tertiären Aminen ausgewählt aus der Gruppe der Amidamine enthält.

Unter einem "Amidamin" wird im Rahmen der vorliegenden Offenbarung ein Carbonsäurealkylamid verstanden, dass mindestens eine, vorzugsweise genau eine tertiäre Aminogruppe aufweist. Ein Amidamin gehört im Rahmen der vorliegenden Offenbarung also zur Gruppe der tertiären Amine.

Unter einem "Esteramin" wird im Rahmen der vorliegenden Offenbarung ein Carbonsäurealkylester verstanden, der mindestens eine, vorzugsweise genau eine tertiäre Aminogruppe aufweist. Ein Esteramin gehört im Rahmen der vorliegenden Offenbarung also zur Gruppe der tertiären Amine.

Eine "quartäre Ammoniumgruppe" wird im Rahmen der vorliegenden Offenbarung auch als "quatäre Ammoniumgruppe" oder "quaternäre Ammoniumgruppe" bezeichnet.

Ein Siloxan mit quartären Ammoniumgruppen wird im Rahmen der vorliegenden Offenbarung auch als "Silkonquat" oder als "quaternisiertes Siloxan" bezeichnet.

Es ist weiterhin bevorzugt, dass der Massenanteil von tertiären Aminen, ausgewählt aus der Gruppe bestehend aus Dialkanolaminen, in der Zusammensetzung bezogen auf die Gesamtmasse der Siloxane (A) und (B) und (C) weniger als 3%, bevorzugt weniger als 2%, weiter bevorzugt weniger als 1%, insbesondere von 0% bis 0,5% beträgt, oder die Zusammensetzung keine tertiären Aminen ausgewählt aus der Gruppe bestehend aus Dialkanolaminen enthält.

Unter einem "Dialkanolamin" wird im Rahmen der vorliegenden Offenbarung ein tertiäres Amin verstanden, mit einem Kohlenwasserstoffrest und zwei hydroxyfunktionellen Alkylresten die jeweils am tertiären Stickstoffatom gebunden sind. Vorzugsweise sind die Dialkanolamine ausgewählt aus Alkyldialkanolaminen (im Folgenden auch als N-Alkyldialkanolamine bezeichnet).

Es ist insbesondere bevorzugt, dass der Massenanteil von tertiären Aminen in der Zusammensetzung bezogen auf die Gesamtmasse der Siloxane (A) und (B) und (C) weniger als 3%, bevorzugt weniger als 2%, weiter bevorzugt weniger als 1%, insbesondere von 0% bis 0,5% beträgt, oder die Zusammensetzung keine tertiären Amine enthält.

Es ist weiterhin bevorzugt, dass der Massenanteil von tertiären Aminen mit einem Molekulargewicht von weniger als 500 g/mol in der Zusammensetzung bezogen auf die Gesamtmasse der Siloxane (A) und (B) und (C) weniger als 3%, bevorzugt weniger als 2%, weiter bevorzugt weniger als 1%, insbesondere von 0% bis 0,5% beträgt, oder die Zusammensetzung keine tertiären Amine mit einem Molekulargewicht von weniger als 500 g/mol enthält.

Es ist bevorzugt, dass der Massenanteil von tertiären Aminen ausgewählt aus der Gruppe der Amidamine in der Zusammensetzung bezogen auf die Gesamtmasse der Siloxane (A) und (B) und (C) in der Summe weniger als 1%, bevorzugt weniger als 0,8%, weiter bevorzugt weniger als 0,6%, insbesondere weniger als 0,4% beträgt, oder die Zusammensetzung keine tertiären Amine ausgewählt aus der Gruppe der Amidamine enthält.

Es ist bevorzugt, dass der Massenanteil von tertiären Aminen ausgewählt aus der Gruppe der Esteraminen in der Zusammensetzung bezogen auf die Gesamtmasse der Siloxane (A) und (B) und (C) in der Summe weniger als 1%, bevorzugt weniger als 0,8%, weiter bevorzugt weniger als 0,6%, insbesondere weniger als 0,4% beträgt, oder die Zusammensetzung keine tertiären Aminen ausgewählt aus der Gruppe der Esteramine enthält.

Im Sinne der Erfindung werden bevorzugt Amine eingesetzt, die keine Schädigung der Haut oder Giftwirkung über die Haut bewirken und die nicht umweltgefährlich sind, insbesondere nicht abwasserschädigend. Vorzugsweise sollten Amine vermieden werden, die gemäß Einstufung nach GHS die Kennzeichnungspflicht mit einem oder mehreren der folgenden H-Sätze aufweisen:

H310 Lebensgefahr bei Hautkontakt
H311 Giftig bei Hautkontakt
H312 Gesundheitsschädlich bei Hautkontakt
H314 Verursacht schwere Verätzungen der Haut und schwere Augenschäden
H315 Verursacht Hautreizungen
H317 Kann allergische Hautreizungen verursachen
H400 Sehr giftig für Wasserorganismen
H410 Sehr giftig für Wasserorganismen mit langfristiger Wirkung

H411 Giftig für Wasserorganismen mit langfristiger Wirkung
H412 Schädlich für Wasserorganismen, mit langfristiger Wirkung
H413 Kann für Wasserorganismen schädlich sein, mit langfristiger Wirkung

Die Herstellung von Silikonen/Siloxanen mit quartären/quaternären Ammoniumgruppen (Silikonquats) aus epoxyfunktionellen Siloxane und tertiären Aminen ist dem Fachmann bekannt. Silikonquats können nach den Verfahren des Standes der Technik hergestellt werden, wie beispielsweise in der DE 3719086 C1, DE 3802622 A1 und DE 102010000993 A1 beschrieben.

Bevorzugt werden die erfindungsgemäßen Siloxane nach dem erfindungsgemäßen Verfahren hergestellt, wobei epoxyfunktionelle Siloxane mit Mischungen aus tertiären Aminen, ausgewählt aus der Gruppe bestehend aus Amidaminen und Esteraminen, vorzugsweise Amidaminen, sowie tertiären Aminen, ausgewählt aus der Gruppe bestehend aus Dialkanolaminen, umgesetzt werden.

Ein weiterer Gegenstand der vorliegenden Erfindung ist daher ein Verfahren, das mindestens einen Verfahrensschritt umfasst, bei dem mindestens ein epoxyfunktionelles Siloxan, das mindestens zwei Epoxidgruppen aufweist, sowohl mit mindestens einem tertiären Amin, ausgewählt aus der Gruppe bestehend aus Amidaminen und Esteraminen, vorzugsweise Amidaminen, als auch mit mindestens einem tertiären Amin, ausgewählt aus der Gruppe bestehend aus Dialkanolaminen, unter Bildung von quartären Ammoniumgruppen umgesetzt wird.

Vorzugsweise dient das Verfahren zur Herstellung des erfindungsgemäßen Siloxans (A) und/oder der erfindungsgemäßen Zusammensetzung, die besagtes Siloxan (A) enthält. Vorzugsweise handelt es sich bei den tertiären Aminen, ausgewählt aus der Gruppe bestehend aus Amidaminen und Esteraminen, um tertiäre Amine, ausgewählt aus der Gruppe bestehend aus Amidaminen. Ein Verfahren, bei dem tertiäre Amine, ausgewählt aus der Gruppe bestehend aus Amidaminen, eingesetzt werden, ist also gegenüber einem Verfahren, bei dem tertiäre Amine, ausgewählt aus der Gruppe bestehend aus Esteraminen, eingesetzt werden, bevorzugt.

Vorzugsweise werden bei der Umsetzung des epoxyfunktionellen Siloxans solche quartären Ammoniumgruppen erhalten, die sowohl aus einer Reaktion des mindestens einen tertiären Amins, ausgewählt aus der Gruppe bestehend aus Amidaminen und Esteraminen, vorzugsweise Amidaminen, als auch aus einer Reaktion des mindestens einen tertiären Amins, ausgewählt aus der Gruppe bestehend aus Dialkanolaminen, mit jeweils mindestens einer der mindestens zwei Epoxidgruppen des epoxyfunktionellen Siloxan resultieren.

Vorzugsweise werden Epoxidgruppen im molaren Überschuss, weiter bevorzugt in equimolaren Mengen zu tertiären Aminogruppen eingesetzt, damit die tertiären Amine möglichst vollständig umgesetzt werden und ihr Restgehalt nach Umsetzung minimiert wird und weiter bevorzugt auch zusätzlich die Epoxysilane möglichst vollständig umgesetzt werden und ihr Restgehalt nach Umsetzung minimiert wird.

Es ist daher bevorzugt, dass das molare Verhältnis von tertiären Aminogruppen zu Epoxidgruppen von 0,8:1 bis 1:1, weiter bevorzugt von 0,9:1 bis 1, noch weiter bevorzugt von 0,95:1 bis 1:1, noch weiter bevorzugt von 0,99:1 bis 1:1, insbesondere 1:1 beträgt.

Es ist weiterhin bevorzugt, dass das molare Verhältnis ($mV_1$) von tertiären Aminogruppen, die Teil eines tertiären Amins, ausgewählt aus der Gruppe bestehend aus Amidaminen und Esteraminen, vorzugsweise Amidaminen, sind, zu Epoxidgruppen von 0,6:1 bis 0,8:1, weiter bevorzugt von 0,65:1 bis 0,75:1, insbesondere 0,7:1 beträgt;

Es ist außerdem bevorzugt, dass das molare Verhältnis ($mV_2$) von tertiären Aminogruppen, die Teil eines tertiären Amins, ausgewählt aus der Gruppe bestehend aus Dialkanolaminen, sind, zu Epoxidgruppen von 0,4:1 bis 0,2:1, weiter bevorzugt von 0,35:1 bis 0,25:1, insbesondere 0,3:1 beträgt.

Dabei gilt vorzugsweise die Maßgabe: ($mV_1$) + ($mV_2$) = 1:1.

Es ist weiterhin bevorzugt, dass das molare Verhältnis des mindestens einen tertiären Amin, ausgewählt aus der Gruppe bestehend aus Amidaminen und Esteraminen, vorzugsweise Amidaminen, zum mindestens ein tertiären Amin, ausgewählt aus der Gruppe bestehend aus Dialkanolaminen, von 90:10 bis 60:40, bevorzugt von 80:20 bis 65:35, insbesondere 70:30 beträgt.

Insbesondere bevorzugt beträgt das molare Verhältnis von tertiären Aminen, ausgewählt aus der Gruppe bestehend aus Amidaminen, zu tertiären Aminen, ausgewählt aus der Gruppe bestehend aus Alkyldialkanolaminen, von 90:10 bis 60:40, bevorzugt von 80:20 bis 65:35, insbesondere 70:30.

Dadurch kann der Anteil der quartären Ammoniumgruppen, die sich von einem tertiären Amin, ausgewählt aus der Gruppe bestehend aus Dialkanolaminen, ableiten, und der Anteil der quartären Ammoniumgruppen, die sich von tertiären Aminen, ausgewählt aus der Gruppe bestehend aus Amidaminen und Esteraminen, vorzugsweise Amidaminen, ableiten, vorteilhaft eingestellt werden.

In einer ersten Ausführungsform des Verfahrens wird das mindestens eine epoxyfunktionelle Siloxan in einem Verfahrensschritt mit mindestens einem tertiären Amin, ausgewählt aus der Gruppe bestehend aus Dialkanolaminen, umgesetzt und das erhaltene Umsetzungsprodukt in einem darauf mittelbar oder unmittelbar folgenden Verfahrensschritt mit mindestens einem tertiären Amin, ausgewählt aus der Gruppe bestehend aus Amidaminen und Esteraminen, vorzugsweise Amidaminen, weiter umgesetzt.

In einer zweiten Ausführungsform des Verfahrens, die gegenüber der ersten Ausführungsform des Verfahrens bevorzugt ist, wird das mindestens eine epoxyfunktionelle Siloxan in einem Verfahrensschritt mit mindestens einem tertiären Amin, ausgewählt aus der Gruppe bestehend aus Amidaminen und Esteraminen, vorzugsweise Amidaminen, umgesetzt und das erhaltene Umsetzungsprodukt in einem darauf mittelbar oder unmittelbar folgenden Verfahrensschritt mit mindestens einem tertiären Amin, ausgewählt aus der Gruppe bestehend aus Dialkanolaminen, weiter umgesetzt.

In einer dritten Ausführungsform des Verfahrens, die gegenüber der ersten und der zweiten Ausführungsform des Verfahrens bevorzugt ist, wird das mindestens eine epoxyfunktionelle Siloxan mit einer Mischung aus mindestens einem tertiären Amin, ausgewählt aus der Gruppe bestehend aus Amidaminen und Esteraminen, vorzugsweise Amidaminen, und mindestens einem tertiären Amin, ausgewählt aus der Gruppe bestehend aus Dialkanolaminen, umgesetzt.

Es ist bevorzugt, wenn bei der Umsetzung des mindestens einen epoxyfunktionellen Siloxans 90% bis 100% der Epoxidgruppen umgesetzt werden, besonders bevorzugt mehr als 92%. Die %-Angaben geben hierbei die Anzahl der umgesetzten Epoxid-Gruppen dividiert durch die Anzahl der eingesetzten der Epoxidgruppen an. Der Umsatz der Epoxidgruppen, auch als Epoxy-Umsatz bezeichnet, kann mit Hilfe der $^{1}$H-NMR-Spektroskopie, wie in den Beispielen beschrieben, bestimmt werden.

Bevorzugt werden die Verfahrensprodukte nach der Umsetzung der tertiären Amine und des mindestens einen epoxyfunktionellen Siloxans auf die Abwesenheit von restlichen Epoxidgruppen untersucht, wie in den Beispielen beschrieben. Sind weniger als 90% der Epoxidgruppen umgesetzt, wird die Reaktion so lange weiter durchgeführt, bis ein Umsatz von 90% oder mehr erreicht ist. Vorzugsweise wird der Ansatz verworfen, falls ein Umsatz von 90% oder mehr nicht erhalten wird.

Zur Umsetzung der tertiären Amine ist es bevorzugt, die Reaktion durch Katalyse zu beschleunigen. Als Katalysatoren werden bevorzugt Carbonsäuren eingesetzt, vorzugsweise Essigsäure, Isononansäure, Milchsäure, insbesondere Essigsäure.

Der Katalysator wird bevorzugt in einem Massenanteil von 0,5% bis 8%, bevorzugt von 1% bis 5% bezogen auf Gesamtmasse der Reaktanden, also unter Vernachlässigung weiterer nicht reaktiver Inhaltsstoffe, wie beispielsweise Lösemittel, eingesetzt.

Die Umsetzung des epoxyfunktionellen Siloxans mit den tertiären Aminen kann in Gegenwart oder in Abwesenheit, vorzugsweise aber in Gegenwart eines Lösemittels erfolgen. Als geeignete organische Lösemittel werden bevorzugt wasserfreie aliphatische Alkohole, Glykole oder Glykolether eingesetzt, wie beispielsweise Methanol, Ethanol, Propanol, Butanol, 2-Propanol, tert-Butanol, Propylenglykol, Dipropylenglykol, Tripropylenglykol, Hexylenglykol, Pentylenglykol, Butyldiglykol, Dipropylenglykoldimethylether, Dipropylenglykolmonomethylether, Propylenglykolmonomethylether, Tripropylenglykolmonomethylether, insbesondere 2-Propanol, Dipropylenglykol, Hexylenglykol.

Vorzugsweise wird das erhaltene Umsetzungsprodukt gereinigt, indem es einem geeigneten thermischen Trennverfahren unterzogen wird.

Thermische Trennverfahren sind unter diesem Begriff dem Fachmann bekannt und umfassen alle Verfahren, die auf der Einstellung eines thermodynamischen Phasengleichgewichtes beruhen. Bevorzugte thermische Trennverfahren sind ausgewählt aus der Liste enthaltend Destillation, Rektifikation, Adsorption, Kristallisation, Extraktion, Absorption, Trocknung und Ausfrieren, besonders bevorzugt sind Methoden der Destillation und Rektifikation.

Eine bevorzugte Ausführungsform des Verfahrens umfasst daher als weiteren Verfahrensschritt die Destillation und/oder Aufreinigung der Umsetzungsprodukte. Die Destillation und/oder Aufreinigung kann beispielsweise mittels eines Rotationsverdampfers erfolgen, vorzugsweise bei einer Temperatur von 20 bis 250 °C, weiter bevorzugt 40 bis 180 °C und besonders bevorzugt 50 bis 150 °C erfolgen, wobei der Druck dabei vorzugsweise von 0,0001 bis 0,75 bar, noch weiter bevorzugt von 0,001 bis 0,2 bar und besonders bevorzugt von 0,01 bis 0,1 bar beträgt. Die Destillation und/oder Aufreinigung ist insbesondere zur Abtrennung von flüchtigen Bestandteile, insbesondere von Lösemitteln vorteilhaft.

Bevorzugt eingesetzte Esteramine und/oder Amidamine sind solche gemäß Formel (VII)

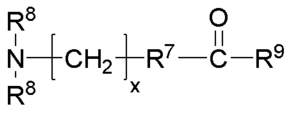
Formel (VII);

wobei $R^7$, $R^8$, $R^9$ und x wie in Formel (II) definiert sind.

Besonders bevorzugt werden Amidamine gemäß Formel (VII) eingesetzt.

Die im erfindungsgemäßen Verfahren eingesetzten Amidamine sind bevorzugt Reaktionsprodukte aus der Umsetzung von Dimethylaminoalkylaminen, insbesondere Dimethylaminopropylamin (DMAPA), mit Fettsäuren oder Fettsäureestern, wie beispielsweise die Triglyceride von Fettsäuren. Besonders bevorzugt sind Amidamine, welche sich von Fettsäuren mit 10 bis 30, weiter bevorzugt 12 bis 22, noch weiter bevorzugt 12 bis 18, insbesondere 16 bis 18 Kohlenstoffatomen ableiten.

Besonders bevorzugt werden daher Amidamine gemäß Formel (VII) mit $R^8$ = Methyl, x = 3, $R^7$= $-NR^{10}-$ mit $R^{10}$ = H eingesetzt.

Weiterhin bevorzugt ist $R^9$ ausgewählt aus der Gruppe bestehend aus Alkylresten mit 9 bis 29, weiter bevorzugt 11 bis 21, noch weiter bevorzugt 11 bis 17, insbesondere 15 bis 17 Kohlenstoffatomen, wobei die Alkyreste unsubstituiert oder mit Hydroxylgruppen substituiert, linear oder verzweigt, gesättigt oder ungesättigt sind, vorzugsweise unsubstituiert, linear und gesättigt sind.

Insbesondere bevorzugt sind Amidamine die unter dem Handelsnamen Tegoamid® von der Firma Evonik kommerziell erhältlich sind, wie beispielsweise 3-N,N-Dimethyl-aminopropyl-cocoylsäureamid (Tegoamid® D 5040 und Tegoamid® CNF), 3-N,N-Dimethyl-aminopropyl-stearylsäureamid (Tegoamid® S 18) und 3-N,N-Dimethyl-aminopropyl-palmitylsäureamid (Tegoamid® PKFC).

Weitere geeignete Amidamine werden in der Veröffentlichung *Safety Assessment of Fatty Acid Amidopropyl Dimethylamines as Used in Cosmetics, Final Report, Release Date: June 24, 2014, Panel meeting Date: June 9-10, 2014, Cosmetic Ingredient Review* (https://www.cir-safety.org/sites/default/files/amidoa062014final.pdf) offenbart, deren diesbezüglicher expliziter Offenbarungsgehalt durch diese Bezugnahme Teil dieser Offenbarung wird. Zu diesen Amidaminen zählen beispielsweise (engliche Bezeichnungen):

*almondamidopropyl dimethylamine*
*avocadamidopropyl dimethylamine*
*babassuamidopropyl dimethylamine*
*behenamidopropyl dimethylamine*
*brassicamidopropyl dimethylamine*
*cocamidopropyl dimethylamine*
*dilinoleamidopropyl dimethylamine*
*isostearamidopropyl dimethylamine*
*lauramidopropyl dimethylamine*
*linoleamidopropyl dimethylamine*
*minkamidopropyl dimethylamine*
*myristamidopropyl dimethylamine*
*oatamidopropyl dimethylamine*
*oleamidopropyl dimethylamine*
*olivamidopropyl dimethylamine*
*palmitamidopropyl dimethylamine*
*ricinoleamidopropyl dimethylamine*
*sesamidopropyl dimethylamine*
*soyamidopropyl dimethylamine*
*stearamidopropyl dimethylamine*
*sunflowerseedamidopropyl dimethylamine*
*tallamidopropyl dimethylamine*
*tallowamidopropyl dimethylamine*
*wheat germamidopropyl dimethylamine*

Bevorzugt eingesetzte Dialkanolamine sind solche gemäß Formel (VIII),

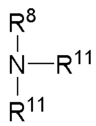

Formel (VIII)

wobei $R^8$ und $R^{11}$ wie in Formel (III) definiert sind.

Mehr bevorzugt wird das Dialkanolamin ausgewählt aus der Gruppe bestehend aus N-Methyldiethanolamin ($R^8$ = $-CH_3$ und $R^{11}$ = $-CH_2CH_2OH$), N-Ethyldiethanolamin ($R^8$ = $-CH_2CH_3$ und $R^{11}$ = $-CH_2CH_2OH$), N-Methyldiisopropanolamin ($R^8$ = $-CH_3$ und $R^{11}$ = $-CH_2CH(CH_3)OH$), N-Ethyldiisopropanolamin ($R^8$ = $-CH_2CH_3$ und $R^{11}$ = $-CH_2CH(CH_3)OH$), N-Isopropyldiaminoethanol ($R^8$ = $-CH(CH_3)_2$ und $R^{11}$ = $-CH_2CH_2OH$), N-Butyldiethanolamin ($R^8$ = $-CH_2CH_2CH_2CH_3$ und $R^{11}$ = $-CH_2CH_2OH$), sowie ihre Alkoxylierungsprodukten, wobei die Alkoxylierungsprodukte vorzugsweise durch Reaktion mit Ethylenoxid, Propylenoxid, Butylenoxid oder Mischungen von zwei oder drei der genannten Alkylenoxide auf die dem Fachmann bekannte Weise erhältlich sind.

Insbesondere bevorzugt eingesetzte Dialkanolamine sind N-Methyldiethanolamin ($R^8$ = $-CH_3$ und $R^{11}$ =

$-CH_2CH_2OH$) und N-Methyldiisopropanolamin ($R^8$ = $-CH_3$ und $R^{11}$ = $-CH_2CH(CH_3)OH$),

Es hat sich überraschenderweise gezeigt, dass die Reaktion von epoxyfunktionellen Siloxanen mit Alkyldialkanolaminen oder den entsprechenden Dialkylalkanolaminen zu einem hohen Umsatz von Epoxidgruppen führt, wohingegen der Umsatz von Epoxidgruppen bei einer Reaktion mit den entsprechenden Trialkanolaminen nur gering ist oder gar nicht erfolgt. Weiterhin wurde überraschenderweise festgestellt, dass die Verwendung von Alkyldialkanolaminen in Mischung mit Amidaminen zu einem geringeren Restgehalt an Amidaminen führt, als die Verwendung der entsprechenden Dialkylalkanolamine in Mischung mit Amidaminen. Zudem ist auch überraschend, dass Alkyldialkanolamine häufig weniger hautreizend und/oder sensibilisierend und/oder aquatoxisch sind als die entsprechenden Dialkylalkanolamine.

In einer bevorzugten Ausführungsform des Verfahrens beträgt der Restgehalt von tertiären Aminen, ausgewählt aus der Gruppe bestehend aus Amidaminen und Esteraminen, vorzugsweise Amidaminen, nach der Umsetzung als Massenanteil bezogen auf die Gesamtmasse der Zusammensetzung weniger als 1%, bevorzugt weniger als 0,8%, weiter bevorzugt weniger als 0,6%, insbesondere weniger als 0,4%.

In einer bevorzugten Ausführungsform des Verfahrens ist das epoxyfunktionelle Siloxan ein Siloxan der Formel (VI),

$$M^1_{a1}M^6_{a5}D^1_{b1}D^6_{b5}\,T^1_{c1}T^4_{c4}Q_d \qquad \text{(VI)},$$

mit

$M^6$ = $[R^{13}R^1{}_2SiO_{1/2}]$,
$D^6$ = $[R^{13}R^1SiO_{2/2}]$,
$R^{13}$ = jeweils unabhängig voneinander gleiche oder verschiedene organische Epoxy-Reste, vorzugsweise ausgewählt aus der Gruppe bestehend aus

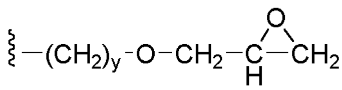

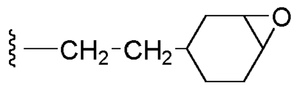

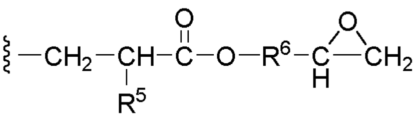

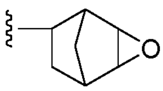

,

insbesondere

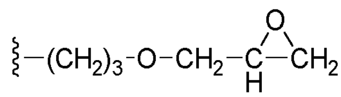

,

wobei

$M^1$, $D^1$, $T^1$, $T^4$, Q, a1, a5, b1, b5, c1, c4, d, $R^1$, $R^5$, $R^6$ und y wie in Formel (I) definiert sind.

Vorzugsweise wird das mindestens eine epoxyfunktionelle Siloxan durch Hydrosilylierung mindestens eines olefinisch ungesättigten Epoxids hergestellt.

Optional wird das mindestens eine epoxyfunktionelle Siloxan, vorzugsweise das epoxyfunktionelle Siloxan gemäß der Formel (VI), vor seiner Umsetzung gereinigt, indem es einem geeigneten thermischen Trennverfahren unterzogen wird.

Besonders bevorzugt wird das epoxyfunktionelle Siloxan hergestellt durch Hydrosilylierung mindestens eines olefinisch ungesättigten Epoxids, vorzugsweise ausgewählt aus der Gruppe bestehend aus Allylglycidylether, Vinylcyclohexenmonoxid und Norbornadienmonoepoxid, insbesondere Allylglycidylether, mit mindestens einem SiH-funktionellen Siloxan der Formel (V),

$$M^1_{a1}M^5_{a5}D^1_{b1}D^5_{b5}T^1_{c1}T^4_{c4}Q_d \qquad \text{(V)},$$

mit

$M^5$ = $[R^1{}_2SiHO_{1/2}]$,

$D^5$ = $[R^1SiHO_{2/2}]$,
a5 = 0 bis 32, bevorzugt 1 bis 10, besonders bevorzugt 2 bis 3, insbesondere 2;
b5 = 0 bis 10, bevorzugt 0 bis 5, insbesondere 0;

wobei
$M^1$, $D^1$, $T^1$, $T^4$, Q, a1, b1, c1, c4, d und $R^1$ wie in Formel (I) definiert sind.

Die Hydrosilylierung erfolgt auf die dem Fachmann bekannte Weise.

Die Hydrosilylierung des erfindungsgemäßen Verfahrens wird bevorzugt mit Hilfe der dem Fachmann geläufigen Katalysatoren der Platingruppe katalysiert, mehr bevorzugt mit Hilfe von Karstedt-Katalysatoren.

Die Hydrosilylierung kann in Gegenwart oder in Abwesenheit, vorzugsweise aber in Gegenwart eines Lösemittels erfolgen. Als geeignete organische Lösemittel werden bevorzugt Toluol, Xylol oder Isopropanol eingesetzt. Die eingesetzten Lösemittel sind vorzugsweise wasserfrei. Sofern das Lösungsmittel eine reaktive Gruppe aufweist, insbesondere eine Hydroxygruppe, kann dies in geringem Umfang zu SiOC-Nebenprodukten führen.

Es ist bevorzugt, wenn bei der Hydrosilylierung mehr als 95%, weiter bevorzugt mehr als 97%, insbesondere 99% bis 100% der SiH-Gruppen umgesetzt werden. Die %-Angaben geben die Anzahl der umgesetzten SiH-Gruppen dividiert durch die Anzahl der eingesetzten der SiH-Gruppen an. Der Nachweis der SiH-Gruppen erfolgt auf die dem Fachmann geläufige Art, bevorzugt gasvolumetrisch nach alkalischer Zersetzung. Dabei kann zum Beispiel eine Probe des Reaktionsgemisches mit einer butanolischen Natriumbutanolat-Lösung (Natriumbutanolat-Gehalt: 5 Gew.-%) umgesetzt und anhand der Menge des entstehenden Wasserstoffes auf die noch vorliegende Menge an SiH-Funktionen geschlossen werden.

Optional wird das mindestens eine SiH-funktionelle Siloxan gemäß Formel (V) vor der Hydrosilylierung gereinigt, indem es einem geeigneten thermischen Trennverfahren unterzogen wird.

Ebenfalls optional wird das erhaltene epoxyfunktionelle Siloxan gereinigt, vorzugsweise mittels eines thermischen Trennverfahrens, wie oben beschrieben.

Die SiH-funktionellen Siloxane können ebenfalls nach bekannten Verfahren durch Equilibrierung erhalten werden. Die Herstellung von linearen SiH-funktionellen Siloxanen mittels Equilibrierung mit Trifluormethansulfonsäure ist beispielsweise in US 5578692 oder EP 2176319 B1 beschrieben.

Herstellungsbedingt ist es möglich, dass die Verfahrensprodukte die zyklischen Siloxane Octamethylcyclotetrasiloxan und Decamethylcyclopentasiloxan enthalten. Octamethylcyclotetrasiloxan und Decamethylcyclopentasiloxan sind nicht biologisch abbaubar. Octamethylcyclotetrasiloxan ist zudem toxikologisch bedenklich. Aus diesen Gründen ist es vorteilhaft, dass der Stoffmengenanteil von Decamethylcyclopentasiloxan und/oder Octamethylcyclotetrasiloxan, möglichst gering ist.

In einer bevorzugten Ausführungsform der erfindungsgemäßen Zusammensetzung und des erfindungsgemäßen Verfahrensprodukts beträgt der Massenanteil von Decamethylcyclopentasiloxan bezogen auf die gesamte erfindungsgemäße Zusammensetzung beziehungsweise das gesamte erfindungsgemäße Verfahrensprodukt, weniger als 1% und beträgt insbesondere bevorzugt von 0 bis 0,1%.

In einer bevorzugten Ausführungsform der erfindungsgemäßen Zusammensetzung und des erfindungsgemäßen Verfahrensprodukts beträgt der Massenanteil von Octamethylcyclotetrasiloxan bezogen auf die gesamte erfindungsgemäße Zusammensetzung beziehungsweise das gesamte erfindungsgemäße Verfahrensprodukt weniger als 1% und beträgt insbesondere bevorzugt von 0 bis 0,1%.

Das erfindungsgemäße Verfahren kann vorzugsweise so ausgeführt werden, dass es zwei Verfahrensschritte umfasst, nämlich 1. Herstellung eines epoxyfunktionellen Siloxans, und 2. Umsetzung des epoxyfunktionellen Siloxans mit einem tertiären Amin, ausgewählt aus der Gruppe bestehend aus Amidaminen und Esteraminen, vorzugsweise Amidaminen, und einem tertiären Amin, ausgewählt aus der Gruppe bestehend aus Dialkanolaminen, zu den erfindungsgemäßen quaternisierten Silikonen. Die Verfahrensschritte der vorgenannten bevorzugten Ausführungsform der Erfindung können in dem erfindungsgemäßen Verfahren als aufeinander folgende getrennt durchgeführte Schritte jeweils als Eintopfreaktion oder auch dosierkontrolliert, vorzugsweise jedoch dosierkontrolliert durchgeführt werden. Die Reaktion kann in einem Batch, Semi-batch oder kontinuierlichen Verfahren durchgeführt werden. Insbesondere bevorzugt ist die dosagekontrollierte Reaktion des Verfahrensschrittes 1 und 2.

Das erfindungsgemäße Verfahren kann in Gegenwart oder in Abwesenheit eines Lösemittels erfolgen. Als geeignete organische Lösemittel für den 1. Verfahrensschritt werden bevorzugt Toluol, Xylol oder 2-Propanol eingesetzt. Als geeignete organische Lösemittel für den 2. Verfahrensschritt werden bevorzugt wasserfreie aliphatische Alkohole, Glykole oder Glykolether eingesetzt, wie beispielsweise Methanol, Ethanol, Propanol, Butanol, 2-Propanol, tert-Butanol, Propylenglykol, Dipropylenglykol, Tripropylenglykol, Hexylenglykol, Pentylenglykol, Butyldiglykol, Dipropylenglykoldimethylether, Dipropylenglykolmonomethylether, Propylenglykolmonomethylether, Tripropylenglykolmonomethylether, insbesondere 2-Propanol, Dipropylenglykol, Hexylenglykol.

Sofern das Lösungsmittel eine reaktive Gruppe aufweist, insbesondere eine Hydroxygruppe, kann es in geringem Umfang zu SiOC-Nebenprodukten führen. Ebenso können die Hydroxygruppen der verwendeten Dialkanolamine

in Spuren SiOC-Nebenprodukte bilden.

Die Reaktanden können dabei in jeder beliebigen Konzentration in einem Lösemittel vorliegen, zum Beispiel 5 bis 99 Gew.-%, bevorzugt 80 bis 95 Gew.-%, insbesondere bevorzugt 85 bis 95 Gew.-% bezogen auf die Gesamtzusammensetzung.

Das erfindungsgemäße Verfahren kann in einer bevorzugten Ausführungsform bei einer Temperatur von 10 bis 150 °C, vorzugsweise von 25 bis 100 °C, bevorzugt von 40 bis 90 °C durchgeführt werden.

Das erfindungsgemäße Verfahren kann im Sinne einer bevorzugten Ausführungsform vorzugsweise bei einem Druck von 0,5 bis 20 bar, bevorzugt 1 bis 5 bar, insbesondere bevorzugt bei Normaldruck durchgeführt werden.

Die erfindungsgemäße Umsetzung kann sowohl bei Tageslicht als auch unter Lichtausschluss, vorzugsweise bei Tageslicht durchgeführt werden.

Die erfindungsgemäße Umsetzung kann sowohl unter inerten Bedingungen (Stickstoff, Argon) als auch unter Sauerstoff- und/oder Luftatmosphäre, vorzugsweise unter Stickstoffatmosphäre durchgeführt werden.

Ein weiterer Gegenstand der Erfindung ist eine Zusammensetzung, die durch das erfindungsgemäße Verfahren erhalten werden kann.

In einer bevorzugten Ausführungsform enthält die erfindungsgemäße Zusammensetzung als weiteren Bestandteil Wasser.

Vorzugsweise ist die Zusammensetzung eine wässrige Emulsion.

Es ist weiter bevorzugt, dass die Zusammensetzung, vorzugsweise die wässrige Emulsion, in Massenanteilen bezogen auf die Gesamtmasse der Zusammensetzung folgende Komponenten enthält:

a) 20% bis 99,5%, bevorzugt 40 bis 97%, insbesondere 60 bis 95% Wasser;
b) 0,5% bis 80%, bevorzugt 3% bis 60%, insbesondere 5% bis 40% mindestens eines Siloxans, umfassend mindestens ein Siloxan (A) und vorzugsweise mindestens ein Siloxan (B) und/oder mindestens ein Siloxan (C);
c) vorzugsweise 1% bis 10% mindestens eines Emulgators;
d) vorzugsweise 5% bis 20% mindestens eines Glykols; und
e) vorzugsweise 0% bis 1% Essigsäure.

Die erfindungsgemäßen Zusammensetzungen, insbesondere die wässrigen Emulsionen, enthalten vorzugsweise weiterhin Additive, die ausgewählt sein können aus der Gruppe bestehend aus Boostern, Emulgatoren, Lösemitteln, Parfüm, Parfümträgern, Farbstoffen, Viskositätsregulatoren, Entschäumern, Konservierungsmitteln, antimikrobiellen Wirkstoffen, Germiziden, Fungiziden, Antioxidantien, organischen Lösemitteln, nicht-siloxanhaltigen Polymeren und anderen nichterfindungsgemäße siloxanhaltige Polymeren, wie beispielsweise nichterfindungsgemäße siloxanhaltige Silikonölen, Tensiden, Gerüststoffen, Bleichmitteln, Bleichaktivatoren, Enzymen, Fluoreszenzmitteln, Schauminhibitoren, Antiredepositionsmitteln, optischen Aufhellern, Vergrauungsinhibitoren, Einlaufverhinderern, Knitterschutzmitteln, Farbübertragungsinhibitoren, Korrosionsinhibitoren, nichterfindungsgemäße Antistatika, Bittermitteln, Bügelhilfsmitteln, Phobier- und Imprägniermitteln, Quell- und Schiebefestmitteln, neutralen Füllsalzen und UV-Absorber. Hierbei können Substanzen einer Klasse auch in einer anderen Klasse Wirksamkeit entfalten.

Insbesondere kann die erfindungsgemäße Zusammensetzungen zwischen 0,001 und 40 Gew.-%, besonders bevorzugt 0,01 bis 20 Gew.-% eines oder mehrerer unterschiedlicher Additive oder Hilfsstoffe, bezogen auf die Gesamtmasse der Siloxane (A) und (B) und (C) oder der enfindungsgmäßen Verfahrensprodukte, enthalten.

Bevorzugt liegen die erfindungsgemäßen Zusammensetzungen als Konzentrate, Compounds/Emulsionskonzentrate und/oder deren wässerige Formulierungen, als wässrige Emulsionen und/oder Lösungen, und/oder oder als eine Formulierung oder Emulsion in organischen Verbindungen wie Polyethern, Polyolen, Alkoholen vor.

Weiterhin besonders bevorzugte erfindungsgemäße Zusammensetzungen sind Konzentrate, welche die erfindungsgemäßen Siloxane oder die erfindungsgemäßen Verfahrensprodukte in einer Konzentrationen von 75 bis 99,99 Gew.-% bezogen auf die gesamte Zusammensetzung enthalten. Diese Konzentrate sind also nur mit geringen Anteilen an Lösemitteln versetzt sind. Bevorzugt sind die Konzentrate keine wässrigen Lösungen.

Weitere besonders bevorzugte erfindungsgemäße Zusammensetzungen sind Compound- oder Emulsionskonzentrate, die die erfindungsgemäßen Siloxane oder die erfindungsgemäßen Verfahrensprodukte in Konzentrationen von 40 bis 90 Gew.-%, bevorzugt 50 bis 80 Gew.-% bezogen auf die Gesamtmasse enthalten. Weitere Bestandteile dieser Compound- oder Emulsionskonzentrate sind Wasser und/oder Lösemittel, ausgewählt aus der Gruppe der Glykole, unverzweigten und/oder verzweigten Alkohole und/oder Alkylether mit 1 bis 6 Kohlenstoffatomen und gegebenenfalls einen oder mehreren nicht-ionischen Emulgatoren, beispielsweise ein Alkoholethoxylat mit 3 bis 25 Ethylenoxideinheiten. Compound- und Emulsionskonzentrate sind in der Regel in Wasser löslich oder selbstemulgierbar.

Besonders bevorzugte erfindungsgemäße wässrige Emulsionen, vorzugsweise Mikroemulsionen, sind griffgebende Mittel zur Behandlung von textilen Flächengebilden.

Flächengebilde im Umfang dieser Erfindung sind massiv oder aus Fasern, wie Holz, Baumwolle, Polyester, Polyamid, künstliche Synthesefasern, Papier und Pappe, Viskose, Cellulose und/oder Lignin-basierte Fasern. Flächengebilde im Umfang dieser Erfindung umfassen ebenfalls harte Oberflächen aus Metall, Keramik, Glas, Holz oder Kunststoff.

Bevorzugte Flächengebilde sind ausgewählt aus der Gruppe umfassend textile Flächengewebe, Haare und Fell, insbesondere bevorzugt sind textile Flächengewebe, Gewirke, Gestricke, Vliesstoffe (sogenannte *non-wovens), Tissue* (Papierfaser) und/oder andere Fasern aus natürlichen und/oder synthetischen Rohstoffen.

Insbesondere bevorzugte erfindungsgemäße Zusammensetzungen sind griffgebende Mittel zur temporären oder permanenten Ausrüstung von Textilien.

Die erfindungsgemäßen Zusammensetzungen können gegebenenfalls weitere nichterfindungsgemäße Textilweichmacher enthalten. Diese sind eine oder mehrere kationische Textil-weichmachende Verbindungen, die eine oder mehrere langkettige Alkylgruppen in einem Molekül aufweisen. Weit verbreitete kationische Textil-weichmachende Verbindungen umfassen beispielsweise Alkanolamin-Esterquat Verbindungen oder bekannte quaternäre Ammoniumverbindungen, mit zwei C18-Acyl Gruppen verestert. Weitere geeignete Ammoniumverbindungen werden in US 2010/0184634 in den Absätzen [0027] bis [0068] offenbart, deren diesbezüglicher expliziter Offenbarungsgehalt durch diese Bezugnahme Teil dieser Offenbarung wird.

Durch Verdünnen mit Wasser können aus den erfindungsgemäßen Konzentraten, Emulsionskonzentraten und Formulierungen z.B. die erfindungsgemäßen Ausrüstmittel für Textilien hergestellt werden.

Die erfindungsgemäßen wässrigen Emulsionen als griffgebende Mittel für textile Flächengebilde enthalten die erfindungsgemäßen Siloxane oder die erfindungsgemäßen Verfahrensprodukte in einem Massenanteil von 3% bis 35%, bevorzugt von 5% bis 25%, insbesondere von 7% bis 20% bezogen auf die gesamte Zusammensetzung.

Als Emulgatoren werden typischerweise Fettalkoholethoxylate mit Ethoxylierungsgraden zwischen 3 und 12 eingesetzt, und zwar in einem Massenverhältnis der Siloxane (A), (B) und (C) zusammen zu den Fettalkoholethoxylaten von 20:1 bis 1:1. Ebenso finden üblicherweise hoch siedende Glykole wie Dipropylenglykol oder Butyldiglykol Anwendung. Diese Glykole können ganz oder teilweise die Fettalkoholethoxylate ersetzen.

Bevorzugt sind Emulgatoren in den erfindungsgemäßen Zusammensetzungen und den erfindungsgemäßen wässrigen Emulsionen in einem Massenanteil von 1% bis 10%, mehr bevorzugt von 1,5% bis 8% bezogen auf die gesamte Zusammensetzung enthalten.

Als Entschäumer können alle für wässrige Textilflotten aus dem Stand der Technik als geeignet bekannten Entschäumer eingesetzt werden. Beispiele für geeignete handelsübliche Entschäumer sind unter dem Namen Dow Corning® DB-110A und TEGO® Antifoam® MR 1015 erhältlich.

Bevorzugt enthält die erfindungsgemäßen Zusammensetzung mindestens einen Entschäumer in einen Massenanteil von 0,0001% bis 0,05%, besonders bevorzugt von 0,001% bis 0,01% bezogen auf die gesamte Zusammensetzung.

Als Konservierungsmittel kann die Zusammensetzung aus dem Stand der Technik als geeignet bekannte bakterizide und/oder fungizide Wirkstoffe enthalten, wobei wasserlösliche Wirkstoffe bevorzugt sind. Beispiele für geeignete handelsübliche Bakerizide sind Methylparaben, 2-Brom-2-nitro-1,3-propandiol, 2-Methyl-4-isothiazolin-3-on und 5-Chlor-2-methyl-4-isothiazolin-3-on.

Ebenso kann die erfindungsgemäße Zusammensetzung ein Konservierungsmittel, vorzugsweise einen Oxidationsinhibitor enthalten. Beispiele für geeignete handelsübliche Oxidationsinhibitoren sind Ascorbinsäure, 2,6-Di-tert-butyl-4-methlyphenol (BHT), Butylhydroxyanisol (BHA), Tocopherol und Propylgallat. Bevorzugt enthalten die erfindungsgemäßen Zusammensetzungen mindestens ein Konservierungsmittel in einem Massenanteil von 0,0001% bis 0,5%, besonders bevorzugt von 0,001% bis 0,2% bezogen auf die gesamte Zusammensetzung. Insbesondere können die Zusammensetzungen mindestens einen Oxidationsinhibitor in einem Massenanteil von 0,001% bis 0,1%, besonders bevorzugt von 0,001% bis 0,01-% bezogen auf die gesamte Zusammensetzung enthalten.

Als organische Lösemittel kann die Zusammensetzung kurzkettige Alkohole, Glykole und Glykolmonoether enthalten, wobei Ethanol, 2-Propanol, 1,2-Propandiol und Dipropylenglycol bevorzugt sind. Insbesondere können die erfindungsgemäßen Zusammensetzungen mindestens ein organisches Lösemittel in einem Massenanteil von 0,1% bis 10%, besonders bevorzugt von 0,2% bis 5% bezogen auf die gesamte Zusammensetzung enthalten.

Ein weiterer Gegenstand der Erfindung ist die Verwendung der erfindungsgemäßen Siloxane und/oder Zusammensetzungen und/oder Verfahrensprodukte

a) zur Behandlung, vorzugsweise Ausrüstung und/oder Imprägnierung, von Flächengebilden;
b) in Reinigungs- und Pflegeformulierungen für den Haushalt und für industrielle Anwendungen, insbesondere in Weichspülmitteln,
c) in kosmetischen, pharmazeutischen und dermatologischen Zusammensetzungen, insbesondere in kosmetischen Reinigungs- und Pflegeformulierungen, Haarbehandlungsmitteln und Haarnachbehandlungsmitteln; und/oder
d) zur Reinigung und Pflege von harten Oberflächen, vorzugsweise zur Reinigung und Pflege von Fahrzeugen, insbesondere als Additiv in Trocknungshilfen für Autowaschstraßen.

Bevorzugt ist die Verwendung der erfindungsgemäßen Siloxane und/oder Zusammensetzungen und/oder Verfahrensprodukte zur Ausrüstung von textilen Flächengebilden.

Mehr bevorzugt ist die Verwendung der erfindungsgemäßen Siloxane und/oder Zusammensetzungen und/oder Verfahrensprodukte in hydrophilen griffgebenden Zusammensetzungen, insbesondere in Textil-weichmachenden Zusammensetzungen (Weichspüler).

Weiterhin bevorzugt werden die erfindungsgemäßen Siloxane, Zusammensetzungen und/oder Verfahrensprodukte als Weichmacher für Flächengebilde verwendet.

Sie werden beispielsweise in Weichspülmitteln, insbesondere wässrigen Weichspülmitteln, eingesetzt. Wässrige Weichspülmittel werden üblicherweise beim Waschen von Wäsche in einer Waschmaschine dem letzten Spülgang zugesetzt, um der Wäsche einen weicheren Griff zu geben. Solche Weichspülmittel enthalten die erfindungsgemäßen Siloxane in einer Menge von 2 bis 20 Gew.-% bezogen auf die Weichspülmittelzusammensetzung, dispergiert in einer wässrigen Lösung.

Für die Verwendung als Weichmacher für Flächengebilde weisen die erfindungsgemäßen Siloxane ein molares Verhältnis von Siliziumatomen zu quartären Ammoniumgruppen von mehr als 25:1, bevorzugt von 50:1 bis 200:1 auf.

Silikonquats werden nicht nur zur Verbesserung des Weichgriffs/Warengriffs in textilen Prozessen eingesetzt, sondern auch als Antistatika mit reibungsreduzierender Wirkung.

Weiterhin bevorzugt werden die erfindungsgemäßen Siloxane, die erfindungsgemäßen Zusammensetzungen und die erfindungsgemäßen Verfahrensprodukte daher als Antistatika eingesetzt.

Weiterhin bevorzugt werden die erfindungsgemäßen Siloxane, die erfindungsgemäßen Zusammensetzungen und die erfindungsgemäßen Verfahrensprodukte als Gleitmittel eingesetzt. Vorzugsweise weisen sie also eine reibungsreduzierende Wirkung auf.

Zur Verwendung als Antistatika und/oder als Gleitmittel sind insbesondere solche Siloxane geeignet, die bezogen auf die Anzahl der quartären Ammoniumgruppen ein geringes Molekulargewicht aufweisen.

Vorzugsweise weisen die erfindungsgemäßen Siloxane zur Verwendung als Antistatika daher weniger als 50 Siliziumatome, insbesondere 15 bis 30 Siliziumatome auf.

Für die Verwendung als Antistatika oder für die Verwendung zur Behandlung harter Oberflächen, insbesondere im Automobilbereich, weisen die erfindungsgemäßen Siloxane ein molares Verhältnis von Siliziumatomen zu quartären Ammoniumgruppen von weniger als 25:1, bevorzugt von 5:1 bis 25:1, insbesondere 10:1 bis 15:1 auf.

Weiterhin bevorzugt werden die erfindungsgemäßen Siloxane, Zusammensetzungen und/oder Verfahrensprodukte als Reinigungs- und Pflegemittel für harte Oberflächen eingesetzt, vorzugsweise zur Reinigung und Pflege von Fahrzeugen, insbesondere als Additiv in Trocknungshilfen für Autowaschstraßen.

Die Reinigung und Pflege harter Oberflächen, insbesondere das Waschen von Fahrzeugen in Autowaschanlagen, kann in eine Vor- und Hauptwäsche unterteilt sein. Dabei können unterschiedliche Zusammensetzungen verwendet werden. Bei der Reinigung werden Schmutzpartikel auf der Fahrzeugoberfläche entfernt. An diese Reinigung schließt sich der Spülvorgang an, bei dem Reinigungsmittelreste entfernt werden. Dieser Schritt dient der Vorbereitung für die Anwendung eines Trocknungsmittels, das vor der abschließenden Gebläsetrocknung das Fahrzeug hydrophobiert und so der verbleibende Wasserfilm leichter entfernt werden kann. Der Spülvorgang ist deshalb vorteilhaft, da Trocknungsmittel einen kationischen Charakter haben und sich sonst nach der Anwendung anionischer Reinigungsformulierungen schwerlösliche Salze bilden könne, die auf dem Fahrzeug zu Flecken führen und so weder zum gewünschten Glanzeffekt noch zur Hydrophobierung führen. Die erfindungsgemäßen Silikonquats bilden bei Anwendungen, wo ein Verbleiben der oberflächenaktiven Verbindung auf dem behandelten Gut gefordert ist, die wesentlichen Inhaltsstoffe dieser Formulierungen. Wie bei Anwendungen im Bereich Weichspüler, Textilausrüstung oder Haarspülungen, so finden die erfindungsgemäßen Silikonquats auch bei Trockneranwendungen in Autowaschanlagen ihre Verbreitung. Da auch Fahrzeuglacke, wie die meisten Oberflächen, ein negatives elektrisches Oberflächenpotential aufweisen, breiten sich die Silikonquats nach dem Aufsprühen der Trocknungsmittelformulierung auf dem Fahrzeug aus und verdrängen den vorhandenen Wasserfilm. Die Silikonquats führen zu einer Verstärkung des Farb-und Glanzeindrucks des Lackes und schützen vor Witterungseinflüssen.

Die erfindungsgemäßen Siloxane und/oder die erfindungsgemäßen Zusammensetzung und/oder die erfindungsgemäßen Verfahrensprodukte weisen zahlreiche Vorteile gegenüber Silikonquats des Standes der Technik auf, insbesondere zeigen sie:

a) eine höhere Wirkung bei gleicher Einsatzkonzentration;

b) reduzierte Anteile unerwünschter organischer Verbindungen, insbesondere niedermolekularer organischer Verbindungen, und somit eine signifikante Reduktion des mit diesen Verbindungen assoziierten Gefahrenpotentials von hautsensibilisierender Wirkung oder Gewässerschädigung bei Eintrag ins Oberflächenwasser;

c) eine bessere Verarbeitbarkeit und eine niedrigere Viskosität bei gleicher Wirkstoffmenge und gleichzeitig einem geringeren Lösemittel- bzw. Emulgatorverbrauch;

d) eine längere Haltbarkeit;

e) eine geringere Penetrationsneigung des Ausrüstmittels;
f) eine unveränderte Atmungsaktivität der mit ihnen ausgerüsteten Textilien;
g) ein hohes Effektniveau der mit ihnen ausgerüsteten Textilien auch nach mehreren Wäschen;
h) eine Verbesserung der haptischen Eigenschaften und einen angenehmeren Tragekomfort der mit ihnen ausgerüsteten Textilien; und/oder
i) eine gute Lagerstabilität, d.h. die Viskosität bleibt bei Lagerung stabil und die Neubildung zyklischer Siloxane ist minimiert; und/oder
j) eine verbesserte pH-Stabilität bis zu einem pH-Wert von 11.

**Beispiele**

Allgemeine Methoden

Kenrspinresonanzspektroskopie (NMR-Spektroskopie)

Die Charakterisierung der Siloxane kann mit Hilfe der $^{1}$H-NMR- und $^{29}$Si-NMR-Spektroskopie erfolgen. Diese Methoden, insbesondere unter Berücksichtigung der Multiplizität der Kopplungen, sind dem Fachmann geläufig.
Der Umsatz der Epoxidgruppen (Epoxy-Umsatz) kann mit Hilfe der $^{1}$H-NMR-Spektroskopie bestimmt werden.

Gel-Permeations-Chromatographie (GPC):

GPC-Messungen zur Bestimmung der Polydispersität und gewichtsmittleren Molmassen Mw werden unter den folgenden Messbedingungen durchgeführt: Säulenkombination SDV 1000/10000 Å (Länge 55 cm), Temperatur 35 °C, THF als mobile Phase, Fließrate 0,35 ml/min, Probenkonzentration 10 g/l, RI-Detektor, Auswertung der Polymere gegen Polystyrol-Standard (162-2520000 g/mol).

Hochleistungsflüssigkeitschromatographie (*high performance liquid chromatography,* HPLC):

Zur Bestimmung der Konzentration von Amidaminen (Tegoamid® S18, Tegoamid® D5040, Tegoamid® PKFC) wird eine Umkehrphasen-HPLC mit Gradient-Bedingungen durchgeführt. Eine RP-C18 Säule (Inertsil ODS-3, GL Science) wird als stationäre Phase eingesetzt. Acetonitril und verdünnte Schwefelsäure werden als binäres Eluenten-System angewendet. Die Detektion erfolgt per UV-Detektor bei einer Wellenlänge von 210 nm. Als externer Standard für die Kalibrierung werden spezifische Amidamine verwendet, welche in der jeweiligen Synthese des Siloxans/Silikonquats zum Einsatz kommen. Der Restgehalt ist in Gewichtsprozent bezogen auf die entsprechende Zusammensetzung angegeben.

Gaschromatographie:

Der Massenanteil an zyklischen Siloxanen, insbesondere Octamethylcyclotetrasiloxan (D4) und Decamethylcyclopentasiloxan (D5), kann mit Hilfe einer gaschromatographischen Methode (GC-Methode) bestimmet werden, in der die Substanzen dem Siedepunkt nach aufgetrennt und mittels Wärmeleitfähigkeitsdetektor detektiert werden. Dabei wird ein Aliquot der zu untersuchenden Probe ohne weitere Verdünnung mittels GC analysiert. Dieses wird in einem Gaschromatograph, welcher mit einem split/splitless Injektor, einer Kapillarsäule und einem Wärmeleitfähigkeitsdetektor ausgestattet ist, unter folgenden Bedingungen durchgeführt:

| | |
|---|---|
| Injektor: | 290 °C, Split 40 ml |
| Injektionsvolumen: | 1 µl |
| Säule: | 5 m *0,32 mm HP5 1µm |
| Trägergas: | Helium, const. flow 2 mL/min |
| Temperaturprogramm: | 1 Minute bei 80 °C, dann 80 °C - 300 °C mit 30 °C/min, dann 10 Minuten bei 300 °C konditionieren. |
| Detektor: | WLD bei 320 °C<br>Make Up Gas 6 mL/min<br>Referenzgas 18 mL/min |

Die zyklischen Siloxane werden ihrem Siedepunkt nach aufgetrennt. Der Massenanteil der einzelnen Substanzen wird bestimmt als prozentualer Anteil der für die jeweilige Substanz bestimmten Peakflächen im Vergleich zur Gesamtfläche aller detektierten Substanzen (Area-% Methode).

Viskosität:

Die Viskosität wird mit einem R/S-CPS Plus Rheometer der Firma Brookfield unter Verwendung der Messplatte RP75 bei 25°C gemessen. Die Messmethode ist in der DIN 53019 (DIN 53019-1:2008-09, DIN 53019-2:2001-02 und DIN 53019-3:2008-09) beschrieben.

Allgemeine Synthesevorschrift:

Die quaternisierten Siloxane (hier auch als Wirkstoffe oder Silikonquats bezeichnet) werden auf die dem Fachmann bekannte Weise, wie im Stand der Technik, beispielsweise in den Druckschriften DE 102010000993 A1 und DE 3802622 A1 beschrieben, hergestellt. Die Herstellung erfolgt in drei Stufen. In der ersten Stufe erfolgt die Herstellung der SiH-funktionellen Siloxane. In der zweiten Stufe werden aus den hergestellten SiH-funktionellen Siloxanen mittels Hydrosilylierung epoxyfunktionelle Siloxane hergestellt. In der dritten Stufe werden die erhaltenen epoxyfunktionellen Siloxane mit tertiären Aminen unter Säurekatalyse zu wie folgt umgesetzt:

1. Stufe - Herstellung von SiH-funktionellen Siloxanen:

Lineare endständige SiH-Siloxane:

In einem inertisierten 500 ml Dreihalskolben mit KPG-Rührer, Rückflusskühler und Innenthermometer wurden die jeweiligen Mengen (vgl. Tabelle 1) Decamethylcyclopentasiloxan (D5) und $\alpha,\omega$-Dihydrogenpolydimethylsiloxan ($\alpha,\omega$-Dihydrogen-PDMS) mit einem SiH-Wert von 2,97 mmol/g vorgelegt und unter Rühren mit 0,25 g Trifluormethansulfonsäure versetzt. Nach 6h Rühren bei 40°C wurden 5g Natriumhydrogencarbonat zugegeben und 2h gerührt. Nach Filtration erhielt man transparente, flüssige, farblose Produkte.

Einwaagen und weitere Angaben zur Herstellung der SiH-funktionellen Siloxane sind der Tabelle 1 zu entnehmen.

Tabelle 1: Einwaagen und weitere Angaben zur Herstellung der SiH-funktionellen Siloxane gemäß Formel (V)

| SiH-Siloxan | a5 | b1 | $R^1$ | $\alpha,\omega$-Dihydrogen-PDMS | D5 |
|---|---|---|---|---|---|
| SH1 | 2 | 48 | Methyl | 45,5 g | 204,5 g |
| SH2 | 2 | 78 | Methyl | 28,5 g | 221,5 g |
| SH4 | 2 | 18 | Methyl | 114,6 g | 135,4 g |
| SH5 | 2 | 28 | Methyl | 76,2 g | 173,8 g |

Verzweigtes SiH-Siloxan (SH3):

Die Herstellung erfolgte wie in der Druckschrift EP 2176319 B1 offenbart.

44,2 g (0,248 mol) Methyltriethoxysilan, 125,3 g eines $\alpha,\omega$-Dihydrogenpolydimethylsiloxans mit einem Wasserstoffgehalt von 2,97 mmol SiH/g und 1352,5 g Decamethylcyclopentasiloxan wurden in einem Vierhalskolben, ausgestattet mit einem KPG-Rührer, einem Innenthermometer, einem Tropftrichter und einer Destillationsbrücke, unter Rühren bei Raumtemperatur vorgelegt, 1,5 g Trifluormethansulfonsäure zugegeben und 30 Minuten gerührt. Innerhalb von weiteren 30 Minuten wurde unter Rühren eine Mischung aus 13,4 g deionisiertem Wasser und 20 ml Methanol zugetropft und weitere 30 Minuten gerührt. Die Reaktionsmischung wurde für 1 Stunde auf 40 °C erwärmt und anschließend im Wasserstrahlpumpenvakuum von ca. 50 mbar 1 Stunde bei 40 °C destilliert. Nach Neutralisation mit 30,4 g Natriumhydrogencarbonat und Filtration wurden 152 g des vorgetrockneten sulfonsauren Kationenaustauscherharzes Lewatit® K 2621 zugegeben, 4 Stunden bei 40 °C gerührt und abfiltriert. Man erhielt eine klare, farblose Flüssigkeit.

2. Stufe - Herstellung von epoxyfunktionellen Siloxanen:

In einem inertisierten 500 ml Dreihalskolben mit KPG-Rührer, Innenthermometer und Rückflusskühler wurden die jeweiligen Mengen SiH-Siloxan und Allylglycidylether (AGE) (vgl. Tabelle 2) vermischt und unter Rühren auf 70°C aufgeheizt. Es wurden 0,13 g Karstedt-Katalysator (0,1% Pt) mit einer Spritze hinzugegeben und weitere 2h bei 80°C

gerührt, bei Bedarf unter Gegenkühlung der anfänglichen Exothermie. Nach 3h Destillation bei 120°C und 1 mbar erhielt man ein transparentes leicht beigefarbenes flüssiges Produkt der Viskosität 135 mPa*s. Die Hydrosilylierungsreaktion wurde in Bezug auf den Wasserstoffgehalt der SiH-funktionellen Siloxane zu einem vollständigen Umsatz gebracht. Unter vollständigem Umsatz wird im Rahmen der vorliegenden Erfindung verstanden, dass mehr als 99% der SiH-Funktionen umgesetzt wurden. Der Nachweis erfolgt auf die dem Fachmann geläufige Art gasvolumetrisch nach alkalischer Zersetzung.

Einwaagen und weitere Angaben zur Herstellung der epoxyfunktionellen Siloxane sind der Tabelle 2 zu entnehmen.

Tabelle 2: Einwaagen und weitere Angaben zur Herstellung der epoxyfunktionellen Siloxane gemäß Formel (VI)

| Epoxysiloxan | a5 | b1 | c1 | $R^1$ | SiH-Siloxan | AGE |
|---|---|---|---|---|---|---|
| SE1 | 2 | 48 | 0 | Methyl | 231,4 g SH1 | 18,6 g |
| SE2 | 2 | 78 | 0 | Methyl | 238,1 g SH2 | 11,9 g |
| SE3 | 6 | 316 | 4 | Methyl | 241,1 g SH3 | 8,9 g |
| SE4 | 2 | 18 | 0 | Methyl | 208,0 g SH4 | 42,0 g |
| SE5 | 2 | 28 | 0 | Methyl | 220,4 g SH5 | 29,6 g |

3. Stufe - Herstellung der Silikonquats:

In einem inertisierten 500 ml Dreihalskolben mit KPG-Rührer, Tropftrichter, Innenthermometer und Rückflusskühler wurden die jeweiligen Mengen (vgl. Tabelle 3) Amidamin, Alkanolamin und Lösemittel vorgelegt, die jeweilige Menge Carbonsäure zudosiert und bei Raumtemperatur für 1 Stunde gerührt. Anschließend wurden das jeweilige epoxyfunktionelle Siloxan zugetropft, auf 80°C erhitzt und 12 bis 16 Stunden gerührt, bis ein Umsatz an Epoxidgruppen (auch als Epoxy-Umsatz bezeichnet) von mindestens 90% erreicht war. Der Umsatz der Epoxidgruppen wurde NMR-spektroskopisch bestimmt. Optional wurde das Lösemittel durch Destillation entfernt und durch anschließendes Abmischen mit einem anderen Lösemittel, also durch Verdünnung des erhaltenen Destillationsrückstands mit einem anderen Lösemittel, ausgetauscht.

Bei der Herstellung der Silikonquats wurden die folgenden Rohstoffe verwendet:

Amid1 = 3-N,N-Dimethyl-aminopropyl-cocoylsäureamid, Tegoamid® D 5040, Evonik

Amid2 = 3-N,N-Dimethyl-aminopropyl-stearylsäureamid, Tegoamid® S 18, Evonik

Amid3 = 3-N,N-Dimethyl-aminopropyl-palmitylsäureamid, Tegoamid® PKFC, Evonik

MDEA = N-Methyldiethanolamin, 99%ig, Sigma-Aldrich

MDIPA = N-Methyldiisopropanolamin, BASF

TEA = Triethanolamin, 99%ig, Sigma-Aldrich

DMAE = Dimethylglycin (Dimethylaminoessigsäure), >98%ig, Alfa-Aesar

HOAc = Essigsäure, p. A., Baker

INA = Isononansäure, 97%ig, Alfa-Aesar

IPA = Isopropanol, >99,9%ig, Sasol

tBuOH = tert.-Butanol, ACS, Reag. Ph Eur, Merck

DPG = Dipropylenglykol, >=99%ig, Lyondell

PG = 1,2-Propylenglykol, >=99%ig, Lyondell

DMM = Dipropylenglykoldimethylether, >94%ig, TCI Europe N.

Amid1 wird dabei hergestellt durch Umsetzung von gehärtetem Kokosfett mit 3-Aminopropyldimethylamin (DMAPA). Die Umsetzung führt zu einer Verteilung der Kettenlänge des Fettsäurerests des resultierenden Amidamins von C8 bis C18 mit einem Maximum bei C12.

Bei der Herstellung der Silikonquats wurden die folgenden epoxyfunktionellen Siloxane eingesetzt:

Tabelle 3: Epoxyfunktionelle Siloxane gemäß Formel (VI)

| Nr. | a1 | a5 | b1 | b5 | c1 | c4 | d | $R^1$ | $R^{12}$ |
|---|---|---|---|---|---|---|---|---|---|
| SE1 | 0 | 2 | 48 | 0 | 0 | 0 | 0 | Methyl | 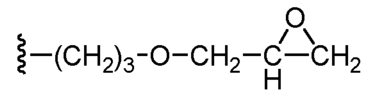 |
| SE2 | 0 | 2 | 78 | 0 | 0 | 0 | 0 | Methyl | 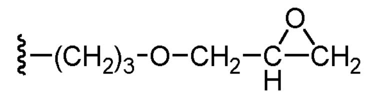 |
| SE3 | 0 | 6 | 316 | 0 | 4 | 0 | 0 | Methyl | 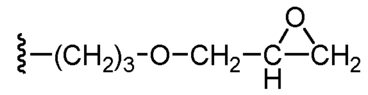 |
| SE4 | 0 | 2 | 18 | 0 | 0 | 0 | 0 | Methyl | 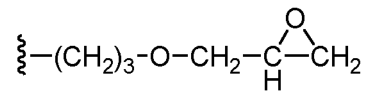 |
| SE5 | 0 | 2 | 28 | 0 | 0 | 0 | 0 | Methyl | 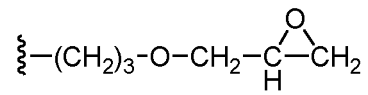 |

Einwaagen und weitere Angaben zur Herstellung der erfindungsgemäßen Siloxane gemäß Formel (I) sind den Tabelle 4 und 5 zu entnehmen.

Tabelle 4 - Teil 1: Einwaagen und weitere Angaben zur Herstellung der erfindungsgemäßen Siloxane gemäß Formel (I) (Gehaltsangaben in Gew.-% bezogen auf die Gesamtzusammensetzung)

| | B1 | B2 | B3 | B4 | B5 | B6 | B7 |
|---|---|---|---|---|---|---|---|
| SE1 | 860,2 g | | | | | | |
| SE2 | | 472,1 g | 393,4 g | 262,3 g | 262,3 g | 217,0 g | |
| SE3 | | | | | | | 297,7 g |
| SE4 | | | | | | | |
| Amid1 | 109,1 g | 39,3 g | | 21,8 g | 21,8 g | | 17,5 g |
| Amid2 | | | 39,6 g | | | 21,0 g | |
| Amid3 | | | | | | | |
| MDEA | 17,9 g | 6,4 g | 5,36 g | 3,6 g | 3,6 g | 2,9 g | 2,9 g |
| MDIPA | | | | | | | |
| DMAE | | | | | | | |
| HOAc | 30,9 g | 11,1 g | 9,3 g | 6,2 g | | 4,9 g | 4,9 g |
| INA | | | | | 16,3 g | | |
| IPA | 254,5 g | 132,2 g | 110,2 g | | 76 g | | 57,0 g |
| DPG | | | | 73,5 g | | | |
| tBuOH | | | | | | | |

(fortgesetzt)

| | B1 | B2 | B3 | B4 | B5 | B6 | B7 |
|---|---|---|---|---|---|---|---|
| DMM | | | | | | 13,0 g | |
| Destillation | ja | ja | ja | nein | ja | nein | ja |
| Abmischung | ja | ja | ja | nein | ja | ja | nein |
| Aktivgehalt[1)] | 95% | 80% | 97,5% | 80% | 97,5% | 80% | 100% |
| Lösemittel | 5% PG | 20% DPG | 2,5% PG | 20% DPG | 2,5% PG | 15% DPG<br>5% DMM | |
| Epoxy-Umsatz | 99% | 97% | 99% | 97% | 98% | 87% | 96% |
| Viskosität, 25°C, mPa*s | 3960 | 1605 | 7414 | 1102 | 4731 | 851 | n. b. |
| Amidamin Restgehalt | 0,4% | 0,17% | 0,35% | 0,6% | 0,4% | | 0,3% |

Tabelle 4 - Teil 2: Einwaagen und weitere Angaben zur Herstellung der erfindungsgemäßen Siloxane gemäß Formel (I) (Gehaltsangaben in Gew.-% bezogen auf die Gesamtzusammensetzung)

| | B8 | B9 | B10 | B11 | B12 | B13 | B14 |
|---|---|---|---|---|---|---|---|
| SE1 | | | | | | | |
| SE2 | 189,8 g | 419,7 g | 419,7 g | 288,5 g | 472,1 g | 239,9 g | |
| SE3 | | | | | | | |
| SE4 | | | | | | | 472,9 g |
| Amid1 | 15,3 g | 39,9 g | 39,9 g | 24,0 g | | | |
| Amid2 | | | | | | 23,7 g | 157,8 g |
| Amid3 | | | | | 41,1 g | | |
| MDEA | 2,5 g | 3,8 g | 3,8 g | | 6,4 g | | 21,9 g |
| MDIPA | | | | | | 4,0 g | |
| DMAE | | | | 3,4 g | | | |
| HOAc | 4,3 g | 9,9 g | 9,9 g | 4,8 g | 11,1 g | 5,6 g | 37,4 g |
| INA | | | | | | | |
| IPA | 53 g | 118 g | | | 132,7 g | 68,3 g | 121,8 g |
| DPG | | | | | | 67,9 g | |
| tBuOH | | | 118 g | 80,2 g | | | |
| DMM | | | | | | | |
| Destillation | ja | ja | ja | ja | ja | ja | ja |
| Abmischung | ja | ja | ja | ja | ja | ja | ja |
| Aktivgehalt[1)] | 97,5% | 97,5% | 97,5% | 97,5% | 97,5% | 80% | 50% |
| Lösemittel | 2,5% PG | 2,5% PG | 2,5% PG | 2,5% PG | 2,5% PG | 20% DPG | 50% PG |
| Epoxy-Umsatz | 99% | 98% | 96% | 98% | 99% | 96% | 100% |
| Viskosität, 25°C, mPa*s | 8746 | 9104 | 8546 | ca. 20000 | 7544 | 1217 | |

(fortgesetzt)

| | B8 | B9 | B10 | B11 | B12 | B13 | B14 |
|---|---|---|---|---|---|---|---|
| Amidamin Restgehalt | 0,2% | 0,6% | 0,7% | 0,8% | 0,3% | 0,1% | 0,8% |

Tabelle 4 - Teil 3: Einwaagen und weitere Angaben zur Herstellung der erfindungsgemäßen Siloxane gemäß Formel (I) (Gehaltsangaben in Gew.-% bezogen auf die Gesamtzusammensetzung)

| | B15 | B16 |
|---|---|---|
| SE5 | 217,7g | 217,7 g |
| Amid1 | 43,6 g | |
| Amid2 | | 52,6 g |
| MDEA | 7,2 g | 7,2 g |
| HOAc | 12,4 g | 12,4 g |
| IPA | 70,2 g | 72,5 g |
| Destillation | ja | ja |
| Abmischung | ja | ja |
| Aktivgehalt[1)] | 50% | 50% |
| Lösemittel | 50% PG | 50% PG |
| Epoxy-Umsatz | 100% | 98% |
| Viskosität, 25°C, mPa*s | 705 | 616 |
| Amidamin Restgehalt | 0,6% | 0,6% |

Tabelle 5: Einwaagen und weitere Angaben zur Herstellung von nichterfindungsgemäßen Siloxanen (Gehaltsangaben in Gew.-% bezogen auf die Gesamtzusammensetzung)

| | V1 | V2 | V3 | V4 |
|---|---|---|---|---|
| SE1 | | | 860,2 g | |
| SE2 | 271,2 g | 314,6 g | | 472,1 g |
| SE3 | | | | |
| Amid1 | | | 156 g | 56,1 g |
| Amid2 | | | | |
| MDEA | | 14,3 g | | |
| TEA | 14,9 g | | | |
| HOAc | 6,2 g | 7,4 g | 30,9 g | 11,1 g |
| INA | | | | |
| IPA | 73,0 g | | 254,5 g | 132,2 g |
| tBuOH | | 84,1 g | | |
| Destillation | ja | ja | ja | ja |
| Abmischung | nein | ja | ja | ja |
| Aktivgehalt[1)] | | 97,5% | 95% | 97,5% |
| Lösemittel | | 2,5% PG | 5% PG | 2,5% PG |

(fortgesetzt)

| | V1 | V2 | V3 | V4 |
|---|---|---|---|---|
| Epoxy-Umsatz | 0% 2-phasig | 92,3% | 98% | 97% |
| Viskosität, 25°C, mPa*s | - | 8429 | 4631 | 9114 |
| Amidamin Restgehalt | - | - | 1,8 % | 1,4 % |
| 1) Aktivgehalt = Massenanteil der Siloxane (Wirkstoffe) bezogen auf die Gesamtmasse der Zusammensetzung | | | | |

Bei der Synthese der erfindungsgemäßen Beispiele B1 bis B14 wurden Mischungen von Dialkanolaminen und Amidaminen eingesetzt. Im Fall der Vergleichsbeispiele V1 und V2 wurden dagegen keine Amidamine sondern nur Alkanolamine verwendet, und zwar ein Trialkanolamin bei V1 und ein Dialkanolamin bei V2. Bei der Synthese der Vergleichsbeispiele V3 und V4 wurden wiederum ausschließlich Amidamine und keine Alkanolamine eingesetzt. Die nichterfindungsgemäßen Siloxanzusammensetzungen V3 und V4 wiesen einen höheren Restgehalt an Amidamin auf als die erfindungsgemäßen Siloxanzusammensetzungen B1 bis B14. Bei V1 war eine Phasentrennung zu beobachten, eine Reaktion des epoxyfunktionellen Siloxans mit dem Alkanolamin konnte nicht festgestellt werden. Bei V2 wurde dagegen ein Epoxy-Umsatz beobachtet, eine Reaktion fand hier also statt. Da Amidamine weder bei V1 noch V2 verwendet wurden, erübrigt sich die Angabe eines Restgehalts.

Eine nichterfindungsgemäße Abmischung aus 70 Teilen V4 mit 30 Teilen V2 wurde durch Rühren mit einem Magnetrührfisch bei Raumtemperatur in einem Probenglas hergestellt und der HPLC-Messung unterworfen. Die Messung des Amidamin-Restgehaltes ergab 0,8% gegenüber der Theorie von 0,9%. Das erfindungsgemäße Produkt B5, welches durch Reaktion der gleichen Vorstufe SE2 mit einer Mischung von 0,7 Moläquivalenten Amid1 und 0,3 Moläquivalenten MDEA bezogen auf 1 Moläquivalent Epoxygruppen hergestellt wurde, weist einen per HPLC gemessenen Restgehalt Amidamin von 0,4% auf. Der Lösemittelanteil der Mischung aus V4 und V2 sowie bei B5 entsprach in allen Fällen 2,5% PG. Dieser Vergleich zeigt, dass durch die erfindungsgemäße Herstellung der neuen gemischfunktionellen Silikonquats und deren Zusammensetzungen signifikant niedrigere Amidaminrestgehalte erzielt werden als im Vergleich durch die naheliegende Abmischung erreichbar sind.

Lagerstabilitätstests der Silikonquats:

Jeweils zwei 100 ml Probengläser mit Schraubverschluss wurden jeweils zur Hälfte mit den Silikonquats B5, B6 und B7 gefüllt. Ein Probenglas wurde bei Raumtemperatur (RT) verschlossen gelagert und das jeweils zweite Probenglas wurde verschlossen in einem handelsüblichen LaborTrockenschrank der Firma Binder bei 50°C gelagert. Nach definierten Lagerzeiten wurde die Viskosität der Proben bei 25°C bestimmt und / oder der Gehalt an zyklischen Siloxanen per GC-Analytik überprüft. Zur besseren Vergleichbarkeit und Messbarkeit wurde der 100%ige hochviskose Silikonquat B7 mit 20% DPG auf 80% Aktivgehalt abgemischt. Die Ergebnisse der Lagerstabilitätstests sind in Tabelle 6 zusammengefasst.

Tabelle 6: Ergebnisse der Lagerstabilitätstests der Silikonquats (Inhaltsangaben in Gew.-% bezogen auf die Gesamtzusammensetzung)

| Probe | Lagerzeit | Lagertemperatur | Viskosität bei 25°C, [mPa*s] | Gehalt D4, [Gew%] |
|---|---|---|---|---|
| B5 | 0 Wochen | RT | 4731 | 0,02 |
| B5 | 4 Wochen | RT | n. b. | 0,02 |
| B5 | 12 Wochen | RT | n. b. | 0,03 |
| B5 | 4 Wochen | 50°C | n. b. | 0,06 |
| B6 | 0 Wochen | RT | 851 | 0,08 |
| B6 | 4 Wochen | RT | 1031 | 0,08 |
| B6 | 12 Wochen | RT | 1093 | 0,08 |
| B7-80%ig | 0 Wochen | RT | 6396 | 0,09 |
| B7-80%ig | 4 Wochen | RT | 5330 | 0,09 |
| B7-80%ig | 8 Wochen | RT | 6001 | 0,09 |

(fortgesetzt)

| Probe | Lagerzeit | Lagertemperatur | Viskosität bei 25°C, [mPa*s] | Gehalt D4, [Gew%] |
|---|---|---|---|---|
| B7-80%ig | 12 Wochen | RT | 6888 | 0,09 |

Die Lagertests zeigen, dass sich keine signifikanten Veränderungen der Viskosität ergeben und die Silikonquats auch nach längerer Lagerzeit noch gut dosiert werden können. Weiterhin zeigen die Lagertests, dass die Neubildung zyklischer Siloxane während der Lagerzeit minimiert ist, dahingehend dass ≤0,05 Gew.-% Zunahme an D4 festgestellt wird. Die besonders gut ausdestillierte Probe 5 zeigt, dass über einen längeren Lagerzeitraum der Anteil von D4 <0,1 Gew.-% gegeben ist. Der Gehalt an D4 in der jeweiligen Probe hängt von der Destillationsgüte bei der Herstellung ah und nicht von der Lagerzeit.

Anwendungstechnische Bespiele:

Verwendete Materialien:

Tabelle 7: Emulgatoren

| Emulgatoren | Handelsname |
|---|---|
| C12-15 Pareth-7 / 9 / 12 | Tomadol® 25-7, Evonik<br>Tomadol® 25-9, Evonik<br>Tomadol® 25-12, Evonik |
| Isotridecanol - 6 / 8 / 12 | Lutensol® TO 6, BASF<br>Lutensol® TO 8, BASF<br>Marlipal® O13/120, BASF |
| Laureth- 6 / 12 | Lutensol® AO 6, BASF<br>Marlipal 24/120, Sasol |
| Sorbitansesquioctanoat | TEGO® SQS 25, Evonik |
| Methyldiisopropanolamin Esterquat | REWOQUAT® CR 3099, Evonik |

Tabelle 8: Hilfsstoffe

| Weitere Silikonverbindungen | Handelsname |
|---|---|
| Alkyl/Polyether-modifiziertes Silicon-Copolymer | TEGOPREN® 7008, Evonik<br>TEGOPREN® 7009, Evonik |

Flächengebilde:

Textilien: Baumwollgewebe(Flächengewicht 205 g/$m^2$, Dicke: 400 μm); Polyestermischgewebe (65 Gew.-% Polyester und 35 Gew.-% Baumwolle, Flächengewicht 170 g/$m^2$, Dicke: 200 μm); Polyamidgewebe (Polyamid 6.6, Flächengewicht 65 g/$m^2$, Dicke: 50 μm); alle Proben von WFK-Testgewebe GmbH (Christenfeld 10 41379 Brüggen).

Formulierung und Ausrüstung:

Herstellung der Emulsionen:

Die synthetisierten Siloxanzusammensetzungen ausgewählt aus B1 bis B14 (basierend auf Mischungen aus Alkanolamin und Amidamin), V2 (basierend auf Alkanolamin), V4 (basierend auf Amidamin) sowie V5 (Magnasoft® DerMa NT als kommerzielles Vergleichsprodukt) wurden vorgelegt und falls erforderlich durch Zugabe eines Glykols auf den gewünschten Aktivgehalt, also den gewünschten Massenanteil an Wirkstoff (Siloxan), weiter verdünnt. Dies

erwies sich als vorteilhaft, da besonders gute Ergebnisse nämlich erzielt wurden, wenn die Wirkstoffe aus einem Lösemittel heraus weiter umgesetzt wurden, insbesondere wenn sie als Mischungen mit Lösemitteln mit einem Aktivgehalt von 80% eingesetzt werden. Im Anschluss daran wurden die so erhaltenen Mischungen RE1 bis RE10 vorgelegt und die Emulgatoren sowie gegebenenfalls weitere Hilfsstoffe und/oder Glykole hinzugegeben. Danach wurde langsam unter ständigem Rühren mit einem Propellerrührer Wasser zugegeben. Der pH-Wert wurde durch anschließende Zugabe von Essigsäure auf einen pH-Wert von ca. 4 eingestellt. Es wurde solange gerührt bis die Zusammensetzung homogen ist. Man erhielt so die Emulsionen E1 bis E26 und C1 bis C5.

Foulardverfahren (Typ HVF, Mathis AG):

Zur Ausprüfung der jeweiligen Emulsionen wurden die oben beschriebenen Gewebe mit einer Flotte, die jeweils 8 g/l der entsprechenden Emulsion enthielt, appliziert, auf ca. 70 bis 80 Gew.-% Flottenaufnahme abgequetscht und getrocknet. Die angewendeten Werte für Druck und Geschwindigkeit sind in Tabelle 9 zu entnehmen. Die Auftragung im Foulard verlief bei Raumtemperatur.

Tabelle 9: Beim Foulard Verfahren eingesetzte Drücke und Walzengeschwindigkeiten.

| Bezeichnung | Druck [bar] | Geschwindigkeit [m/min] |
|---|---|---|
| Baumwollgewebe | 2,4-5,8 | 2 |
| Polyestermischgewebe | 1,0 - 1,2 | 2 |
| Polyamidgewebe | 1,0 | 1 - 2 |

Ausziehverfahren (Exhaust) Verfahren aus lösungsmittelhaltigen Formulierungen:

Zur Ausprüfung der Wirkstoffe wurden die oben genannten Gewebe mit einer Flotte, die jeweils 20 g/l des entsprechenden Wirkstoffes enthielt, ausgerüstet. Es wurde ein Flottenverhältnis (Gewebe zu Flotte) von 1:15 gewählt. Verwendete Lösungsmittel sind Wasser, Butylacetat und Ethylacetat. Das Testgewebe wurde für 30 min in der Flotte unter kontinuierlichem Schütteln auf der Schüttelplatte (Typ: 3006, Hersteller: GFL) behandelt. Nach 30 min wurde das Testgewebe aus dem Bad entfernt, leicht ausgewrungen, aufgeschüttelt und getrocknet. Ein Blindwert wurde unter den gleichen Bedingungen nur mit demineralisiertem Wasser behandelt.

Trocknungsverfahren (Lab Dryer Typ LTE, Mathis AG, Ventilatordrehzahl 2000 U/min):
Die Flächengebilde wurden bei 105 °C (plus Verweilzeit, d.h. der Aufheizzeit des Textilgewebes) 2 min lang getrocknet und anschließend bei 160 °C bis 180 °C (ohne Verweilzeit) für 0,5 min bis 1 min kondensiert um die Ausrüstung zu fixieren. Die genauen Bedingungen sind in Tabelle 10 zusammengefasst.

Tabelle 10: Bedingungen für das Trocknungsverfahren

| | Trocknung | | Fixierung | |
|---|---|---|---|---|
| | [°C] | [min] | [°C] | [min] |
| Baumwollgewebe (Exhaust) | 105 | 2,0 | 160 | 1,0 |
| Polyestermischgewebe (Exhaust) | 105 | 2,0 | 180 | 0,5 |
| Polyamidgewebe (Exhaust) | 105 | 2,0 | 180 | 0,5 |
| Baumwollgewebe (Foulard) | 105 | 2,0 | 150 | 3,0 |
| Polyestermischgewebe (Foulard) | 105 | 2,0 | 150 | 3,0 |
| Polyamidgewebe (Foulard) | 105 | 2,0 | 150 | 3,0 |

Prüfung der Ausrüstung:

Weichgriff/Warengriff:

Der Weichgriff/Warengriff ist ein fundamentaler Qualitätsparameter eines Gewebes. Er kann durch z.B. Glätte, Kompressibilität, und Steifigkeit beschrieben werden. Normalerweise wird der Weichgriff/Warengriff durch subjektive Beurteilung über ein Austesten per Hand bestimmt. Zusätzlich gibt es hierzu Messinstrumente die dies objektiv bestimmen.

Griffbeurteilung (Grifftest) über Messinstrumente (TSA-Wert / Hand-Feel):

Ein zugeschnittenes Stück Textilgewebe wurde nach vorheriger Konditionierung (4 Stunden) bei 25 °C und 50 % rel. Luftfeuchtigkeit in den TSA (Tissue Soft Analyzer, Fa Emtec Electronic GmbH) eingelegt und festgespannt. Das Messgerät ermittelt dann Einzelwerte für Weichheit, Glattheit und Steifigkeit des Textilgewebes und ermittelte daraus den Gesamteindruck, das Handgefühl (*HF*/*Hand-Feel*). Dieser TSA-Wert (HF-Wert) wurde mittels eines Algorithmus der Firma EMTEC, der speziell auf Textilien ausgelegt ist, ermittelt Ein höherer HF-Wert bedeutet eine höhere Weichheit. Die Bewertungen erfolgen im Vergleich zu einer analogen Behandlung ohne Wirkstoff.

Griffbeurteilung (Grifftest) per Hand (Paneltest):

Zur Beurteilung des Weichgriffs/Warengriffes wurde ein erfahrenes Team aus 10 Fachleuten zusammengestellt, das die anonymisierten Griffmuster, der mit den Emulsionen ausgerüsteten oben genannten Gewebe, mit Hilfe eines Handpaneltests auf einer Skala von 1 bis 5 bewertete, wobei die Note 1 einen sehr schlechten und die Note 5 einen sehr guten Weichgriff/Warengriff bedeutet. Das Ergebnis des Paneltests wird als Mittelwert aller Bewertungen angegeben. Bei den Griffmustern aus Maschenware wurde zusätzlich eine nicht offensichtlich gekennzeichnete unbehandelte Probe hinzugelegt.

Antistatische Eigenschaften:

Die Messung der antistatischen Eigenschaften erfolgt in Anlehnung an die DIN 54345 T.1 (Ringelektrode) mit 100 V Messspannung (Messgerät Tera-Ohm-Meter 6206). Durch das Ausrüsten mit Antistatika wird der elektrische Widerstand an textilen Flächengebilden verringert. Die Reduzierung des Widerstandes ist ein Maß für die antistatische Wirksamkeit.

Zur Bestimmung der antistatischen Eigenschaften wurden folgende Geräte und Gewebe verwendet:

- Standardtestgewebe : Polyester (100%, Typ 30 A von wfk / Krefeld)
- Waschmaschine zur Vorbehandlung und Foulard zur Ausrüstung des Gewebes
- Klimaraum (23 ± 1°C , 50 - 60 % r.F)
- Tera-Ohm-Meter 6206 (Firma Eltex)
- Messelektrode Typ 6216 (Firma Eltex) n. DIN 54345 T.1

Die ausgerüsteten Gewebe werden vor der Messung für einen Tag im Klimaraum gelagert um eine ausgeglichene Feuchte zu gewährleisten. 10x15 cm Stücke werden auf eine ebene Fläche gelegt und die Ringelektrode darauf platziert. Der Widerstand der unterschiedlichen Ausrüstungen wird gemessen.

Anwendungstechnische Ergebnisse:

Die Syntheseprodukte B3 bis B12 sowie V2 und V4 als auch ein marktübliches Vergleichsprodukt V5 wurden, sofern der Aktivgehalt nicht bereits bei 80 Gew.-% bezogen auf die Zusammensetzung betrug, durch Zugabe von Butyldiglykol (BDG), auf einen einheitlichen Aktivgehalt von 80% gebracht. Die so erhaltenen Mischungen RE1 bis RE10 sind in Tabelle 8 zusammengefasst. Aus diesen Mischungen wurden, wie oben beschrieben, die Emulsionen E1 bis E26 und C1 bis C12 hergestellt. Die Zusammensetzungen der Emulsionen und ihre Eigenschaften sind in den folgenden Tabellen zusammengefasst.

Tabelle 11: Vormischungen für die vergleichende anwendungstechnische Prüfung

| | RE8 | RE1 | RE2 | RE4 | RE5 | RE6 | RE7 | RE3 | RE9 | RE10 |
|---|---|---|---|---|---|---|---|---|---|---|
| Siloxan | B3 | B4 | B8 | B9 | B10 | B11 | B12 | V2 | V4 | V5 |
| zusätzliches Lösemittel | BDG | - | BDG | BDG | BDG | BDG | BDG | BDG | BDG | BDG |

Tabelle 12: Erfindungsgemäße Emulsionen und ihre Eigenschaften (Gehaltsangaben in Gew.-% bezogen auf die Gesamtzusammensetzung)

| % | E1 | E2 | E3 | E4 | E5 |
|---|---|---|---|---|---|
| RE8 | 18,2 | 23,8 | 32,5 | 25 | 25 |

(fortgesetzt)

| % | E1 | E2 | E3 | E4 | E5 |
|---|---|---|---|---|---|
| TEGOPREN® 7008 | 5,6 | - | - | - | - |
| TOMADOL® 25-7 | 7,7 | - | - | - | 1,7 |
| TOMADOL® 25-9 | - | 2,5 | - | 1,5 | - |
| TOMADOL® 25-12 | - | - | - | - | 3,4 |
| TEGO® SQS 25 | - | 2,5 | 5,5 | - | - |
| REWOQUAT® CR 3099 | - | - | 5,7 | - | - |
| Butyldiglycol | 8,6 | - | - | 10,0 | - |
| Dipropylengykol | - | 5,0 | 15,0 | - | - |
| Wasser | 60,1 | 66,2 | 41,4 | 63,2 | 69,6 |
| Essigsäure | 0,3 | - | - | 0,3 | 0,3 |
| Aussehen | klare Lösung | milchig weiß | klare Lösung | klare Lösung | opak |
| Emulsionsart | Microemulsion | Macroemulsion | Microemulsion | Microemulsion | Microemulsion |
| Eigenschaft | bauschig | bauschig | substantiv | kosteneffizient | lösemittelreduziert |
| Grifftest (TSA-Wert) | 39,2 | 38,8 | 38,4 | 37,6 | 37,2 |

Tabelle 13: Nichterfindungsgemäße Emulsionen und ihre Eigenschaften (Gehaltsangaben in Gew.-% bezogen auf die Gesamtzusammensetzung)

| % | C1 | C2 | C3 | C4 | C5 |
|---|---|---|---|---|---|
| RE9 | 18,2 | 23,8 | 32,5 | 25 | 25 |
| TEGOPREN® 7008 | 5,6 | - | - | - | - |
| TOMADOL® 25-7 | 7,7 | - | - | - | 1,7 |
| TOMADOL® 25-9 | - | 2,5 | - | 1,5 | - |
| TOMADOL® 25-12 | - | - | - | - | 3,4 |
| TEGO® SQS 25 | - | 2,5 | 5,5 | - | - |
| REWOQUAT® CR 3099 | - | - | 5,7 | - | - |
| Butyldiglycol | 8,6 | - | - | 10,0 | - |
| Dipropylengykol | - | 5,0 | 15,0 | - | - |
| Wasser | 60,1 | 66,2 | 41,4 | 63,2 | 69,6 |
| Essigsäure | 0,3 | - | - | 0,3 | 0,3 |
| Aussehen | klare Lösung | milchig weiß | klare Lösung | klare Lösung | opak |

(fortgesetzt)

| % | C1 | C2 | C3 | C4 | C5 |
|---|---|---|---|---|---|
| Emulsionsart | Microemulsion | Macroemulsion | Microemulsion | Microemulsion | Microemulsion |
| Eigenschaft | bauschig | bauschig | substantiv | kosteneffizient | lösemittelreduziert |
| Grifftest (TSA-Wert) | 37,4 | 37,0 | 37,3 | 36,5 | 36,3 |

Die erfindungsgemäßen Emulsionen E1 bis E5 aus Tabelle 12 unterscheiden sich von den entsprechenden nichterfindungsgemäßen Emulsionen C1 bis C5 aus Tabelle 13 nur im verwendeten Wirkstoff bei ansonsten gleicher Zusammensetzung. Die erfindungsgemäßen Emulsionen zeigen im Vergleich mit den nichterfindungsgemäßen Emulsionen deutlich verbesserte TSA-Werte (HF-Werte, *Hand-Feel*). Die verbesserten Griffeigenschaften konnten in Paneltests bestätigt werden. Neben dem *Hand-Feel* ist auch eine gute Wasseraufnahme für den Tragekomfort relevant. Hier zeigt die Ausrüstung mit den erfindungsgemäßen Emulsionen keine Nachteile gegenüber einer Ausrüstung mit Emulsionen, die auf Wirkstoffen des Standes der Technik basieren. Je nach Stoffqualität (Dicke und Webart) und Formulierung lassen sich sogar bessere Wasseraufnahmevermögen bzw. Wasserrückhaltevermögen erzielen. Das Wasseraufnahmevermögen bzw. Wasserrückhaltevermögen wird darüber hinaus auch durch die Wahl der eingesetzten Emulgatoren beeinflusst.

Tabelle 14: Erfindungsgemäße Emulsionen mit Hilfsstoffen zur Verbesserung der Bauschigkeit und ihre Eigenschaften (Gehaltsangaben in Gew.-% bezogen auf die Gesamtzusammensetzung)

| % | E6 | E7 | E8 | E9 | E10 | E11 |
|---|---|---|---|---|---|---|
| RE0 | 20 | - | 20 | - | 20 | - |
| RE1 | - | 20 | - | 20 | - | 20 |
| TEGOPREN® 7008 | 4,0 | 4,0 | - | - | - | - |
| TEGOPREN® 7009 | - | - | 4,0 | 4,0 | - | - |
| Lutensol® TO 8 | 1,5 | 1,5 | 1,5 | 1,5 | 1,5 | 1,5 |
| Butyldiglykol | 10,0 | 10,0 | 10,0 | 10,0 | 10,0 | 10,0 |
| Dipropylengykol | 5,5 | 5,5 | 5,5 | 5,5 | 5,5 | 5,5 |
| Wasser | 58,7 | 58,7 | 58,7 | 58,7 | 58,7 | 58,7 |
| Essigsäure | 0,3 | 0,3 | 0,3 | 0,3 | 0,3 | 0,3 |
| Aussehen | klare Lösung | klare Lösung | Separation | Separation | klare Lösung | klare Lösung |
| Grifftest (TSA-Wert) | 39,3 | 38,1 | 38,0 | 38,4 | 36,8 | 37,2 |
| Paneltest (1-5; 5 = Bestnote) | 5 | 4,3 | 4,3 | 4,3 | 3,8 | 4,0 |

Tabelle 14 zeigt, dass sich durch den zusätzlichen Einsatz von Hilfsstoffen/Additiven zur Verbesserung der Bauschigkeit (Tegopren® 7008 und Tegopren® 7009) die Griffbeurteilungen weiter verbessern lassen. Dies gilt gleichermaßen für Griffbeurteilungen, die über Messinstrumente (TSA-Wert, Hand-Feel) als auch per Hand (Paneltest) bestimmt wurden. Die erfindungsgemäßen Wirkstoffe zeigen in Kombination mit einem Hilfsstoff/Additiv zur Verbesserung der Bauschigkeit dabei die besten Ergebnisse, wenn sie aus einem Lösemittel heraus umgesetzt wurden, insbesondere wenn sie als Mischungen mit einem Aktivgehalt von 80% eingesetzt wurden. Diese Ausrüstung ist auch bei einer Griffbeurteilung im Rahmen eines Paneltests überzeugend.

Tabelle 15: Emulsionen mit kostenoptimierten Hilfsstoffen und ihre Eigenschaften (Gehaltsangaben in Gew.-% bezogen auf die Gesamtzusammensetzung)

| % | E12 | C6 | E13 | E14 | C7 | E15 |
|---|---|---|---|---|---|---|
| RE2 | 20,5 | - | - | - | - | - |
| RE3 | - | 20,5 | - | - | - | - |
| RE4 | - | - | 20,5 | - | - | - |
| RE5 | - | - | - | 20,5 | - | - |
| RE9 | - | - | - | - | 20,5 | - |
| RE6 | - | - | - | - | - | 20,5 |
| Isotridecanol 8EO | 1,5 | 1,5 | 1,5 | 1,5 | 1,5 | 1,5 |
| Butyldiglykol | 10,0 | 10,0 | 10,0 | 10,0 | 10,0 | 10,0 |
| Dipropylengykol | 4,5 | 4,5 | 4,5 | 4,5 | 4,5 | 4,5 |
| Wasser | 63,2 | 63,2 | 63,2 | 63,2 | 63,2 | 63,2 |
| Essigsäure | 0,3 | 0,3 | 0,3 | 0,3 | 0,3 | 0,3 |
| Aussehen | klare Lösung | Separation | klare Lösung | klare Lösung | Separation | klare Lösung |
| Grifftest (TSA-Wert) | 38,5 | 37,3 | 37,1 | 36,7 | 37,3 | 37,6 |

Tabelle 15 zeigt, dass bei Verwendung von kostenoptimierten Hilfsstoffen, wie Isotridecanol 8EO, ebenfalls sehr gute Griffbeurteilungen bei den erfindungsgemäßen Emulsionen erreicht werden, ohne dass eine Phasenseparation zu beobachten ist.

Tabelle 16: Erfindungsgemäße und nichterfindungsgemäße Emulsionen mit Additiven zur Verbesserung der Bauschigkeit und ihre Eigenschaften (Gehaltsangaben in Gew.-% bezogen auf die Gesamtzusammensetzung)

| % | E16 | C8 | E17 | E18 | C10 | E19 |
|---|---|---|---|---|---|---|
| RE2 | 18,5 | - | - | - | - | - |
| RE3 | - | 18,5 | - | - | - | - |
| RE4 | - | - | 18,5 | - | - | - |
| RE5 | - | - | - | 18,5 | - | - |
| RE9 | - | - | - | - | 18,5 | - |
| RE6 | - | - | - | - | - | 18,5 |
| TEGOPREN® 7008 | 5,6 | 5,6 | 5,6 | 5,6 | 5,6 | 5,6 |
| Rewopal® LA 6 | 7,7 | 7,7 | 7,7 | 7,7 | 7,7 | 7,7 |
| Butyldiglykol | 5,0 | 5,0 | 5,0 | 5,0 | 5,0 | 5,0 |
| Wasser | 62,9 | 62,9 | 62,9 | 62,9 | 62,9 | 62,9 |
| Essigsäure | 0,3 | 0,3 | 0,3 | 0,3 | 0,3 | 0,3 |
| Aussehen | klare Lösung | Separation | klare Lösung | klare Lösung | klare Lösung | klare Lösung |
| Grifftest (TSA-Wert) | 38,3 | 37,9 | 37,3 | 38,2 | 37,4 | 37,3 |

Tabelle 16 zeigt die Vorteile der erfindungsgemäßen Emulsionen. Die nichterfindungsgemäße Emulsion C8 zeigt gute Ergebnisse im Grifftest und keinen Restgehalt an Amidaminen, da sie auf einem Wirkstoff (V2) basiert, der nur aus Alkanolaminen als tertiäre Amine hergestellt wurde. Die Emulsion C8 hat aber den Nachteil, dass eine Phasenseparation zu beobachten ist. Umgekehrt gibt es keine Phasenseparation bei der nichterfindungsgemäßen Emulsion C10, sondern es wird eine klare Lösung erhalten. Allerdings fällt hier die Griffbeurteilung deutlich schlechter aus. Da die Emulsion zudem auf einem Wirkstoff (V4) basiert, der nur aus Amidaminen als tertiäre Amine hergestellt wurde, ist der Restgehalt von Amidaminen hoch. Die erfindungsgemäßen Zusammensetzungen führen zu einem vorteilhaften Phasenverhalten, einem niedrigen Restgehalt von Amidaminen sowie einer sehr guten Griffbeurteilung.

E12 und E16 zeigen eine besonders gute Griffbeurteilung neben der guten Formulierbarkeit.

Tabelle 17: Erfindungsgemäße Emulsionen und ihre Eigenschaften - Einfluss der Kettenlänge der Fettsäure oder des Alkoxylates (Gehaltsangaben in Gew.-% bezogen auf die Gesamtzusammensetzung)

| % | E20 | E21 | E22 |
|---|---|---|---|
| RE2 | 20,5 | - | - |
| RE7 | - | 20,5 | - |
| RE8 | - | - | 20,5 |
| Lutensol® TO 8 | 1,5 | 1,5 | 1,5 |
| Dipropylenglykol | 4,5 | 4,5 | 4,5 |
| Butyldiglykol | 10,0 | 10,0 | 10,0 |
| Wasser | 63,2 | 63,2 | 63,2 |
| Essigsäure | 0,3 | 0,3 | 0,3 |
| Aussehen | klare Lösung | klare Lösung | klare Lösung |
| Grifftest (TSA-Wert) | 37,2 | 37,5 | 37,6 |

Tabelle 17 zeigt den Einfluss von unterschiedlichen Fettsäureamiden (E20: Cocoyl, E21: Palmityl, E22: Stearyl). Unabhängig von der Wahl des Fettsäureamids werden sehr gute Ergebnisse im Grifftest erhalten. Weiterhin zeigt sich, dass die Bewertungen im Grifftest umso besser ausfallen, je länger die Alkylkettenlänge des Säurerests des Amidamins ist. Dieser Zusammenhang wurde auch im Paneltest bestätigt.

Tabelle 18: Emulsionen und ihre Eigenschaften (Gehaltsangaben in Gew.-% bezogen auf die Gesamtzusammensetzung)

| % | E23 | C11 | C12 |
|---|---|---|---|
| RE8 | 20,5 | - | - |
| RE9 | - | 20,5 | - |
| RE10 | - | - | 20,5 |
| Isotridecanol 6EO | 10,5 | 10,5 | 10,5 |
| Isotridecanol 12EO | 1,8 | 1,8 | 1,8 |
| Wasser | 66,8 | 66,8 | 66,8 |
| Essigsäure | 0,4 | 0,4 | 0,4 |
| Aussehen | klare Lösung | klare Lösung | klare Lösung |
| Grifftest (TSA-Wert) | 37,9 | 37,5 | 36,7 |
| Panel Test (1-5; 5=best) | 4,8 | 4,5 | 4,0 |

Die Ergebnisse in Tabelle 18 zeigen, dass die Verwendung der erfindungsgemäßen Wirkstoffe zu einer besseren Griffbeurteilung im Vergleich zu nichterfindungsgemäßen Wirkstoffen führt, insbesondere zu kommerziell erhältlichen Wirkostoffen führt.

Tabelle 19: Erfindungsgemäße Emulsionen mit Additiven zur Verbesserung der Bauschigkeit und ihre Eigenschaften (Gehaltsangaben in Gew.-% bezogen auf die Gesamtzusammensetzung)

| % | E24 | E25 | E26 |
|---|---|---|---|
| RE2 | 20,5 | - | - |
| RE7 | - | 20,5 | - |
| RE8 | - | - | 20,5 |
| TEGOPREN® 7008 | 5,6 | 5,6 | 5,6 |
| Dodecanol 6EO | 7,7 | 7,7 | 7,7 |
| Dipropylenglykol | 3,3 | 3,3 | 3,3 |
| Butyldiglykol | 5,0 | 5,0 | 5,0 |
| Wasser | 63,5 | 63,5 | 63,5 |
| Aussehen | klare Lösung | klare Lösung | klare Lösung |
| Griff Test (TSA-Wert) | 36,8 | 37,3 | 37,6 |

Auch die Ergebnisse in Tabelle 19 machen deutlich, dass die Verwendung erfindungsgemäßer Siloxane zu einer besseren Griffbeurteilung führt.

Es bleibt festzuhalten, dass die Verwendung der erfindungsgemäßen Siloxane zu einer besseren Griffbeurteilung, einem besseren Phasenverhalten und/oder einem geringeren Amidamingehalt führen.

Zur Prüfung der antistatischen Eigenschaften wurden die Silikonquats in demineralisiertem Wasser auf einen Aktivgehalt von 20 Gew.% verdünnt und anschließend mit dem oben beschriebenen Verfahren durch einen Foulardierprozess auf das Polyestergewebe aufgetragen.

Tabelle 20: Antistatikzusammensetzungen (Gehaltsangaben in Gew.-% bezogen auf die Gesamtzusammensetzung)

| | C13 | E27 | C14 | C15 | E28 |
|---|---|---|---|---|---|
| Silikonquat | 40% V6 [2)] | 40% B14 [3)] | 21% V7 [4)] | 25% V8 [5)] | 25% RE8 [6)] |
| Wasser | 60% | 60% | 79% | 75% | 75% |
| Aktivgehalt | 20% | 20% | 20% | 20% | 20% |
| Aussehen | klar | opak | milchig | milchig | 2 Phasen |
| [2)] Zusammensetzung mit einem Silikonquat hergestellt aus Amid1 und SE4 (b1 = 18)<br>[3)]Zusammensetzung mit einem Silikonquat hergestellt aus Amid2 und SE4 (b1 = 18)<br>[4)] Zusammensetzung mit einem Silikonquat hergestellt aus Amid1 und SE2 (b1 = 78)<br>[5)] Zusammensetzung mit einem Silikonquat hergestellt aus Amid1 und SE2 (b1 = 78)<br>[6)] Zusammensetzung mit einem Silikonquat hergestellt aus Amid2 und SE2 (b1 = 78) | | | | | |

Der Vergleich von E27 mit C13 sowie von E28 mit C14 und C15 zeigt, dass bei gleicher Siloxankettenlänge bei den erfindungsgemäßen Zusammensetzungen E27 und E28 eine etwas schlechtere Löslichkeit zu sehen ist. Eingesetzt als Antistatik- und Gleitmittel-Wirkstoff wird aber durch die etwas schlechtere Löslichkeit die unerwünschte Penetration des Produktes in die textile Fasermatrix reduziert. Der Antistatik-Wirkstoff verbleibt stärker auf der Oberfläche als bei den Vergleichsbeispielen und führt zu einer besseren Gleitwirkung bei einer ähnlichen antistatischen Wirkung. Um die Produkte zu vergleichen, wurden keine weiteren Hilfsstoffe, die üblicherweise in Spinnpräparationen verwendet werden, eingesetzt. Im Fall von E28 war eine Phasentrennung zu beobachten war. Aus diesem Grund wurden hierfür keine antistatischen Messungen durchgeführt.

Tabelle 21: Antistatische Eigenschaften

| | Widerstand in Ω (0,01 g Probe / 1g Textil) | Widerstand in Ω (0,02g Probe / 1g Textil) | Widerstand in Ω (0,03g Probe / 1g Textil) |
|---|---|---|---|
| C13 | 5,94E+08 | 4,75E+08 | 2,80E+07 |
| E27 | 2,29E+09 | 1,37E+09 | 4,96E+08 |

(fortgesetzt)

| | Widerstand in Ω (0,01 g Probe / 1g Textil) | Widerstand in Ω (0,02g Probe / 1g Textil) | Widerstand in Ω (0,03g Probe / 1g Textil) |
|---|---|---|---|
| C14 | 1,03E+10 | 2,81E+10 | 2,02E+10 |
| C15 | 9,05E+10 | 3,83E+10 | 8,41E+09 |
| E28 | nicht bestimmt | nicht bestimmt | nicht bestimmt |
| Blindwert 7) | 4,13E+11 | 4,13E+11 | 4,13E+11 |
| 7) Wert des unbehandelten Polyestergewebes | | | |

Die Proben mit kurzkettigen Siloxanen C13 und E27 zeigen eine ausreichende antistatische Wirkung. Die antistatischen Eigenschaften des erfindungsgemäßen Beispiels E27 fallen etwas geringer aus als beim nichterfindungsgemäßen Beispiel C13. Dieser Unterschied ist akzeptabel und wird durch die bessere Gleitwirkung und den besseren Weichgriff/Warengriff des erfindungsgemäßen Beispiels mehr als ausgeglichen. Das erfindungsgemäße Beispiel neigt weniger zur Penetration in das Gewebe und verbessert somit den Weichgriff/Warengriff. Die Bestimmung des Widerstands erfolgt unter den gemäß der genannten DIN einzuhaltenden idealisierten Rahmenbedingungen. In der industriellen Anwendung zeigt sich aber, dass die antistatische Ausrüstung im Falle von C13 mit der Zeit nachlässt, wohingegen die antistatischen Eigenschaften von E27 weitgehend unverändert bleiben. Es wird davon ausgegangen, dass die unter realen Bedingungen erhöhte Reibung zu einer erhöhten Penetration der nichterfindungsgemäßen Probe in das Textil führt, sodass die antistatische Ausrüstung mit der Zeit nachlässt. Das erfindungsgemäße Produkt neigt dagegen weniger zur Penetration und führt zu einer weitgehend konstanten Ausrüstung unter produktionsüblichen Stressbedingungen.

Wasserverdünnbare Formulierungen für die Autopflege:

Verwendete Materialien:

| | |
|---|---|
| Carspray 90 | Di-(Oleyl carboxyethyl) Hydroxyethyl Methylammonium Methosulfat |
| REWOCARE DOC | Diethylhexylcarbonat |
| TEGO POLISH ADDITIV 5 | Decamethylcyclopentasiloxan, D5 |
| REWOPAL MPG 40 | Tetraethylenmonophenylether |
| DPG | Dipropylenglykol |
| TEGOPREN 6922 | Quaternium 80 (Silikonquat) |
| REWOQUAT CR 3099 | Di Oleic Acid Isopropylester Dimethylammonium Methosulfat |
| Butyl Cellosolve | 2-Butylethanol |
| REWOCARE OT | Isooctyl Tallowate |

Tabelle 22: Autopflegeformulierungen (Gehaltsangaben in Gewichtsteilen)

| Bestandteil | Formulierung mit Benchmark | Formulierung mit B15 | Formulierung mit B16 |
|---|---|---|---|
| Carspray 90 | 12 Teile | 12 Teile | 12 Teile |
| REWOCARE® DOC | 5 Teile | 5 Teile | 5 Teile |
| D5 | 2 Teile | 2 Teile | 2 Teile |
| REWOPAL® MPG 40 | 6 Teile | 6 Teile | 6 Teile |
| DPG | 8 Teile | 8 Teile | 8 Teile |
| TEGOPREN® 6922 | 0,8 Teile | | |
| B15 | | 0,8 Teile | |
| B16 | | | 0,8 Teile |

(fortgesetzt)

| Bestandteil | Formulierung mit Benchmark | Formulierung mit B15 | Formulierung mit B16 |
|---|---|---|---|
| Wasser | 65,7 Teile | 65,7 Teile | 65,7 Teile |
| Essigsäure, conc. | 0,5 Teile | 0,5 Teile | 0,5 Teile |

Tabelle 23: Autopflegeformulierungen (Gehaltsangaben in Gewichtsteilen)

| Bestandteil | Formulierung mit Benchmark | Formulierung mit B15 | Formulierung mit B16 |
|---|---|---|---|
| REWOQUAT® CR 3099 | 10 Teile | 10 Teile | 10 Teile |
| REWOPAL® MPG 40 | 8,6 Teile | 8,6 Teile | 8,6 Teile |
| Butyl Cellosolve | 6,2 Teile | 6,2 Teile | 6,2 Teile |
| REWOCARE® OT | 8 Teile | 8 Teile | 8 Teile |
| D5 | 3 Teile | 3 Teile | 3 Teile |
| TEGOPREN® 6922 | 0,8 Teile | | |
| B15 | | 0,8 Teile | |
| B16 | | | 0,8 Teile |
| Wasser | 62,9 Teile | 62,9 Teile | 62,9 Teile |
| Essigsäure, conc. | 0,5 Teile | 0,5 Teile | 0,5 Teile |

Diese Autopflegeformulierungen wurden hinsichtlich ihrer Wasserverdünnbarkeit getestet, indem 1 Teil Autopflegeformulierung (siehe Tabelle 22 und 23) mit 26 Teilen Wasser verdünnt wurde. Dabei dürfen keine Trübungen erkennbar sein.

Tabelle 24: Basis-Formulierungen für Weichspüler (ohne Parfum, Farbe und andere Additive) (Gehaltsangaben in Gewichtsteilen)

| Bestandteil | Formulierung mit Benchmark | Formulierung mit B3 |
|---|---|---|
| SQ1 | 0,15 | |
| B3 | | 0,15 Teile |
| REWOQUAT® WE 18 | 5,65 Teile | 5,65 Teile |
| Wasser | 94,2 Teile | 94,2 Teile |

**Patentansprüche**

**1.** Siloxan (A) gemäß der Formel (I),

$$M^1_{a1}M^2_{a2}M^3_{a3}M^4_{a4}D^1_{b1}D^2_{b2}D^3_{b3}T^1_{c1}T^4_{c4}Q_d \qquad \text{Formel (I),}$$

mit

$M^1 = [R^1_3SiO_{1/2}]$;
$M^2 = [R^2R^1_2SiO_{1/2}]$;
$M^3 = [R^3R^1_2SiO_{1/2}]$;
$M^4 = [R^4R^1_2SiO_{1/2}]$;
$D^1 = [R^1_2SiO_{2/2}]$;
$D^2 = [R^1R^2SiO_{2/2}]$;
$D^3 = [R^1R^3SiO_{2/2}]$;
$T^1 = [R^1SiO_{3/2}]$;

$T^4 = [R^4SiO_{3/2}]$;
Q - $[SiO_{4/2}]$;
a1 = 0 bis 32, bevorzugt 0 bis 19, insbesondere 0 bis 12;
a2 = 0 bis 32, bevorzugt 1 bis 10, insbesondere 1 bis 3;
a3 = 0 bis 32, bevorzugt 1 bis 10, insbesondere 1 bis 2;
a4 = 0 bis 6, bevorzugt 0 bis 1, insbesondere 0;
b1 = 1 bis 1000, bevorzugt 5 bis 500, insbesondere 10 bis 400;
b2 = 0 bis 10, bevorzugt 0 bis 5, insbesondere 0;
b3 = 0 bis 10, bevorzugt 0 bis 5, insbesondere 0;
c1 = 0 bis 10, bevorzugt 0 bis 5, insbesondere 0 bis 4;
c4 = 0 bis 5, bevorzugt 0 bis 2, insbesondere 0;
d = 0 bis 10, bevorzugt 0 bis 5, insbesondere 0 bis 4;
$R^1$ = jeweils unabhängig voneinander gleiche oder verschiedene Kohlenwasserstoffreste, vorzugsweise mit 1 bis 30 Kohlenstoffatomen, weiter bevorzugt Alkylreste mit 1 bis 30 Kohlenstoffatomen oder aromatische Kohlenwasserstoffreste mit 6 bis 30 Kohlenstoffatomen, noch weiter bevorzugt Alkylreste mit 1 bis 14 Kohlenstoffatomen oder monocylische aromatische Kohlenwasserstoffreste, wobei die Alkylreste vorzugsweise linear oder verzweigt, gesättigt oder ungesättigt sind, noch weiter bevorzugt Methyl, Ethyl, Propyl oder Phenyl, insbesondere Methyl;
$R^2 = R^{21}-R^{22}$;
$R^{21}$ = jeweils unabhängig voneinander gleiche oder verschiedene zweiwertige Kohlenwasserstoffreste mit mindestens einer Hydroxylgruppe und gegebenenfalls weiteren Sauerstoffatomen und vorzugsweise 2 bis 30 Kohlenstoffatomen, weiter bevorzugt zusätzlich enthaltend 1 bis 2 weitere Sauerstoffatome, noch weiter bevorzugt enthaltend funktionelle Gruppen ausgewählt aus Ether-, Carbonyl- und Estergruppen, noch weiter bevorzugt jeweils unabhängig voneinander gleiche oder verschiedene zweiwertige Reste ausgewählt aus der Gruppe bestehend aus

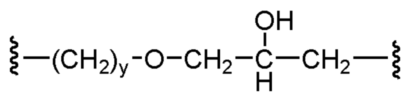 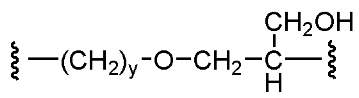

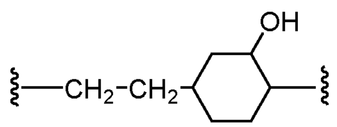 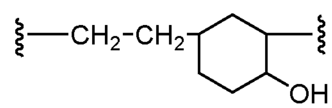

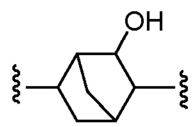 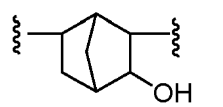

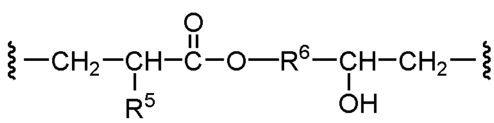 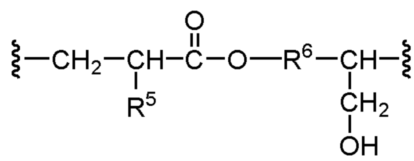 ,

insbesondere jeweils unabhängig voneinander gleiche oder verschiedene zweiwertige Reste ausgewählt aus der Gruppe bestehend aus

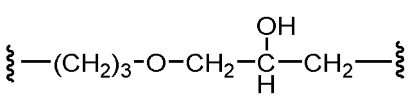 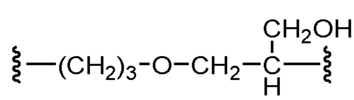 ;

$R^{22}$ = jeweils unabhängig voneinandergleiche oder verschiedene Reste der Formel (II),

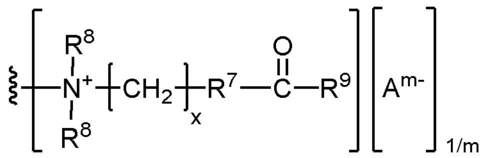 Formel (II);

$R^3 = R^{31}-R^{32}$;
$R^{31} = R^{21}$;
$R^{32}$ = jeweils unabhängig voneinander gleiche oder verschiedene Reste der Formel (III),

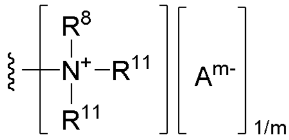 Formel (III)

$R^4$ = jeweils unabhängig voneinander gleiche oder verschiedene Alkoxygruppen oder Acyloxygruppen, vorzugsweise mit 1 bis 6 Kohlenstoffatomen, weiter bevorzugt Acetoxygruppen und/oder Methoxy-, Ethoxy-, n-Propoxy-, iso-Propoxy-, n-Butoxy-, tert-Butoxy-Gruppen und/oder Alkoxygruppen, abgeleitet von Glykolresten, beispielsweise Propylenglykol, Dipropylenglykol, Tripropylenglykol, Hexylenglykol, Pentylenglykol, Butyldiglykol, insbesondere iso-Propoxy-Gruppen;
$R^5$ = jeweils unabhängig voneinander gleiche oder verschiedene Reste ausgewählt aus der Gruppe bestehend aus Wasserstoff und Kohlenwasserstoffresten, vorzugsweise mit 1 bis 6 Kohlenstoffatomen, weiter bevorzugt ausgewählt aus der Gruppe bestehend aus Alkylresten mit 1 bis 6 Kohlenstoffatomen, wobei die Alkylreste vorzugsweise linear oder verzweigt, gesättigt oder ungesättigt sind, insbesondere Methyl;
$R^6$ = jeweils unabhängig voneinander gleiche oder verschiedene zweiwertige Kohlenwasserstoffreste, die optional Ethergruppen enthalten, vorzugsweise mit 1 bis 6 Kohlenstoffatomen, weiter bevorzugt Methylen;
$R^7$ = jeweils unabhängig voneinander gleiche oder verschiedene zweiwertige Reste ausgewählt aus der Gruppe bestehend aus -O- und $-NR^{10}-$, vorzugsweise $-NR^{10}-$;
$R^8$ = jeweils unabhängig voneinander gleiche oder verschiedene Reste ausgewählt aus der Gruppe bestehend aus Kohlenwasserstoffresten, vorzugsweise mit 1 bis 30 Kohlenstoffatomen, weiter bevorzugt ausgewählt aus der Gruppe bestehend aus linearen oder verzweigten, gesättigten oder ungesättigten Alkylresten mit 1 bis 12 Kohlenstoffatomen, noch weiter bevorzugt jeweils unabhängig voneinander gleiche oder verschiedene Reste ausgewählt aus der Gruppe bestehend aus Methyl, Ethyl, n-Propyl, iso-Propyl, n-Butyl, iso-Butyl, tert.-Butyl, noch weiter bevorzugt jeweils unabhängig voneinander gleiche oder verschiedene Reste ausgewählt aus der Gruppe bestehend aus Methyl, Ethyl, n-Propyl, iso-Propyl, insbesondere Methyl;
$R^9$ =jeweils unabhängig voneinander gleiche oder verschiedene Reste ausgewählt aus der Gruppe bestehend aus Wasserstoff und Kohlenwasserstoffresten, vorzugsweise mit 1 bis 30 Kohlenstoffatomen, weiter bevorzugt ausgewählt aus der Gruppe bestehend aus Alkylresten mit 1 bis 30 Kohlenstoffatomen, noch weiter bevorzugt Alkylresten mit 12 bis 24 Kohlenstoffatomen, insbesondere mit 16 bis 22 Kohlenstoffatomen, wobei die Kohlenwasserstoffreste beziehungsweise Alkylreste vorzugsweise linear oder verzweigt, substituiert oder unsubstituiert gesättigt oder ungesättigt, besonders bevorzugt linear, unsubstituiert und gesättigt sind;
$R^{10}$ = jeweils unabhängig voneinander gleiche oder verschiedene Reste aus der Gruppe bestehend aus Wasserstoff, $-C(=O)R^9$ und Kohlenwasserstoffresten, vorzugsweise mit 1 bis 6 Kohlenstoffatomen, weiter bevorzugt Alkylreste mit 1 bis 6 Kohlenstoffatomen, wobei die Kohlenwasserstoffreste beziehungsweise Alkylreste vorzugsweise linear oder verzweigt, substituiert oder unsubstituiert gesättigt oder ungesättigt, besonders bevorzugt linear, unsubstituiert und gesättigt sind, insbesondere bevorzugt ist $R^{10}$ Wasserstoff;
$R^{11}$ = jeweils unabhängig voneinander gleiche oder verschiedene Reste ausgewählt aus der Gruppe bestehend aus Kohlenwasserstoffresten, die mindestens eine Hydroxygruppe und vorzugsweise 1 bis 6 Kohlenstoffatome aufweisen, bevorzugt Alkylreste, die mindestens eine Hydroxygruppe und vorzugsweise 1 bis 6 Kohlenstoffatome aufweisen, wobei die Alkylreste vorzugsweise linear oder verzweigt, gesättigt oder ungesättigt sind, und Resten der Formel (IV)

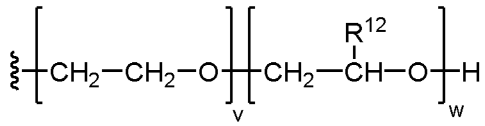 Formel (IV),

bevorzugt 2-Hydroxyethyl und/oder 2-Hydroxypropyl;
$R^{12}$ = jeweils unabhängig voneinander gleiche oder verschiedene Reste ausgewählt aus der Gruppe bestehend aus Kohlenwasserstoffresten, vorzugsweise mit 1 bis 6 Kohlenstoffatomen, weiter bevorzugt Alkylreste mit 1 bis 6 Kohlenstoffatomen, wobei die Alkylreste vorzugsweise linear oder verzweigt, gesättigt oder ungesättigt sind, vorzugsweise Methyl und Ethyl, insbesondere Methyl;
$A^{m-}$ = jeweils unabhängig voneinander gleiche oder verschiedene Anionen, ausgewählt aus anorganischen oder organischen Anionen der Säuren $H_mA$, sowie deren Derivate;
m = 1 bis 3, bevorzugt 1 bis 2, insbesondere 1;
v = 0 bis 30, bevorzugt 0 bis 10, insbesondere 1 bis 3;
w = 0 bis 30, bevorzugt 0 bis 10;
x = 2 bis 18, bevorzugt 3;
y = 2 bis 18, bevorzugt 3;

**dadurch gekennzeichnet, dass** die Maßgaben (i) und (ii) gelten:

(i)

$$a2 + b2 \geq 1;$$

(ii)

$$a3 + b3 \geq 1;$$

2. Siloxan (A) nach Anspruch 1, **dadurch gekennzeichnet, dass** zudem entweder die Maßgabe (iii) oder die Maßgabe (iv) gilt:

(iii)

$$a1 = a4 = b2 = b3 = c1 = c4 = d = 0$$

und

$$a2 = a3 = 1;$$

(iv)

$$b2 = b3 = 0$$

und

$$c1 + c4 + d \geq 1$$

und
$a2 + a3 + a4 \geq 3$, vorzugsweise $a2 \geq 2$, $a3 \geq 1$ und $a4 = 0$;

3. Zusammensetzung, **dadurch gekennzeichnet, dass** sie mindestens ein Siloxan (A) gemäß mindestens einem der

Ansprüche 1 und 2 umfasst.

4. Zusammensetzung nach Anspruch 3, **dadurch gekennzeichnet, dass** die Zusammensetzung zusätzlich mindestens ein Siloxan ausgewählt aus der Gruppe bestehend aus Siloxanen (B) und Siloxanen (C) umfasst, wobei gilt:

Siloxan (B) ist ein Siloxan, das sich zumindest darin, vorzugsweise genau darin, von einem Siloxan gemäß Anspruch 1 unterscheidet, dass die Maßgaben (v) und (vi) anstelle der Maßgaben (i) bis (iv) gelten:

(v)

$$a2 = b2 = 0,$$

(vi)

$$a3 + b3 \geq 2;$$

Siloxan (C) ist ein Siloxan, das sich zumindest darin, vorzugsweise genau darin, von einem Siloxan gemäß Anspruch 1 unterscheidet, dass die Maßgaben (vii) und (viii) anstelle der Maßgaben (i) und (iv) gelten:

(vii)

$$a3 = b3 = 0 ,$$

(viii)

$$a2 + b2 \geq 2.$$

5. Zusammensetzung nach mindestens einem der Ansprüche 3 und 4, **dadurch gekennzeichnet, dass**

a. der Massenanteil des mindestens einen Siloxans (A) bezogen auf die Gesamtmasse der Siloxane von 20% bis 70%, bevorzugt von 25% bis 60%, insbesondere von 30% bis 50% beträgt;
und/oder
b. der Massenanteil des mindestens einen Siloxans (B) bezogen auf die Gesamtmasse der Siloxane von 0% bis 15%, bevorzugt von 1% bis 10% beträgt;
und/oder
c. der Massenanteil des mindestens einen Siloxans (C) bezogen auf die Gesamtmasse der Siloxane von 3% bis 80%, bevorzugt von 5% bis 60%, insbesondere von 10% bis 50% beträgt.

6. Zusammensetzung nach mindestens einem der Ansprüche 3 bis 5, **dadurch gekennzeichnet, dass** sie Amidamine enthält, wobei der Massenanteil von Amidaminen bezogen auf die Gesamtmasse der Siloxane weniger als 1%, bevorzugt weniger als 0,8%, weiter bevorzugt weniger als 0,6%, insbesondere weniger als 0,4% beträgt, oder keine Amidamine enthält.

7. Verfahren umfassend mindestens einen Verfahrensschritt, bei dem mindestens ein epoxyfunktionelles Siloxan, das mindestens zwei Epoxidgruppen aufweist, sowohl mit mindestens einem tertiären Amin, ausgewählt aus der Gruppe bestehend aus Amidaminen und Esteraminen, vorzugsweise Amidaminen, als auch mit mindestens einem tertiären Amin, ausgewählt aus der Gruppe bestehend aus Dialkanolaminen, unter Bildung von quartären Ammoniumgruppen umgesetzt wird.

8. Verfahren nach Anspruch 7 zur Herstellung eines Siloxans (A) gemäß mindestens einem der Ansprüche 1 bis 2 oder einer Zusammensetzung gemäß mindestens einem der Ansprüche 3 bis 6.

9. Verfahren nach mindestens einem der Ansprüche 7 und 8, **dadurch gekennzeichnet, dass** das epoxyfunktionelle Siloxan hergestellt wird durch Hydrosilylierung mindestens eines olefinisch ungesättigten Epoxids, vorzugsweise

ausgewählt aus der Gruppe bestehend aus Allylglycidylether, Vinylcyclohexenmonoxid und Norbornadienmonoepoxid, insbesondere Allylglycidylether, mit mindestens einem SiH-funktionellen Siloxan der Formel (V),

$$M^1_{a1}M^5_{a5}D^1_{b1}D^5_{b5}T^1_{c1}T^4_{c4}Q_d \qquad (V),$$

mit

$M^5 = [R^1_2SiHO_{1/2}]$,
$D^5 = [R^1SiHO_{2/2}]$,
a5 = 0 bis 32, bevorzugt 1 bis 10, besonders bevorzugt 2 bis 3, insbesondere 2;
b5 = 0 bis 10, bevorzugt 0 bis 5, insbesondere 0;

wobei
$M^1$, $D^1$, $T^1$, $T^4$, Q, a1, b1, c1, c4, d und $R^1$ wie in Formel (I) definiert sind.

**10.** Verfahren nach mindestens einem der Ansprüche 7 bis 9, **dadurch gekennzeichnet, dass** das epoxyfunktionelle Siloxan ein Siloxan der Formel (VI) ist,

$$M^1_{a1}M^6_{a5}D^1_{b1}D^6_{b5}\ T^1_{c1}T^4_{c4}Q_d \qquad (VI),$$

mit

$M^6 = [R^{13}R^1_2SiO_{1/2}]$,
$D^6 = [R^{13}R^1SiO_{2/2}]$,
$R^{13}$ = jeweils unabhängig voneinander gleiche oder verschiedene organische Epoxy-Reste, vorzugsweise ausgewählt aus der Gruppe bestehend aus

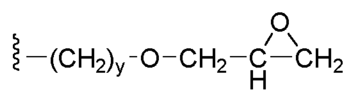

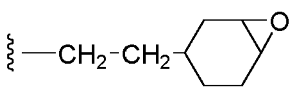

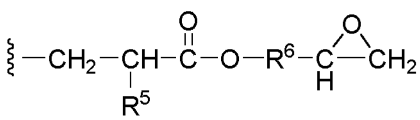

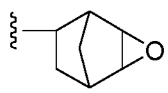

,

insbesondere

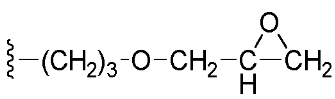

,

wobei
$M^1$, $D^1$, $T^1$, $T^4$, Q, a1, a5, b1, b5, c1, c4, d, $R^1$, $R^5$, $R^6$ und y wie in Formel (I) definiert sind.

**11.** Verfahren nach mindestens einem der Ansprüche 7 bis 10, **dadurch gekennzeichnet, dass** der Restgehalt von tertiären Aminen, ausgewählt aus der Gruppe bestehend aus Amidaminen und Esteraminen, vorzugsweise Amidaminen, nach der Umsetzung als Massenanteil bezogen auf die Gesamtmasse der Zusammensetzung weniger als 1%, bevorzugt weniger als 0,8%, weiter bevorzugt weniger als 0,6%, insbesondere weniger als 0,4% beträgt.

**12.** Verfahren nach mindestens einem der Ansprüche 7 bis 11, **dadurch gekennzeichnet, dass** das tertiäre Amin, ausgewählt aus der Gruppe bestehend aus Amidaminen und Esteraminen, ein tertiäres Amin gemäß der Formel (VII) ist,

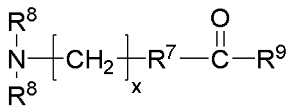

Formel (VII),

wobei
$R^8$, $R^7$, $R^9$ und x wie in Formel (II) definiert sind.

**13.** Verfahren nach mindestens einem der Ansprüche 7 bis 12, **dadurch gekennzeichnet, dass** das tertiäre Amin, ausgewählt aus der Gruppe bestehend aus Dialkanolaminen, ein tertiäres Amin gemäß der Formel (VIII) ist,

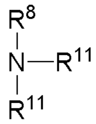

Formel (VIII)

wobei $R^8$ und $R^{11}$ wie in Formel (III) definiert sind.

**14.** Zusammensetzung, erhältlich durch ein Verfahren gemäß mindestens einem der Ansprüche 7 bis 13.

**15.** Zusammensetzung, vorzugweise eine wässrige Emulsion, **dadurch gekennzeichnet, dass** die Zusammensetzung Wasser sowie mindestens ein Siloxan (A) gemäß mindestens einem der Ansprüche 1 und 2 oder eine Zusammensetzung gemäß mindestens einem der Ansprüche 3 bis 6 und/oder Anspruch 14 und/oder ein Verfahrensprodukt eines Verfahrens gemäß mindestens einem der Ansprüche 7 bis 13 umfasst,
vorzugsweise umfassend, angeben in Massenanteilen bezogen auf die Gesamtmasse der Zusammensetzung, die folgenden Komponenten:

a) 20% bis 99,5%, bevorzugt 40 bis 97%, insbesondere 60 bis 95% Wasser;
b) 0,5% bis 80%, bevorzugt 3% bis 60%, insbesondere 5% bis 40% mindestens eines Siloxans, umfassend mindestens ein Siloxan (A) und vorzugsweise mindestens ein Siloxan (B) und/oder mindestens ein Siloxan (C);
c) vorzugsweise 1% bis 10% mindestens eines Emulgators;
d) vorzugsweise 5% bis 20% mindestens eines Glykols; und
e) vorzugsweise 0% bis 1% Essigsäure.

**16.** Verwendung eines Siloxans (A) gemäß mindestens einem der Ansprüche 1 und 2 und/oder einer Zusammensetzung gemäß mindestens einem der Ansprüche 3 bis 6 und/oder mindestens einem der Ansprüche 14 bis 15 und/oder eines Verfahrensprodukts eines Verfahren gemäß mindestens einem der Ansprüche 7 bis 13

a) zur Behandlung, vorzugsweise Ausrüstung und/oder Imprägnierung, von Flächengebilden;
b) in Reinigungs- und Pflegeformulierungen für den Haushalt und für industrielle Anwendungen, insbesondere in Weichspülmitteln,
c) in kosmetischen, pharmazeutischen und dermatologischen Zusammensetzungen, insbesondere in kosmetischen Reinigungs- und Pflegeformulierungen, Haarbehandlungsmitteln und Haarnachbehandlungsmitteln; und/oder
d) zur Reinigung und Pflege von harten Oberflächen, vorzugsweise zur Reinigung und Pflege von Fahrzeugen, insbesondere als Additiv in Trocknungshilfen für Autowaschstraßen.

## Claims

**1.** Siloxane (A) of the formula (I)

$$M^1_{a1}M^2_{a2}M^3_{a3}M^4_{a4}D^1_{b1}D^2_{b2}D^3_{b3}T^1_{c1}T^4_{c4}Q_d \qquad \text{Formula (I)}$$

with

$M^1 = [R^1_3SiO_{1/2}]$;

$M^2 = [R^2R^1{}_2SiO_{1/2}]$;
$M^3 = [R^3R^1{}_2SiO_{1/2}]$;
$M^4 = [R^4R^1{}_2SiO_{1/2}]$;
$D^1 = [R^1{}_2SiO_{2/2}]$;
$D^2 = [R^1R^2SiO_{2/2}]$;
$D^3 = [R^1R^3SiO_{2/2}]$;
$T^1 = [R^1SiO_{3/2}]$;
$T^4 = [R^4SiO_{3/2}]$;
$Q = [SiO_{4/2}]$;
a1 = 0 to 32, preferably 0 to 19, especially 0 to 12;
a2 = 0 to 32, preferably 1 to 10, especially 1 to 3;
a3 = 0 to 32, preferably 1 to 10, especially 1 to 2;
a4 = 0 to 6, preferably 0 to 1, especially 0;
b1 = 1 to 1000, preferably 5 to 500, especially 10 to 400;
b2 = 0 to 10, preferably 0 to 5, especially 0;
b3 = 0 to 10, preferably 0 to 5, especially 0;
c1 = 0 to 10, preferably 0 to 5, especially 0 to 4;
c4 = 0 to 5, preferably 0 to 2, especially 0;
d = 0 to 10, preferably 0 to 5, especially 0 to 4;
$R^1$ = each independently identical or different hydrocarbon radicals, preferably having 1 to 30 carbon atoms, further preferably alkyl radicals having 1 to 30 carbon atoms or aromatic hydrocarbon radicals having 6 to 30 carbon atoms, even further preferably alkyl radicals having 1 to 14 carbon atoms or monocyclic aromatic hydrocarbon radicals, where the alkyl radicals are preferably linear or branched, saturated or unsaturated, even further preferably methyl, ethyl, propyl or phenyl, especially methyl; $R^2 = R^{21}-R^{22}$;
$R^{21}$ = each independently identical or different divalent hydrocarbon radicals having at least one hydroxyl group and optionally further oxygen atoms and preferably 2 to 30 carbon atoms, further preferably additionally containing 1 to 2 further oxygen atoms, even further preferably containing functional groups selected from ether, carbonyl and ester groups, even further preferably each independently identical or different divalent radicals selected from the group consisting of

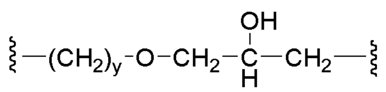
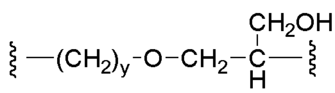

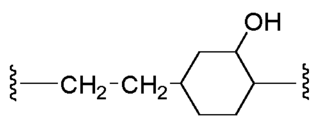
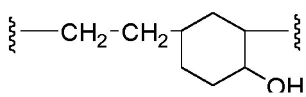

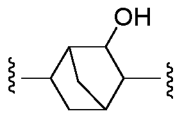
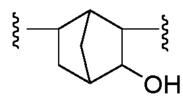

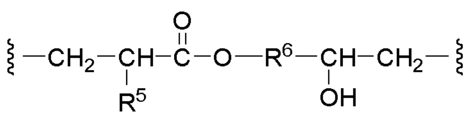
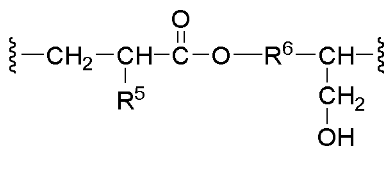
,

especially each independently identical or different divalent radicals selected from the group consisting of

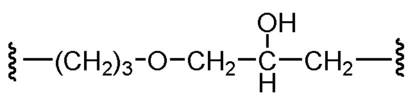
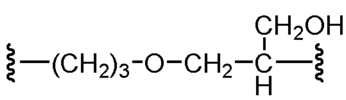
;

$R^{22}$ = each independently identical or different radicals of the formula (II),

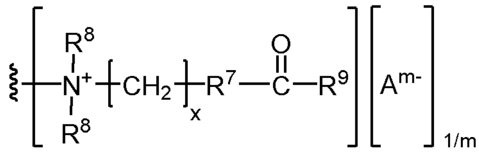

Formula (II);

$R^3 = R^{31}\text{-}R^{32}$;
$R^{31} = R^{21}$;
$R^{32}$ = each independently identical or different radicals of the formula (III)

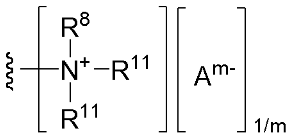

Formula (III)

$R^4$ = each independently identical or different alkoxy groups or acyloxy groups, preferably having 1 to 6 carbon atoms, further preferably acetoxy groups and/or methoxy groups, ethoxy groups, n-propoxy groups, isopropoxy groups, n-butoxy groups, tert-butoxy groups and/or alkoxy groups derived from glycol radicals, for example propylene glycol, dipropylene glycol, tripropylene glycol, hexylene glycol, pentylene glycol, butyldiglycol, especially isopropoxy groups;
$R^5$ = each independently identical or different radicals selected from the group consisting of hydrogen and hydrocarbon radicals, preferably having 1 to 6 carbon atoms, further preferably selected from the group consisting of alkyl radicals having 1 to 6 carbon atoms, where the alkyl radicals are preferably linear or branched, saturated or unsaturated, especially methyl;
$R^6$ = each independently identical or different divalent hydrocarbon radicals optionally containing ether groups, preferably having 1 to 6 carbon atoms, further preferably methylene;
$R^7$ = each independently identical or different divalent radicals selected from the group consisting of -O- and $-NR^{10}-$, preferably $-NR^{10}-$;
$R^8$ = each independently identical or different radicals selected from the group consisting of hydrocarbon radicals, preferably having 1 to 30 carbon atoms, further preferably selected from the group consisting of linear or branched, saturated or unsaturated alkyl radicals having 1 to 12 carbon atoms, even further preferably each independently identical or different radicals selected from the group consisting of methyl, ethyl, n-propyl, isopropyl, n-butyl, isobutyl, tert-butyl, even further preferably each independently identical or different radicals selected from the group consisting of methyl, ethyl, n-propyl, isopropyl, especially methyl;
$R^9$ = each independently identical or different radicals selected from the group consisting of hydrogen and hydrocarbon radicals, preferably having 1 to 30 carbon atoms, further preferably selected from the group consisting of alkyl radicals having 1 to 30 carbon atoms, even further preferably alkyl radicals having 12 to 24 carbon atoms, especially having 16 to 22 carbon atoms, where the hydrocarbon radicals or alkyl radicals are preferably linear or branched, substituted or unsubstituted, saturated or unsaturated, more preferably linear, unsubstituted and saturated;
$R^{10}$ = each independently identical or different radicals selected from the group consisting of hydrogen, $-C(=O)R^9$ and hydrocarbon radicals, preferably having 1 to 6 carbon atoms, further preferably alkyl radicals having 1 to 6 carbon atoms, where the hydrocarbon radicals or alkyl radicals are preferably linear or branched, substituted or unsubstituted, saturated or unsaturated, more preferably linear, unsubstituted and saturated; $R^{10}$ is especially preferably hydrogen;
$R^{11}$ = each independently identical or different radicals selected from the group consisting of hydrocarbon radicals having at least one hydroxyl group and preferably 1 to 6 carbon atoms, preferably alkyl radicals having at least one hydroxyl group and preferably 1 to 6 carbon atoms, where the alkyl radicals are preferably linear or branched, saturated or unsaturated, and radicals of the formula (IV)

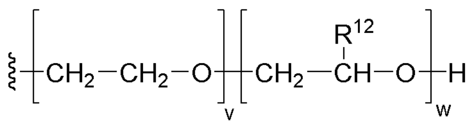

Formula (IV),

preferably 2-hydroxyethyl and/or 2-hydroxypropyl;
$R^{12}$ = each independently identical or different radicals selected from the group consisting of hydrocarbon radicals, preferably having 1 to 6 carbon atoms, further preferably alkyl radicals having 1 to 6 carbon atoms, where the alkyl radicals are preferably linear or branched, saturated or unsaturated, preferably methyl and ethyl, especially methyl;
$A^{m-}$ = each independently identical or different anions selected from inorganic or organic anions of the acids $H_mA$, and derivatives thereof,
m = 1 to 3, preferably 1 to 2, especially 1;
v = 0 to 30, preferably 0 to 10, especially 1 to 3;
w = 0 to 30, preferably 0 to 10;
x = 2 to 18, preferably 3;
y = 2 to 18, preferably 3;

**characterized in that** conditions (i) and (ii) are applicable:

(i)

$$a2 + b2 \geq 1;$$

(ii)

$$a3 + b3 \geq 1.$$

2. Siloxane (A) according to Claim 1, **characterized in that**, in addition, either condition (iii) or condition (iv) is applicable:

(iii)

$$a1 = a4 = b2 = b3 = c1 = c4 = d = 0$$

and

$$a2 = a3 = 1;$$

(iv)

$$b2 = b3 = 0$$

and

$$c1 + c4 + d \geq 1$$

and
$a2 + a3 + a4 \geq 3$, preferably $a2 \geq 2$, $a3 \geq 1$ and $a4 = 0$.

3. Composition, **characterized in that** it comprises at least one siloxane (A) according to at least one of Claims 1 and 2.

4. Composition according to Claim 3, **characterized in that** the composition additionally comprises at least one siloxane selected from the group consisting of siloxanes (B) and siloxanes (C), where:

siloxane (B) is a siloxane that differs from a siloxane according to Claim 1 at least **in that**, preferably precisely **in that**, conditions (v) and (vi) are applicable rather than conditions (i) to (iv):

(v)

$$a2 = b2 = 0,$$

(vi)

$$a3 + b3 \geq 2;$$

siloxane (C) is a siloxane that differs from a siloxane according to Claim 1 at least **in that**, preferably precisely **in that**, conditions (vii) and (viii) are applicable rather than conditions (i) and (iv) :

(vii)

$$a3 = b3 = 0,$$

(viii)

$$a2 + b2 \geq 2.$$

**5.** Composition according to at least one of Claims 3 and 4, **characterized in that**

a. the proportion by mass of the at least one siloxane (A) based on the total mass of the siloxanes is from 20% to 70%, preferably from 25% to 60%, especially from 30% to 50%;
and/or
b. the proportion by mass of the at least one siloxane (B) based on the total mass of the siloxanes is from 0% to 15%, preferably from 1% to 10%;
and/or
c. the proportion by mass of the at least one siloxane (C) based on the total mass of the siloxanes is from 3% to 80%, preferably from 5% to 60%, especially from 10% to 50%.

**6.** Composition according to at least one of Claims 3 to 5, **characterized in that** it contains amide amines, where the proportion by mass of amide amines based on the total mass of the siloxanes is less than 1%, preferably less than 0.8%, further preferably less than 0.6%, especially less than 0.4%, or it does not contain any amide amines.

**7.** Process including at least one process step in which at least one epoxy-functional siloxane having at least two epoxy groups is reacted both with at least one tertiary amine selected from the group consisting of amide amines and ester amines, preferably amide amines, and with at least one tertiary amine selected from the group consisting of dialkanolamines to form quaternary ammonium groups.

**8.** Process according to Claim 7 for preparation of a siloxane (A) according to at least one of Claims 1 and 2 or of a composition according to at least one of Claims 3 to 6.

**9.** Process according to at least one of Claims 7 and 8, **characterized in that** the epoxy-functional siloxane is prepared by hydrosilylation of at least one olefinically unsaturated epoxide, preferably selected from the group consisting of allyl glycidyl ether, vinylcyclohexene monoxide and norbornadiene monoepoxide, especially allyl glycidyl ether, with at least one SiH-functional siloxane of the formula (V)

$$M^1_{a1}M^5_{a5}D^1_{b1}D^5_{b5}T^1_{c1}T^4_{c4}Q_d \qquad (V)$$

with

$M^5 = [R^1_2SiHO_{1/2}]$,
$D^5 = [R^1SiHO_{2/2}]$,
a5 = 0 to 32, preferably 1 to 10, more preferably 2 to 3, especially 2;

b5 = 0 to 10, preferably 0 to 5, especially 0;

where
$M^1$, $D^1$, $T^1$, $T^4$, Q, a1, b1, c1, c4, d and $R^1$ are as defined in formula (I).

**10.** Process according to at least one of Claims 7 to 9, **characterized in that** the epoxy-functional siloxane is a siloxane of the formula (VI)

$$M^1_{a1}M^6_{a5}D^1_{b1}D^6_{b5}\ T^1_{c1}T^4_{c4}Q_d \qquad (VI)$$

with

$M^6 = [R^{13}R^1_2SiO_{1/2}]$,
$D^6 = [R^{13}R^1SiO_{2/2}]$,
$R^{13}$ = each independently identical or different organic epoxy radicals, preferably selected from the group consisting of

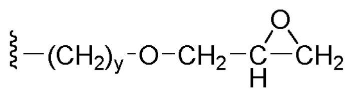

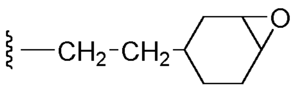

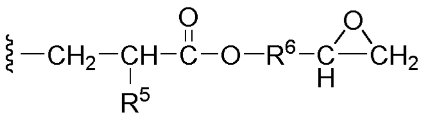

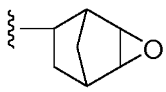

,

especially

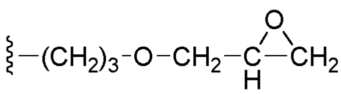

,

where
$M^1$, $D^1$, $T^1$, $T^4$, Q, a1, a5, b1, b5, c1, c4, d, $R^1$, $R^5$, $R^6$ and y are as defined in formula (I).

**11.** Process according to at least one of Claims 7 to 10, **characterized in that** the residual content of tertiary amines selected from the group consisting of amide amines and ester amines, preferably amide amines, after the reaction, as a proportion by mass based on the total mass of the composition, is less than 1%, preferably less than 0.8%, further preferably less than 0.6%, especially less than 0.4%.

**12.** Process according to at least one of Claims 7 to 11, **characterized in that** the tertiary amine selected from the group consisting of amide amines and ester amines is a tertiary amine of the formula (VII)

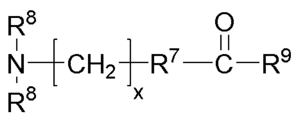

Formula (VII)

where
$R^8$, $R^7$, $R^9$ and x are as defined in formula (II) .

**13.** Process according to at least one of Claims 7 to 12, **characterized in that** the tertiary amine selected from the group consisting of dialkanolamines is a tertiary amine of the formula (VIII)

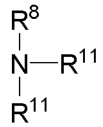

Formula (VIII)

where $R^8$ and $R^{11}$ are as defined in formula (III).

**14.** Composition obtainable by a process according to at least one of Claims 7 to 13.

**15.** Composition, preferably an aqueous emulsion, **characterized in that** the composition comprises water and at least one siloxane (A) according to at least one of Claims 1 and 2 or a composition according to at least one of Claims 3 to 6 and/or Claim 14 and/or a process product from a process according to at least one of Claims 7 to 13, preferably comprising, reported in parts by mass based on the total mass of the composition, the following components:

a) 20% to 99.5%, preferably 40% to 97%, especially 60% to 95%, water;
b) 0.5% to 80%, preferably 3% to 60%, especially 5% to 40%, of at least one siloxane comprising at least one siloxane (A) and preferably at least one siloxane (B) and/or at least one siloxane (C) ;
c) preferably 1% to 10% of at least one emulsifier;
d) preferably 5% to 20% of at least one glycol; and
e) preferably 0% to 1% acetic acid.

**16.** Use of a siloxane (A) according to at least one of Claims 1 and 2 and/or of a composition according to at least one of Claims 3 to 6 and/or at least one of Claims 14 and 15 and/or of a process product from a process according to at least one of Claims 7 to 13

a) for treatment, preferably finishing and/or impregnation, of two-dimensional structures;
b) in cleaning and care formulations for the household and for industrial purposes, especially in fabric softeners;
c) in cosmetic, pharmaceutical and dermatological compositions, especially in cosmetic cleansing and care formulations, hair treatment products and hair aftertreatment products; and/or
d) for cleaning and care of hard surfaces, preferably for cleaning and care of motor vehicles, especially as additive in drying aids for carwash facilities.

## Revendications

**1.** Siloxane (A) selon la formule (I),

$$\mathbf{M^1_{a1}M^2_{a2}M^3_{a3}M^4_{a4}D^1_{b1}D^2_{b2}D^3_{b3}T^1_{c1}T^4_{c4}Q_d} \qquad \text{formule (I) ,}$$

avec

$M^1 = [R^1_3SiO_{1/2}]$ ;
$M^2 = [R^2R^1_2SiO_{1/2}]$ ;
$M^3 = [R^3R^1_2SiO_{1/2}]$ ;
$M^4 = [R^4R^1_2SiO_{1/2}]$ ;
$D^1 = [R^1_2SiO_{2/2}]$ ;
$D^2 = [R^1R^2SiO_{2/2}]$ ;
$D^3 = [R^1R^3SiO_{2/2}]$ ;
$T^1 = [R^1SiO_{3/2}]$ ;
$T^4 = [R^4SiO_{3/2}]$ ;
$Q = [SiO_{4/2}]$ ;
a1 = 0 à 32, préférablement 0 à 19, en particulier 0 à 12 ;
a2 = 0 à 32, préférablement 1 à 10, en particulier 1 à 3 ;
a3 = 0 à 32, préférablement 1 à 10, en particulier 1 à 2 ;
a4 = 0 à 6, préférablement 0 à 1 , en particulier 0 ;
b1 = 1 à 1000, préférablement 5 à 500, en particulier 10 à 400 ;

b2 = 0 à 10, préférablement 0 à 5, en particulier 0 ;
b3 = 0 à 10, préférablement 0 à 5, en particulier 0 ;
c1 = 0 à 10, préférablement 0 à 5, en particulier 0 à 4 ;
c4 = 0 à 5, préférablement 0 à 2, en particulier 0 ;
d = 0 à 10, préférablement 0 à 5, en particulier 0 à 4 ;
$R^1$ = radicaux hydrocarbonés à chaque fois indépendamment les uns des autres identiques ou différents, de préférence comportant 1 à 30 atomes de carbone,

plus préférablement radicaux alkyle comportant 1 à 30 atomes de carbone ou radicaux hydrocarbonés aromatiques comportant 6 à 30 atomes de carbone, encore plus préférablement radicaux alkyle comportant 1 à 14 atomes de carbone ou radicaux hydrocarbonés aromatiques monocycliques,
les radicaux alkyle étant de préférence linéaires ou ramifiés, saturés ou insaturés,
encore plus préférablement méthyle, éthyle, propyle ou phényle, en particulier méthyle ;

**$R^2$ = $R^{21}$-$R^{22}$ ;**
$R^{21}$ = radicaux hydrocarbonés divalents à chaque fois indépendamment les uns des autres identiques ou différents comportant au moins un groupe hydroxyle et éventuellement d'autres atomes d'oxygène et de préférence 2 à 30 atomes de carbone, plus préférablement contenant de plus 1 à 2 atomes d'oxygène supplémentaires,

encore plus préférablement contenant des groupes fonctionnels choisis parmi des groupes éther, carbonyle et ester,
encore plus préférablement radicaux divalents à chaque fois indépendamment les uns des autres identiques ou différents choisis dans le groupe constitué par

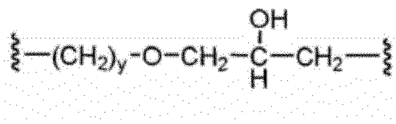
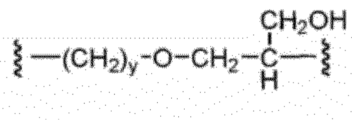

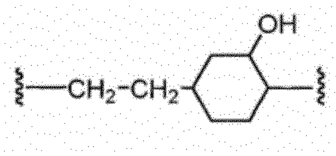
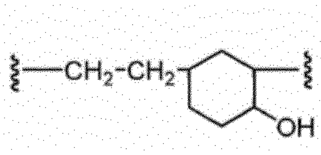

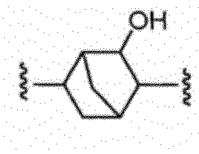
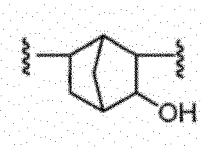

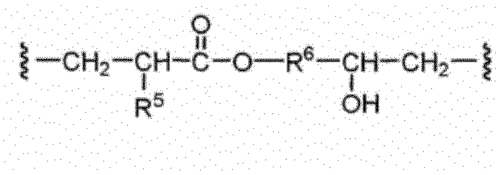
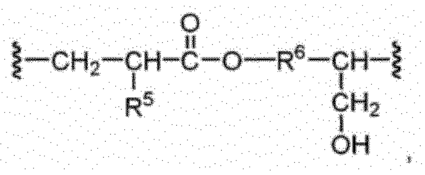
,

en particulier radicaux divalents à chaque fois indépendamment les uns des autres identiques ou différents choisis dans le groupe constitué par

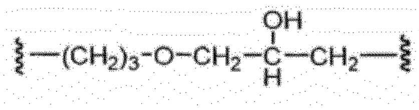
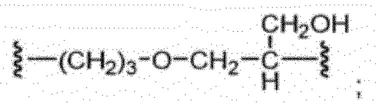
;

$R^{22}$ = radicaux à chaque fois indépendamment les uns des autres identiques ou différents de formule (II),

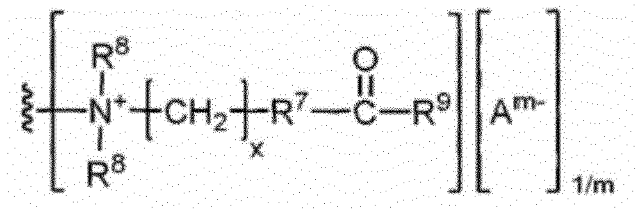 formule (II) ;

$R^3 = R^{31}\text{-}R^{32}$;

$R^{31} = R^{21}$;

$R^{32}$ = radicaux à chaque fois indépendamment les uns des autres identiques ou différents de formule (III),

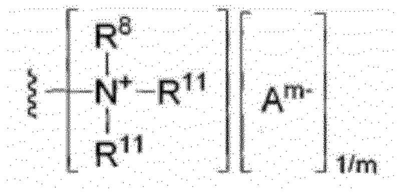 formule (III) ;

$R^4$ = groupes alcoxy ou groupes acyloxy à chaque fois indépendamment les uns des autres identiques ou différents, de préférence comportant 1 à 6 atomes de carbone,

plus préférablement groupes acétoxy et/ou groupes méthoxy, éthoxy, n-propoxy, iso-propoxy, n-butoxy, tert-butoxy, et/ou groupes alcoxy, issus de radicaux de glycol, par exemple propylèneglycol, dipropylèneglycol, tripropylèneglycol, hexylèneglycol, pentylèneglycol, butyldiglycol,
en particulier groupes iso-propoxy ;

$R^5$ = radicaux à chaque fois indépendamment les uns des autres identiques ou différents choisis dans le groupe constitué par hydrogène et radicaux hydrocarbonés, de préférence comportant 1 à 6 atomes de carbone, plus préférablement choisis dans le groupe constitué par des radicaux alkyle comportant 1 à 6 atomes de carbone, les radicaux alkyle étant de préférence linéaires ou ramifiés, saturés ou insaturés, en particulier méthyle ;
$R^6$ = radicaux hydrocarbonés à chaque fois indépendamment les uns des autres identiques ou différents, qui contiennent éventuellement des groupes éther, de préférence comportant 1 à 6 atomes de carbone, plus préférablement méthylène ;
$R^7$ = radicaux divalents à chaque fois indépendamment les uns des autres identiques ou différents choisis dans le groupe constitué par -O- et $-NR^{10}-$, de préférence $-NR^{10}-$ ;
$R^8$ = radicaux à chaque fois indépendamment les uns des autres identiques ou différents choisis dans le groupe constitué par des radicaux hydrocarbonés, de préférence comportant 1 à 30 atomes de carbone, plus préférablement choisis dans le groupe constitué par des radicaux alkyle linéaires ou ramifiés, saturés ou insaturés comportant 1 à 12 atomes de carbone, encore plus préférablement des radicaux à chaque fois indépendamment les uns des autres identiques ou différents choisis dans le groupe constitué par méthyle, éthyle, n-propyle, iso-propyle, n-butyle, iso-butyle, tert-butyle, encore plus préférablement des radicaux à chaque fois indépendamment les uns des autres identiques ou différents choisis dans le groupe constitué par méthyle, éthyle, n-propyle, iso-propyle, en particulier méthyle ;
$R^9$ = radicaux à chaque fois indépendamment les uns des autres identiques ou différents choisis dans le groupe constitué par hydrogène et des radicaux hydrocarbonés, de préférence comportant 1 à 30 atomes de carbone, plus préférablement choisis dans le groupe constitué par des radicaux alkyle comportant 1 à 30 atomes de carbone, encore plus préférablement des radicaux alkyle comportant 12 à 24 atomes de carbone, en particulier comportant 16 à 22 atomes de carbone, les radicaux hydrocarbonés, respectivement les radicaux alkyle étant de préférence linéaires ou ramifiés, substitués ou non substitués, saturés ou insaturés, particulièrement préférablement linéaires, non substitués et saturés ;
$R^{10}$ = radicaux à chaque fois indépendamment les uns des autres identiques ou différents choisis dans le groupe constitué par hydrogène, $-C(=O)R^9$ et des radicaux hydrocarbonés de préférence comportant 1 à 6

atomes de carbone, plus préférablement des radicaux alkyle comportant 1 à 6 atomes de carbone, les radicaux hydrocarbonés, respectivement les radicaux alkyle étant de préférence linéaires ou ramifiés, substitués ou non substitués, saturés ou insaturés, particulièrement préférablement linéaires, non substitués et saturés, en particulier préférablement $R^{10}$ étant hydrogène ;
$R^{11}$ = radicaux à chaque fois indépendamment les uns des autres identiques ou différents choisis dans le groupe constitué par des radicaux hydrocarbonés, qui présentent au moins un groupe hydroxy et de préférence 1 à 6 atomes de carbone, préférablement des radicaux alkyle, qui présentent au moins un groupe hydroxy et de préférence 1 à 6 atomes de carbone, les radicaux alkyle étant de préférence linéaires ou ramifiés, saturés ou insaturés, et des radicaux de formule (IV)

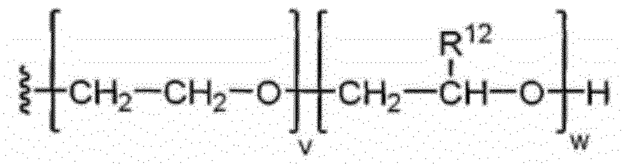
formule (IV),

préférablement 2-hydroxyéthyle et/ou 2-hydroxypropyle ;
$R^{12}$ = radicaux à chaque fois indépendamment les uns des autres identiques ou différents choisis dans le groupe constitué par des radicaux hydrocarbonés, de préférence comportant 1 à 6 atomes de carbone, plus préférablement des radicaux alkyle comportant 1 à 6 atomes de carbone, les radicaux alkyle étant de préférence linéaires ou ramifiés, saturés ou insaturés, de préférence méthyle et éthyle, en particulier méthyle ;
$A^{m-}$ = anions à chaque fois indépendamment les uns des autres identiques ou différents choisis parmi des anions inorganiques ou organiques des acides $H_mA$, ainsi que leurs dérivés ;
m = 1 à 3, préférablement 1 à 2, en particulier 1 ;
v = 0 à 30, préférablement 0 à 10, en particulier 1 à 3 ;
w = 0 à 30, préférablement 0 à 10 ;
x = 2 à 18, préférablement 3 ;
y = 2 à 18, préférablement 3 ;
**caractérisé en ce que** les mesures (i) et (ii) s'appliquent :

(i)

$$a2 + b2 \geq 1;$$

(ii)

$$a3 + b3 \geq 1;$$

.

**2.** Siloxane (A) selon la revendication 1, **caractérisé en ce qu'**en outre soit la mesure (iii), soit la mesure (iv) s'applique :

(iii)

$$a1 = a4 = b2 = b3 = c1 = c4 = d = 0$$

et

$$a2 = a3 = 1;$$

(iv)

$$b2 = b3 = 0$$

et

$$c1 + c4 + d \geq 1$$

et

**a2 + a3 +a4** ≥ **3**, de préférence **a2** ≥ **2, a3** ≥ **1** et **a4 = 0.**

**3.** Composition, **caractérisée en ce qu'**elle comprend au moins un siloxane (A) selon au moins l'une quelconque des revendications 1 et 2.

**4.** Composition selon la revendication 3, **caractérisée en ce que** la composition comprend de plus au moins un siloxane choisi dans le groupe constitué par des siloxanes (B) et des siloxanes (C), où :

le siloxane (B) est un siloxane qui se différencie d'un siloxane selon la revendication 1 au moins **en ce que**, de préférence exactement **en ce que** les mesures (v) et (vi) s'appliquent à la place des mesures (i) à (iv) :

(v)

$$a2 = b2 = 0,$$

(vi)

$$a3 + b3 \geq 2;$$

le siloxane (C) est un siloxane qui se différencie d'un siloxane selon la revendication 1 au moins **en ce que**, de préférence exactement **en ce que** les mesures (vii) et (viii) s'appliquent à la place des mesures (i) et (iv) :

(vii)

$$a3 = b3 = 0\ ,$$

(viii)

$$a2 + b2 \geq 2.$$

**5.** Composition selon au moins l'une quelconque des revendications 3 et 4, **caractérisée en ce que**

a. la proportion en masse de l'au moins un siloxane (A), par rapport à la masse totale des siloxanes, est de 20 % à 70 %, préférablement de 25 % à 60 %, en particulier de 30 % à 50 % ; et/ou
b. la proportion en masse de l'au moins un siloxane (B), par rapport à la masse totale des siloxanes, est de 0 % à 15 %, préférablement de 1 % à 10 % ; et/ou
c. la proportion en masse de l'au moins un siloxane (C), par rapport à la masse totale des siloxanes, est de 3 % à 80 %, préférablement de 5 % à 60 %, en particulier de 10 % à 50 %.

**6.** Composition selon au moins l'une quelconque des revendications 3 à 5, **caractérisée en ce qu'**elle contient des amidamines, la proportion en masse d'amidamines par rapport à la masse totale des siloxanes étant inférieure à 1 %, préférablement inférieure à 0,8 %, plus préférablement inférieure à 0,6 %, en particulier inférieure à 0,4 %, ou ne contient aucune amidamine.

**7.** Procédé comprenant au moins une étape de procédé, dans lequel au moins un siloxane fonctionnalisé par époxy, qui présente au moins deux groupes époxy, est transformé aussi bien avec au moins une amine tertiaire, choisie dans le groupe constitué par des amidamines et des esteramines, de préférence des amidamines, qu'avec au moins une amine tertiaire choisie dans le groupe constitué par des dialcanolamines, avec formation de groupes ammonium

quaternaire.

**8.** Procédé selon la revendication 7 pour la préparation d'un siloxane (A) selon au moins l'une quelconque des revendications 1 et 2 ou d'une composition selon au moins l'une quelconque des revendications 3 à 6.

**9.** Procédé selon au moins l'une quelconque des revendications 7 et 8, **caractérisé en ce que** le siloxane fonctionnalisé par époxy est préparé par hydrosilylation d'au moins un époxyde oléfiniquement insaturé, de préférence choisi dans le groupe constitué par l'éther d'allyle et de glycidyle, le monoxyde de vinylcyclohexène et le monoépoxyde de norbornadiène, en particulier l'éther d'allyle et de glycidyle, avec au moins un siloxane fonctionnalisé par SiH de formule (V)

$$M^1_{a1}M^5_{a5}D^1_{b1}D^5_{b5}T^1_{c1}T^4_{c4}Q_d \qquad (V),$$

avec

$M^5 = [R^1_2SiHO_{1/2}]$,
$D^5 = [R^1SiHO_{2/2}]$,
a5 = 0 à 32, préférablement 1 à 10, particulièrement préférablement 2 à 3, en particulier 2 ;
b5 = 0 à 10, préférablement 0 à 5, en particulier 0 ;
**$M^1$, $D^1$, $T^1$, $T^4$, Q, a1, b1, c1, c4, d** et $R^1$ étant définis comme dans la formule (I).

**10.** Procédé selon au moins l'une quelconque des revendications 7 à 9, **caractérisé en ce que** le siloxane fonctionnalisé par époxy est un siloxane de formule (VI),

$$\mathbf{M^1_{a1}M^6_{a5}D^1_{b1}D^6_{b5}\,T^1_{c1}T^4_{c4}Q_d} \qquad (VI),$$

avec

$M^6 = [R^{13}R^1_2SiO_{1/2}]$,
$D^6 = [R^{13}R^1SiO_{2/2}]$,
$R^{13}$ = radicaux époxy organiques à chaque fois indépendamment les uns des autres identiques ou différents, de préférence choisis dans le groupe constitué par

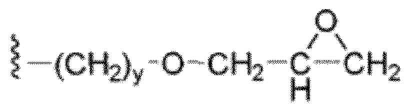

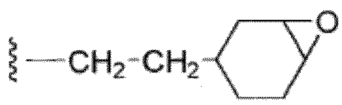

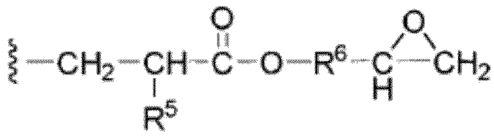

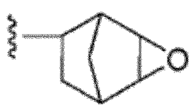
,

en particulier

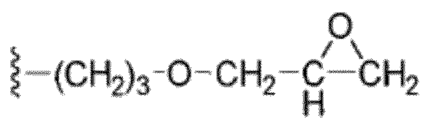
,

**$M^1$, $D^1$, $T^1$, $T^4$, Q, a1, a5, b1, b5, c1, c4, d, $R^1$, $R^5$, $R^6$** et y étant définis comme dans la formule (I).

**11.** Procédé selon au moins l'une quelconque des revendications 7 à 10, **caractérisé en ce que** la teneur résiduelle en amines tertiaires, choisies dans le groupe constitué par des amidamines et des esteramines, de préférence des amidamines, après la transformation, en tant que proportion en masse par rapport à la masse totale de la composition, est inférieure à 1 %, préférablement inférieure à 0,8 %, plus préférablement inférieure à 0,6 %, en particulier inférieure

à 0,4 %.

**12.** Procédé selon au moins l'une quelconque des revendications 7 à 11, **caractérisé en ce que** l'amine tertiaire, choisie dans le groupe constitué par des amidamines et des esteramines, est une amine tertiaire selon la formule (VII),

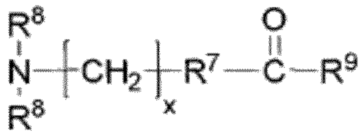

formule (VII),

$R^8$,$R^7$,$R^9$ et x étant définis comme dans la formule (II) .

**13.** Procédé selon au moins l'une quelconque des revendications 7 à 12, **caractérisé en ce que** l'amine tertiaire, choisie dans le groupe constitué par des dialcanolamines, est une amine tertiaire selon la formule (VIII),

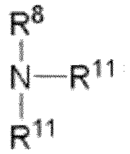

formule (VIII)

$R^8$ et $R^{11}$ étant définis comme dans la formule (III) .

**14.** Composition, pouvant être obtenue par un procédé selon au moins l'une quelconque des revendications 7 à 13.

**15.** Composition, de préférence émulsion aqueuse, **caractérisée en ce que** la composition comprend de l'eau ainsi qu'au moins un siloxane (A) selon au moins l'une quelconque des revendications 1 et 2 ou une composition selon au moins l'une quelconque des revendications 3 à 6 et/ou la revendication 14 et/ou un produit de procédé d'un procédé selon au moins l'une quelconque des revendications 7 à 13,
de préférence comprenant, indiqué en proportions en masse par rapport à la masse totale de la composition, les composants suivants :

a) 20 % à 99,5 %, préférablement 40 à 97 %, en particulier 60 à 95 % d'eau ;
b) 0,5 % à 80 %, préférablement 3 % à 60 %, en particulier 5 % à 40 % d'au moins un siloxane, comprenant au moins un siloxane (A) et de préférence au moins un siloxane (B) et/ou au moins un siloxane (C) ;
c) de préférence 1 % à 10 % d'au moins un émulsifiant ;
d) de préférence 5 % à 20 % d'au moins un glycol ; et
e) de préférence 0 % à 1 % d'acide acétique.

**16.** Utilisation d'un siloxane (A) selon au moins l'une quelconque des revendications 1 et 2 et/ou d'une composition selon au moins l'une quelconque des revendications 3 à 6 et/ou au moins l'une quelconque des revendications 14 à 15 et/ou d'un produit de procédé d'un procédé selon au moins l'une quelconque des revendications 7 à 13

A) pour le traitement, de préférence l'équipement et/ou l'imprégnation, de matériaux en feuille ;
B) dans des formulations de nettoyage et d'entretien pour le ménage et pour des applications industrielles, en particulier dans des agents adoucissants,
C) dans des compositions cosmétiques, pharmaceutiques et dermatologiques, en particulier dans des formulations cosmétiques de nettoyage et d'entretien, des agents de traitement capillaire et des agents de posttraitement capillaire ; et/ou
D) pour le nettoyage et l'entretien de surfaces dures, de préférence pour le nettoyage et l'entretien de véhicules, en particulier en tant qu'additif dans des auxiliaires de séchage pour des voies de lavage de voitures.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102010000993 A1
- DE 102009029450 A1
- DE 102011078382 A1
- DE 102010001531 A1
- US 5248783 A
- DE 3719086 C1
- DE 3802622 A1
- US 5578692 A
- EP 2176319 B1
- US 20100184634 A